# EUROPÄISCHE PATENTSCHRIFT

(11) **EP 0 385 452 B1**
(45) Veröffentlichungstag und Bekanntmachung des Hinweises auf die Patenterteilung: **01.06.1994**
(21) Anmeldenummer: 90103932.1
(22) Anmeldetag: 01.03.1990
(51) Int. Cl.: C07C 235/80, C07C 235/74, G01N 27/333

(54) **Carbonsäureamide, die mit Magnesiumionen lipophile Komplexe bilden, Verfahren zur Herstellung der lipophilen Magnesiumkomplexe und ionenselektive Teile**
Carboxylic acid amides which form lipophilic complexes with magnesium ions, method for preparation of the lipophilic magnesium complexes and ion selective components
Amides d'acides carboxyliques qui forment des complexes lipophiliques avec des ions de magnésium, procédé pour la préparation des complexes lipophiliques de magnésium et composants ions sélectifs

(30) Priorität: 03.03.1989 CH 811/89
(43) Veröffentlichungstag der Anmeldung: 05.09.1990
(73) Patentinhaber: Willi Möller AG, CH-8050 Zürich (CH)
(72) Erfinder: Simon, Wilhelm, CH-8006 Zürich (CH); Rouilly, Marizel Veronique, CH-8050 Zürich (CH); Rusterholz, Bruno, CH-8800 Thalwil (CH)
(74) Vertreter: Blum, Rudolf Emil Ernst

(56) Entgegenhaltungen:
- EP-A- 0 307 933
- FR-A- 2 306 683
- US-A- 3 957 607
- HELVETICA CHIMICA ACTA, Band 63, Nr. 237, 1980, S. 2264-2230, Schweizerische Chemische Gesellschaft, CH; D. ERNE et al: "Lipophilic amides of EDTA, NTA and iminodiacetic acid as ionophores for alkaline earth metal cations"
- ANALYTICAL CHEMISTRY, Band 60, Nr. 19, 1988, S. 2013-2016, American Chemical Society; M. V. ROUILLY et al: "Neutral-carrier-based magnesium-selective electrode"

## Beschreibung

Verschiedene Carbonsäureamide, hauptsächlich Dicarbonsäurediamide, aber auch einige Tricarbonsäuretriamide und Tetracarbonsäuretetra-amide, die mit Kationen lipophile Komplexe bilden, sind bekannt und es ist auch bekannt, dass diese Carbonsäureamide als ionenselektive Komponente von ionenselektiven Teilen zur Bestimmung der fraglichen Kationen in Probelösungen herangezogen werden können.

Seit langem war man bestrebt, Carbonsäureamide bereitzustellen, die eine ausreichend hohe Selektivität für Magnesiumionen gegenüber Alkalimetallionen und Calciumionen aufweisen, sodass mit deren Hilfe die Bestimmung von Magnesiumionen in Probelösungen möglich ist, welche als weitere Komponenten Natriumionen, Calciumionen und Kaliumionen enthalten. Bisher bekannte lipophile Komplexbildner für Magnesiumionen hatten jedoch im besten Fall etwa eine gleich hohe Selektivität für Magnesiumionen und Calciumionen oder mit Hilfe der entsprechenden Komponenten konnte eine Bestimmung von Magnesiumionen nur im basischen Medium vorgenommen werden, weil die Anwesenheit von Protonen störte.

Ziel der vorliegenden Erfindung war es, Carbonsäureamide bereitzustellen, die eine ausreichend hohe Selektivität für Magnesiumionen gegenüber anderen Alkalimetallionen oder Erdalkalimetallionen-aufweisen, sodass eine Bestimmung der Magnesiumionen auch in biologischem Material, wie Körperflüssigkeiten, möglich ist, und bei denen ferner die Komplexbildung mit den Magnesiumionen durch Protonen nicht gestört wird, sodass sie auch im neutralen oder sogar leicht sauren pH-Bereich stattfinden kann.

### BESCHREIBUNG DES STANDES DER TECHNIK

Dicarbonsäurediamide, welche mit Kationen, beispielsweise Metallkationen,lipophile Komplexe bilden, sind seit langem bekannt und es sei in diesem Zusammenhang beispielsweise auf die entsprechenden Dicarbonsäurediamide verwiesen, die in der USA Patentschrift Nr. 3,957,607 von Simon et al. geoffenbart sind.

In der Veröffentlichung von F. Lanter, D. Erne, D. Ammann und W. Simon in Analytical Chemistry, Band 52, Nr. 14, Dezember 1980, Seiten 2400 bis 2402, werden magnesiumselektive Mikroelektroden beschrieben, deren ionenselektive Komponente ein Bernsteinsäurediamid ist, dessen amidbildendes Amin das n-Heptyl-methylamin ist. Die Selektivität dieses Komplexbildners gegenüber Magnesiumionen ermöglichte jedoch nur in einem eingeschränkten Bereich intrazelluläre Magnesiumtests, weil die Selektivität gegenüber Natriumionen, die in biologischem Material, wie Körperflüsigkeiten, in wesentlich höheren Konzentrationen vorliegen, als Magnesiumionen, nicht ausreichend war und weil auch die Selektivität gegenüber Calciumionen unbefriedigend war.

In der Veröffentlichung von D. Erne, N. Stojanac, D. Ammann, P. Hofstetter, E. Pretsch und W. Simon in Helvetica Chimica Acta, Band 63, Heft 8, Seiten 2271 - 2279, 1980, wurde bereits darauf hingewiesen, dass die Magnesiumionen bei der Komplexbildung eine octaedrische Coordination bevorzugen und es wurden dort neben Dicarbonsäurediamiden auch einige Tricarbonsäuretriamide untersucht, weil man annahm, dass die Tricarbonsäuretriamide in der Lage sind, 1 : 2 Kation/Ligand-Komplexe zu bilden, bei denen um das Magnesiumzentralion zwei Moleküle des Tricarbonsäuretriamides so angeordnet sind, dass die sechs Sauerstoffatome der beiden Tricarbonsäuretriamide mit dem Magnesium einen Komplex mit octaedrischer Struktur bilden. Die untersuchten Tricarbonsäuretriamide wiesen die folgende Formel A 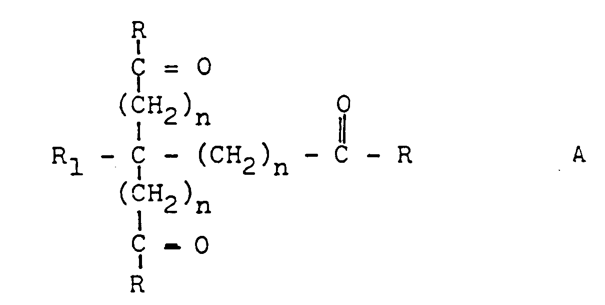
auf, in welcher R₁ Wasserstoff oder Methyl war, n 0, 1 oder 2 bedeutete, und das amidbildende Amin R entweder ein sekundäres Dialkylamin mit 4 oder 6 Kohlenstoffatomen in jeder der Alkylgruppen oder Methyl-n-heptylamin oder Dicyclohexylamin war. Es wurden Vergleiche mit analog aufgebauten Dicarbonsäurediamiden, nämlich den entsprechenden Diamiden der Oxalsäure, der Malonsäure, der Bernsteinsäure und der Glutarsäure durchgeführt. Ueberraschenderweise zeigte es sich, dass das entsprechende Diamid der Bernsteinsäure, in welchem das amidbildende Amin ein n-Heptylmethylamin ist, zwar Calciumionen gegenüber Magnesiumionen um einen Faktor von etwa 20 bevorzugt, dennoch aber überraschenderweise immer noch eine bessere Selektivität gegenüber Magnesiumionen aufweist, als das entsprechende Triamid der Formel A, in welcher R₁ Wasserstoff ist, zwei Symbole n 2 sind und eines der Symbole n 0 ist, wobei jedoch das amidbildende Amin ebenfalls n-Heptylmethylamin ist. Auch von den anderen getesteten Tricarbonsäuretriamiden der Formel A wies keines eine Magnesiumselektivität gegenüber Calcium und Alkalimetallionen auf. Ueberraschenderweise zeigte sogar dasjenige Tricarbonsäuretriamid der Formel A, in welchem R₁ Wasserstoff ist, sämtliche Symbole n 0 bedeuten und das amidbildende Amin Dicyclohexylamin ist, eine gewisse Bevorzugung für Caesiumionen gegenüber Erdalkalimetallionen und anderen Alkalimetallionen.

In der Veröffentlichung von D. Erne, N. Stojanac, D. Ammann, E. Pretsch und W. Simon in Helvetica Chimica Acta, Band 63, Heft 8, Seiten 2264 - 2270, 1980, wurden weitere Dicarbonsäurediamide, Tricarbonsäuretriamide und auch ein Tetracarbonsäuretetraamid auf ihre Selektivität gegenüber Magnesiumionen getestet. Die dort getesteten Tricarbonsäuretriamide unterscheiden sich von den Tricarbonsäuretriamiden der Formel A dahingehend, dass das Zentralatom jetzt ein Stickstoffatom statt eines Kohlenstoffatoms war, und der Wert n war in den getesteten Verbindungen 1. Auch unter den getesteten Diamiden befanden sich solche, bei denen in der Gruppe, welche die beiden Carbonsäureamidgruppierungen miteinander verband, ein Stickstoffatom (tertiäre Aminogruppe) oder ein Sauerstoffatom oder Schwefelatom einer Aether-, beziehungsweise Thioäthergruppierung, vorhanden war, weil man annahm, dass die freien Elektronenpaare dieser Heteroatome zusammen mit den Sauerstoffatomen der beiden Carbonsäureamidgruppierungen die Ausbildung eines 1 : 2 Kation/Ligand-Komplexes mit Magnesiumkationen begünstigen würden.

Das getestete Tetracarbonsäuretetraamid hatte die folgende Struktur B 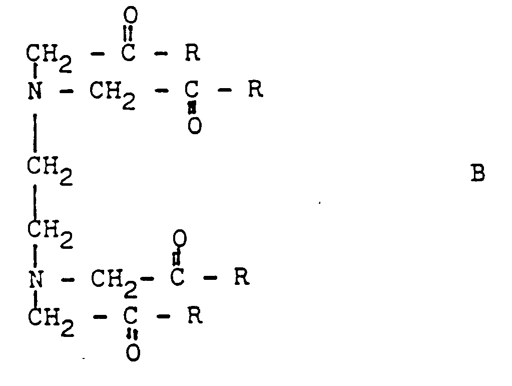
in welcher das amidbildende Amin n-Heptyl-methylamin war. In diesem getesteten Tetracarbonsäuretetra-amid standen also sechs Coordinationsstellen für die Ausbildung eines 1 : 1 Kation/Ligand-Komplexes, der eine octaedrische Struktur aufweist, zur Verfügung, denn es wurde angenommen, dass das Magnesiumzentralion in der Lage ist, mit den freien Elektronenpaaren der beiden Stickstoffatome und denjenigen der 4 Sauerstoffatome des Tetracarbonsäure-tetraamides in Wechselwirkung zu treten. Keines der dort in entsprechenden Kunststoffmembranen getesteten Dicarbonsäurediamide, Tricarbonsäuretriamide, beziehungsweise Tetracarbonsäure-tetraamide, wies jedoch eine höhere Selektivität für Magnesiumionen im Vergleich zu Calciumionen und/oder Alkalimetallionen, insbesondere Natriumionen auf. Das Tetracarbonsäure-tetraamid der Struktur B hatte sogar überraschenderweise eine höhere Selektivität für Bariumionen, Calciumionen und Natriumionen als für Magnesiumionen und war damit völlig ungeeignet.

In der Veröffentlichung von D. Erne, D. Ammann, A.F. Zhukov, F. Behm, E. Pretsch und W. Simon in Helvetica Chimica Acta, Band 65, Heft 2, Seiten 538 - 545, 1982, sind eine grosse Anzahl an Dicarbonsäurediamiden beschrieben, die bezüglich ihrer Selektivität gegenüber verschiedenen Alkalimetallionen und Erdalkalimetallionen in entsprechenden ionenselektiven Membranen getestet wurden. Auch dort konnte keine einzige Verbindung gefunden werden, die eine bessere Selektivität gegenüber Magnesiumionen als Alkalimetallionen oder anderen Erdalkalimetallionen aufwies.

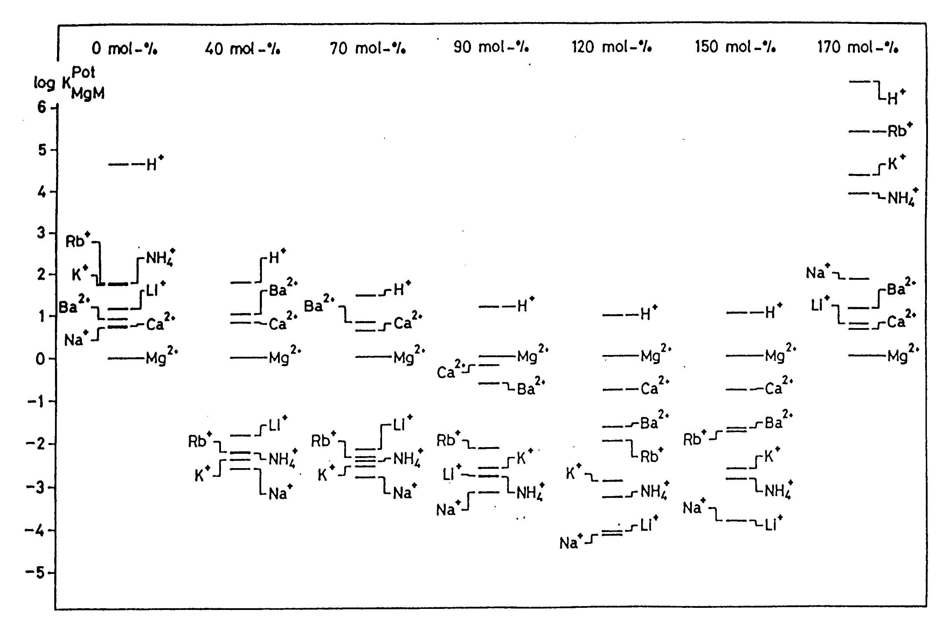

Trotz der ausgedehnten Forschungsarbeiten, die auf diesem Gebiet gemacht wurden, war bis in jüngster Zeit das Bernsteinsäurediamid, in welchem das amidbildende Amin n-Heptyl-methylamin war,immer noch diejenige Verbindung, bei der man hoffte, durch eine Modifikation einen geeigneten lipophilen Komplexbildner für Magnesiumionen zu entwickeln. In der Veröffentlichung von M.V. Rouilly, M. Badertscher, E. Pretsch, G. Suter und W. Simon in Analytical Chemistry, Band 60, 19, Seiten 2013 - 2016, vom Oktober 1988, ist eine magnesiumselektive Elektrode beschrieben, in welcher die magnesiumselektive Komponente dasjenige Derivat des oben genannten Bernsteinsäurediamides ist, das an einem der beiden Kohlenstoffatome der Kette 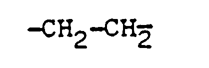
eine Aminogruppe als Substituenten trägt. Dieses aminosubstituierte Dicarbonsäurediamid, nämlich das entsprechende Amid der Asparaginsäure, in welchem das amidbildende Amin das n-Heptyl-methylamin ist, hatte tatsächlich in entsprechenden ionenselektiven Membranen auf Basis eines Polyvinylchloridpolymerisates, des Weichmachers o-Nitrophenyloctyläthers und eines Ionenaustauschers auf Basis eines Kaliumsalzes eines Tetraphenylborates, dessen Benzolkern in der Parastellung einen Chlorsubstituenten trägt, eine gute Selektivität für Magnesiumionen gegenüber anderen Alkalimetallionen und Erdalkalimetallionen, einschliesslich Calciumionen. Es sei in diesem Zusammenhang auf die Figur 3 der genannten Veröffentlichung verwiesen. Der Nachteil dieser magnesiumselektiven Komponente besteht darin, dass mit ihrer Hilfe eine Bestimmung von Magnesiumionen nur in basischen Probelösungen, deren pH-Wert im Bereich von 8 - 9 liegt, möglich ist. Dies ist darauf zurückzuführen, weil höhere Protonenkonzentrationen aufgrund der basischen Eigenschaften der freien Aminogruppe dieses Dicarbonsäurediamides die Komplexbildung mit den Magnesiumionen stören.

In allerjüngster Zeit wurden magnesiumselektive Elektroden zur Bestimmung von Magnesiumionen in Blutserum und zur Bestimmung der Wasserhärte entwickelt, welche als magnesiumselektive Komponente ein Tetracarbonsäure-tetraamid enthalten. Diese Elektroden sind in der Veröffentlichung von M. Müller, M. Rouilly, B. Rusterholz, M. Maj-Żurawska, Z. Hu und W. Simon in Mikrochim. Acta [Wien], III, Seiten 283 - 290, 1988 beschrieben. Die dort untersuchten Tetracarbonsäure-tetraamide weisen die folgende Formel C 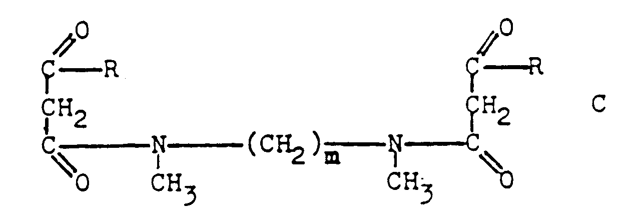
auf, wobei in dieser Formel das durch den Rest R veranschaulichte amidbildende Amin das n-Heptyl-methylamin ist und
in den untersuchten Verbindungen m den Wert 4 oder 6 oder 8 oder 10 aufweist. Von den dort beschriebenen Verbindungen weist diejenige, in welcher m 8 ist in entsprechenden ionenselektiven Membranen auf Basis von Polyvinylchlorid als Polymerkomponente, Chlorparaffin oder o-Nitrophenyläther oder Mischungen davon als Weichmacher und dem oben erwähnten Kaliumsalz des parachlorsubstituierten Tetraphenylborates die vorteilhaftesten Eigenschaften auf, dennoch hatten die besten der dort beschriebenen magnesiumselektiven Membranen für Calciumionen und Magnesiumionen etwa die gleiche Selektivität (siehe die Fig. 4 dieser Veröffentlichung).

In der französischen Patentveröffentlichung 2 306 683 der Bayer Aktiengesellschaft werden Carbonsäureamide beschrieben, welche die in Anspruch 1 auf Seite 101 dieser französischen Patentveröffentlichung angegebene Formel I aufweisen. In diesen Verbindungen kann n einen Wert von 0 - 4 aufweisen und wenn n den Wert 4 besitzt, dann sind die fraglichen Carbonsäureamide solche, die pro Molekül sechs Carbonsäureamidgruppen aufweisen und die folgende Struktur D 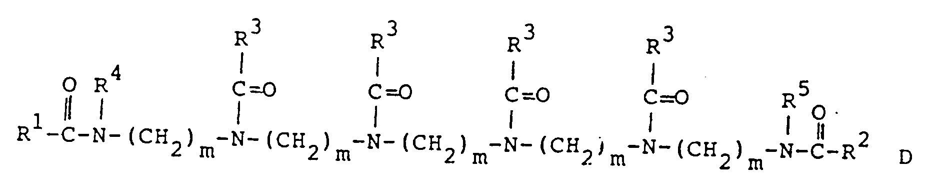
besitzen. Wie aus der Formel D ersichtlich ist, sind diese Verbindungen mit 6 Carbonsäureamidgruppen pro Molekül durch Umsetzung eines Hexaamines mit Monocarbonsäuren der Formel

R³-COOH

entstanden. Die dort beschriebenen Carbonsäureamide dienen als Wirkstoff von pharmazeutischen Präparaten zur Behandlung einer Hypolipidämie. Dementsprechend kann dieser französischen Patentveröffentlichung kein Hinweis entnommen werden, dass die dort beschriebenen Carbonsäureamide in der Lage sind, mit Magnesiumionen lipophile Komplexe zu bilden.

Ziel der vorliegenden Erfindung war es, Verbindungen auf Basis von Carbonsäureamiden zu entwickeln, die in der Lage sind, mit Magnesiumionen lipophile Komplexe zu bilden, und mit deren Hilfe ionenselektive Teile hergestellt werden können, die einerseits eine höhere Selektivität für Magnesiumionen im Vergleich zu Alkalimetallionen und anderen Erdalkalimetallionen, insbesondere Calciumionen, aufweisen und mit deren Hilfe ausserdem die Bestimmung der Magnesiumionen in Probelösungen möglich ist, die einen neutralen oder sauren pH-Wert aufweisen.

Ueberraschenderweise zeigte es sich, dass die angestrebten Ziele durch Carbonsäureamide erzielt werden können, die pro Molekül 6 Carbonsäureamidgruppen aufweisen und sich von 3 aneinander gebundenen Dicarbonsäurediamidstrukturen ableiten.

### BESCHREIBUNG DER ERFINDUNG

Die vorliegende Erfindung betrifft Carbonsäureamide, die mit Magnesiumionen lipophile Komplexe bilden, und die dadurch gekennzeichnet sind, dass sie pro Molekül 6 Carbonsäureamidgruppen aufweisen, und wobei pro Molekül der Carbonsäureamide drei Dicarbonsäurediamidgruppierungen vorliegen, welche die folgende Formel I 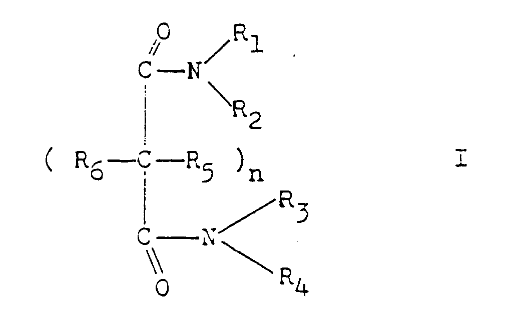
aufweisen, wobei in dieser Formel I die Reste R₁, R₂, R₃ und R₄ unabhängig voneinander
Wasserstoffatome, gegebenenfalls substituierte Alkyl, Alkenyl oder Alkinyl-Reste, gegebenenfalls substituierte Cycloalkylreste oder gegebenenfalls substituierte aromatische oder heterocyclische Reste oder Kombinationen aus zwei oder mehr derartiger Reste,
wie zum Beispiel aryl-, beziehungsweise heterocyclischsubstituierte Alkylreste oder alkylsubstituierte aromatische oder heteroaromatische Reste darstellen,
R₅ und R₆ Wasserstoffatome und Alkylgruppen mit 1 - 4 Kohlenstoffatomen oder Halogenatome sind und
n 0 oder eine ganze Zahl im Bereich von 1 - 3 ist, wobei jedoch
mindestens einer der Reste R₁, R₂, R₃, R₄, R₅ und R₆ gemeinsam mit zwei entsprechenden Resten R₁', R₂', R₃', R₄', R₁'', R₂'', R₃'', R₄'', R₅', R₆', R₅'' oder R₆'' der beiden weiteren Dicarbonsäurediamidstrukturen der entsprechenden Formel I', beziehungsweise I''
einen mehrwertigen aliphatischen, alicyclischen, aromatischen oder heteroaromatischen oder eine Kombination aus derartigen Resten darstellt, welche die fragliche Dicarbonsäurediamidgruppierung der Formel I an ein Stickstoffatom oder Kohlenstoffatom einer weiteren entsprechenden Dicarbonsäurediamidstruktur der Formel I', beziehungsweise I'', des Hexacarbonsäurehexa-amides bindet und wobei die Gesamtstruktur dieser Verbindung mit 6 Carbonsäure-amidgruppen pro Molekül offenkettig oder cyclisch ist, und wobei
gegebenenfalls entweder der Rest R₁ und R₂ gemeinsam mit dem Stickstoffatom, an welches die beiden Reste gebunden sind, oder der Rest R₃ und R₄ gemeinsam mit dem Stickstoffatom, an welches die beiden Reste R₃ und R₄ gebunden sind, einen gegebenenfalls noch weitere Heteroatome aufweisenden heterocyclischen Rest bilden oder gegebenenfalls der Rest R₁ oder der Rest R₂ des einen der beiden Stickstoffatome der Dicarbonsäurediamidgruppierung der Formel I gemeinsam mit dem Rest R₃ oder R₄ des anderen der beiden Stickstoffatome der Dicarbonsäurediamidgruppierung der Formel I diese Dicarbonsäurediamidgruppierung unter Ausbildung einer cyclischen Struktur ringschliesst.

In diesen, aus drei Dicarbonsäurediamidgruppierungen aufgebauten erfindungsgemässen Carbonsäure-amiden liegen also pro Molekül drei Dicarbonsäurediamidgruppierungen vor, welche die folgenden Strukturen I, I' und I'' 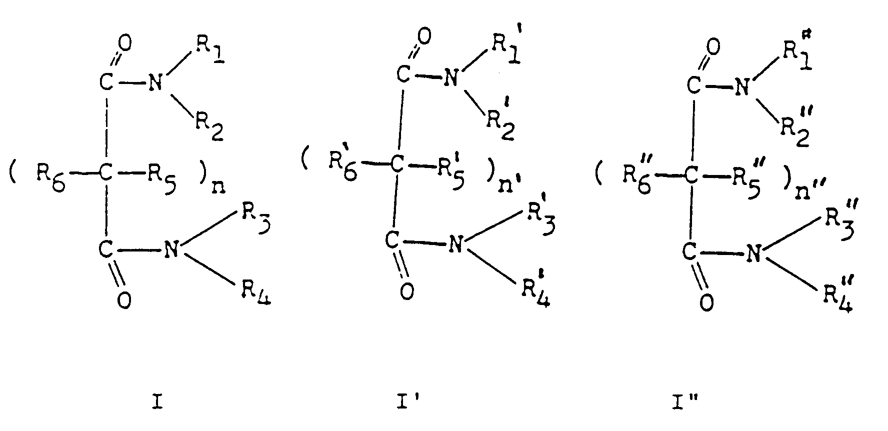
aufweisen, wobei in den Formeln I', beziehungsweise I'', die Reste R₁', R₂', R₃', R₄', R₁'', R₂'', R₃'' und R₄'' die gleiche Bedeutung aufweisen, wie die Reste R₁, R₂, R₃ und R₄ der Dicarbonsäurediamidgruppierung der oben angegebenen Struktur I. In gleicher Weise besitzen auch die Reste R₅', R₆', R₅'' und R₆'' der Dicarbonsäurediamideinheiten der Formeln I' und I'', und auch die Symbole n' und n'' der Dicarbonsäurediamidgruppierungen der Formeln I' und I'' die gleiche Bedeutung, wie die entsprechenden Reste R₅, R₆ und das Symbol n der Dicarbonsäurediamidgruppierung der oben angegebenen Struktur I. Wesentlich ist jedoch, dass mindestens einer der Reste R₁, R₂, R₃, R₄, R₅ und R₆ der Dicarbonsäurediamidgruppierung der Formel I, zusammen mit zwei entsprechenden Resten R₁', R₂', R₃', R₄', R₅', R₆', R₁'', R₂'', R₃'', R₄'', R₅'' oder R₆'' der beiden anderen Dicarbonsäurediamidstrukturen der Formel I', beziehungsweise I'', einen mehrwertigen aliphatischen, alicyclischen, aromatischen, heterocyclischen oder eine Kombination aus derartigen Resten bildet, wobei durch diese mehrwertige Gruppierung die Dicarbonsäurediamidstruktur der Formel I über ein Stickstoffatom derselben oder über ein Kohlenstoffatom derselben an ein Stickstoffatom oder ein Kohlenstoffatom der Dicarbonsäurediamidgruppierung der Formel I' und/oder der Dicarbonsäurediamidgruppierung der Formel I'', gebunden ist, sodass sich eine entsprechende Verbindung mit 6 Carbonsäureamidgruppen pro Molekül ausbildet, welche die Dicarbonsäurediamidstruktur der Formel I, die Dicarbonsäurediamidstruktur der Formel I' und ausserdem die Dicarbonsäurediamidstruktur der Formel I'', enthält, und wobei die Gesamtstruktur dieser Verbindung entweder nicht cyclisch oder monocyclisch oder bicyclisch oder tricyclisch oder polycyclisch ist.

Von den Carbonsäureamiden, die in ihrem Molekül drei Dicarbonsäurediamidgruppierungen der Formel I, I' und I'' aufweisen, sind eine bevorzugte Klasse an Verbindungen diejenigen, in welchen die Bindung der drei Dicarbonsäurediamidgruppierungen aneinander unter Ausbildung einer offenkettigen oder monocyclischen, bicyclischen, tricyclischen oder polycyclischen Gesamtstruktur so erfolgt, dass jeweils zwei Stickstoffatome unterschiedlicher derartiger Dicarbonsäurediamidgruppierungen miteinander über einen entsprechenden zweiwertigen organischen Rest verbunden sind. Diese bevorzugte Gruppe an erfindungsgemässen Hexacarbonsäurehexa-amiden weist also die folgende Formel II 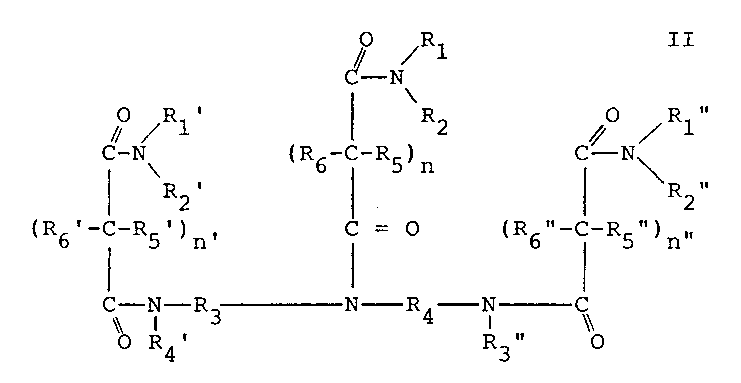
auf, wobei in dieser Formel II die Reste
R₃ und R₄ unabhängig voneinander zweiwertige aliphatische, alicyclische, aromatische oder heteroaromatische Reste oder eine Kombination aus derartigen Resten darstellen, welche die Stickstoffatome der drei Dicarbonsäurediamidgruppierungen der Verbindungen der Formel II miteinander verbinden und die Reste und Symbole
R₁', R₂', R₄', R₅', R₆', R₁'', R₂'', R₃'', R₅'', R₆'', n' und n'' die gleiche Bedeutung aufweisen, wie die Reste und Symbole R₁, R₂, R₃, R₄, R₅, R₆ und n in den Dicarbonsäurediamidgruppierungen der Formel I und wobei gegebenenfalls der Rest R₂ und/oder der Rest R₁, zusammen mit dem Rest R₁' oder R₂' oder R₄' oder R₃'' oder R₂'' oder R₁'' einen zweiwertigen Rest darstellt und/oder der Rest R₁' und/oder der Rest R₂', gemeinsam mit dem Rest R₃'' oder dem Rest R₂'' oder dem Rest R₁'' einen zweiwertigen Rest darstellt
und/oder
der Rest R₁'' oder der Rest R₂'', gemeinsam mit dem Rest R₄' einen zweiwertigen Rest darstellt und wobei die genannten zweiwertigen Reste aliphatische, alicyclische, aromatische oder heteroaromatische Reste oder eine Kombination aus derartigen Resten darstellen, welche die Carbonsäureamide der Formel II zu einer monocyclischen, bicyclischen, tricyclischen oder polycyclischen Verbindung ringschliessen.

Eine weitere Gruppe an bevorzugten erfindungsgemässen Carbonsäureamiden, welche mit Magnesiumionen selektiv lipophile Komplexe bildet, sind diejenigen Verbindungen, die in ihrem Molekül drei Dicarbonsäurediamidgruppierungen der weiter vorne angegebenen Formeln I, I' und I'' aufweisen, und in denen diese drei Dicarbonsäurediamidgruppierungen über Stickstoffatome derselben, unter Ausbildung eines Hexacarbonsäurehexaamides der folgenden Formel VIII 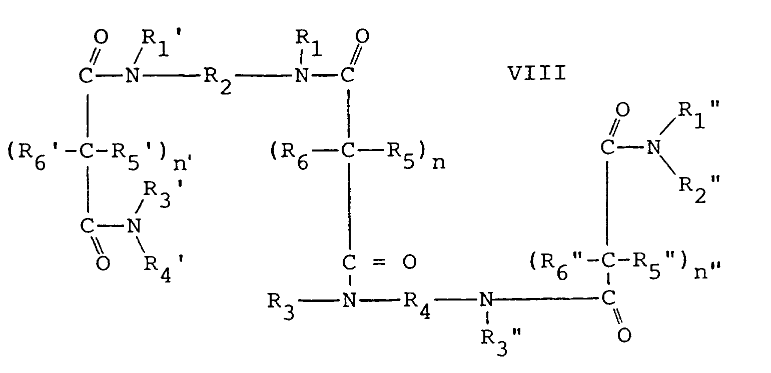
gebunden sind.

In diesen Carbonsäureamiden der Formel VIII bedeuten die Reste
R₂ und R₄ unabhängig voneinander zweiwertige aliphatische, alicyclische, aromatische oder heteroaromatische Reste oder Kombinationen aus diesen Resten und die Reste
R₁, R₁', R₁'', R₂'', R₃, R₃', R₄' und R₃'' haben die gleiche Bedeutung, wie sie weiter oben im Zusammenhang mit entsprechenden Dicarbonsäurediamidgruppierungen der Formeln I' I' und I'' angegeben wurden, oder in den Verbindungen der Formel VIII bildet der Rest R₁'' zusammen mit dem Rest R₂'' und/oder der Rest R₃' zusammen mit dem Rest R₄' und/oder der Rest R₁ zusammen mit dem Rest R₃ und dem entsprechenden Stickstoffatom, an welches diese Reste gebunden sind, einen heterocyclischen Ring, und die Reste
R₅, R₆, R₅', R₆', R₅'' und R₆'' haben die gleiche Bedeutung, wie sie weiter oben im Zusammenhang mit den entsprechenden Dicarbonsäurediamidgruppierungen der Formeln I, I' und I'' angegeben wurden oder
der Rest R₃ und/oder der Rest R₁ bildet zusammen mit dem Rest R₁', R₃', R₄', R₃'', R₂'' oder R₁'' einen zweiwertigen Rest und/oder der Rest
R₃' und/oder der Rest R₄' bildet zusammen mit dem Rest
R₃'' oder R₂'' oder R₁'', einen zweiwertigen Rest und/oder
der Rest R₁'' oder der Rest R₂'' bildet zusammen mit dem Rest R₄' einen zweiwertigen Rest und die erwähnten zweiwertigen Reste sind aliphatische, alicyclische, aromatische oder heteroaromatische Reste oder eine Kombination aus derartigen zweiwertigen Resten und die Carbonsäureamide der Formel VIII weisen entweder eine offenkettige Struktur auf oder durch die entsprechenden zweiwertigen Reste sind die Hexacarbonsäurehexa-amide der Formel VIII zu einer monocyclischen, bicyclischen, tricyclischen oder polycyclischen Gesamtstruktur ringgeschlossen.

In den Carbonsäureamiden der Formel II, beziehungsweise der Formel VIII können die zweiwertigen Reste R₃ und R₄', beziehungsweise die zweiwertigen Reste R₂ und R₄', welche die drei Dicarbonsäurediamidstrukturen miteinander verbinden, oder allenfalls vorhandene weitere zweiwertige Reste, welche die Carbonsäureamide der Formel II, beziehungsweise der Formel VIII zu einer monocyclischen, bicyclischen oder polycyclischen Verbindung ringschliessen, solche aliphatische, alicyclische, aromatische oder heteroaromatische Reste oder eine Kombination aus derartigen Resten darstellen, in welchen die Kohlenstoffhauptkette durch Heteroatome unterbrochen ist, beispielsweise ein oder mehrere Aethersauerstoffatome, Thioäthersauerstoffatome oder Gruppen der Formel 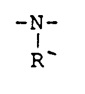
in welchen R 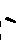 ein Wasserstoffatom, ein aliphatischer, alicyclischer, aromatischer oder heteroaromatischer Rest ist.

Um zu gewährleisten, dass in den Carbonsäure-amiden, die in ihrem Molekül drei Dicarbonsäurediamidgruppierungen der Formeln I, I', bzw. I'' aufweisen, beispielsweise in den bevorzugten Carbonsäure-amiden der Formeln II und VIII die beiden Carbonylgruppen der entsprechenden Dicarbonsäurediamidstrukturen der Formel I, beziehungsweise I', beziehungsweise I'', mit dem Magnesiumion als Zentrum bei der Komplexbildung in Wechselwirkung treten können, ist der Abstand der beiden Carbonylgruppen dieser Dicarbonsäurediamidstrukturen der Formel I, beziehungsweise I', beziehungsweise I'', von wesentlicher Bedeutung. Es zeigte sich, dass diejenigen Verbindungen, in denen n, beziehungsweise n', beziehungsweise n'' einen Wert von 4, 5, oder noch einen höheren Wert aufweiset, ein wesentlich geringeres Komplexbildungsvermögen für Magnesiumionen besitzen-als diejenigen, in denen n, beziehungsweise n', beziehungsweise n'', 0 oder eine Zahl von 1 - 3 ist. Ferner sind grosse raumbeanspruchende Substituenten an der die beiden Carbonylgruppen verbindenden Kohlenwasserstoffkette der Dicarbonsäurediamidstruktur der Formeln I, I', beziehungsweise I'', ungünstig, weil durch sie ebenfalls die Annäherung der beiden Carbonylgruppen der Carbonsäurediamidgruppierungen an das zweiwertige Magnesiumion behindert wird. Aus diesem Grunde müssen dann, wenn n einen Wert von 1 - 3 aufweist, die Substituenten R₅, beziehungsweise R₆, Wasserstoffatome, Alkylgruppen mit 1 - 4 Kohlenstoffatomen, vorzugsweise Methylgruppen, oder Halogenatome, vorzugsweise kleinere Halogenatome, wie Fluor oder Chlor, sein, wobei vorzugsweise sämtliche Reste R₅, R₆, R₅', R₆', R₅'', R₆'' die Bedeutung von Wasserstoffatomen aufweisen.

Bevorzugt sind ferner solche Dicarbonsäurediamidgruppen der Formel I, beziehungsweise I', beziehungsweise I'', in welchen n den Wert 1 oder 2 besitzt, wobei entsprechende Dicarbonsäurediamidstrukturen, in welchen n 1 ist und sowohl der Rest R₅ als auch der Rest R₆ ein Wasserstoffatom bedeutet, ganz speziell bevorzugt sind. Es handelt sich also in diesem Fall um Dicarbonsäurediamidstrukturen der Formel I, I' und I'', die sich vom Malonsäurediamid ableiten (für den Fall, dass n, n', beziehungsweise n'' 1 ist), beziehungsweise um Dicarbonsäurediamidstrukturen der Formeln I, I', beziehungsweise I'', die sich vom Bernsteinsäurediamid ableiten, nämlich für den Fall, dass n, n' und n'' 2 ist.

Speziell bevorzugt sind also solche Carbonsäure-amide, die in ihrem Molekül drei Dicarbonsäurediamidstrukturen der Formeln I, I', beziehungsweise I'', aufweisen, beziehungsweise solche Carbonsäure-amide der weiter vorne angegebenen Formeln II und VIII, in welchen mindestens eine der Dicarbonsäurediamidstrukturen der Formeln I, I' und I'', vorzugsweise 2, und speziell bevorzugt, alle 3 Dicarbonsäurediamidstrukturen der angegebenen Formeln sich vom Malonsäurediamid ableiten, während die restlichen dieser Dicarbonsäurediamidstrukturen, nämlich 2, beziehungsweise 1, beziehungsweise gar keine sich vom Bernsteinsäureamid ableiten.

Ein weiterer Gegenstand der vorliegenden Erfindung ist ein Verfahren zur Herstellung von lipophilen Komplexen aus Magnesiumionen und den pro Molekül 6 Carbonsäureamidgruppen aufweisenden Verbindungen, das dadurch gekennzeichnet ist, dass man die Magnesiumionen mit diesen Carbonsäureamiden in Berührung bringt.

Des weiteren betrifft die vorliegende Erfindung diese lipophilen Komplexe aus Magnesiumionen und Carbonsäureamiden.

Da bekanntlich Magnesiumionen die Neigung besitzen, Komplexe zu bilden, in denen das Magnesiumion mit 6 Heteroatomen unter Ausbildung einer octaedrischen Struktur in Wechselwirkung tritt, kann angenommen werden, dass die erfindungsgemässen Komplexe solche aus einem Mol Magnesium und einem Mol Carbonsäureamid sind, also 1 : 1 Kation/Ligand-Komplexe.

Die mit den erfindungsgemässen Komplexbildnern gebildeten Magnesiumkomplexe müssen lipophile Eigenschaften aufweisen. Deshalb sollen die Carbonsäureamide vorzugsweise frei von stark hydrophilen Substituenten, wie Carbonsäuregruppierungen und Sulfonsäuregruppierungen sein. Aus diesen Gründen sollen sie vorzugsweise auch frei von Hydroxylgruppen sein oder allenfalls nur eine geringe Anzahl an Hydroxylgruppen pro Molekül enthalten.

Um die lipophilen Eigenschaften der erfindungsgemässen Carbonsäureamide, beziehungsweise der mit diesen gebildeten Magnesiumkomplexe zu gewährleisten, ist es ferner vorteilhaft, wenn die entsprechenden Carbonsäureamide in ihrem Molekül mindestens einen gegenbenenfalls substituierten Alkyl, Alkenyl oder
Alkinyl-Rest aufweisen, der mindestens 4 Kohlenstoffatome aufweist und/oder die Stickstoffatome, beziehungsweise ein Stickstoffatom und ein Kohlenstoffatom, beziehungsweise zwei Kohlenstoffatome zweier unterschiedlicher Dicarbonsäurediamidgruppierungen dieser Verbindungen sollen miteinander durch eine gegebenenfalls substituierte zweiwertige aliphatische Gruppe, die gegebenenfalls ein oder zwei Doppelbindungen oder Dreifachbindungen aufweist, verbunden sein, wobei diese aliphatische Gruppe eine Kettenlänge von mindestens 4 Kohlenstoffatomen aufweist. Vorzugsweise beträgt die Kohlenstoffanzahl dieser gegebenenfalls substituierten Alkyl-, Alkenyl- oder Alkinyl-Reste 5 - 15, und speziell bevorzugt, 6 - 12.

Auch die gegebenenfalls substituierten zweiwertigen aliphatischen Gruppen, welche die Stickstoffatome, beziehungsweise Kohlenstoffatome zweier unterschiedlicher Dicarbonsäurediamidgruppen der Verbindungen miteinander verbinden, sollen vorzugsweise eine Kettenlänge von 5 - 15 Kohlenstoffatomen, speziell bevorzugt, 6 - 12 Kohlenstoffatomen aufweisen.

Speziell bevorzugt sind dementsprechend solche Carbonsäureamide der weiter vorne angegebenen Formel II, in welchen sowohl der Rest R₃ als auch der Rest R₄ eine zweiwertige aliphatische Gruppe einer Kettenlänge von mindestens 4 Kohlenstoffatomen darstellt, vorzugsweise eine entsprechende Alkylengruppe, und speziell bevorzugt einen unverzweigten Alkylenrest der Formel

-(CH₂)-_{z} ,

in welchem z eine ganze Zahl im Bereich von 5 - 15, speziell bevorzugt 6 - 12, ist.

In gleicher Weise sind auch solche Carbonsäure-amide der weiter vorne angegebenen Formel VIII speziell bevorzugt, in welchen sowohl der Rest R₂ als auch der Rest R₄ eine zweiwertige aliphatische Gruppe einer Kettenlänge von mindestens 4 Kohlenstoffatomen darstellt, speziell bevorzugt eine Alkylengruppe der oben angegebenen Struktur, in welcher z eine ganze Zahl im Bereich von 5 - 15, speziell bevorzugt 6 - 12, darstellt.

In den entsprechenden nicht cyclischen Carbonsäure-amiden der Formel II, beziehungsweise VIII, bedeutet vorzugsweise mindestens einer der Reste R₁, R₁', R₁'', beziehungsweise R₄', R₃'', beziehungsweise R₃', R₄', R₃ oder R₃'', einen aliphatischen Rest mit einer Kettenlänge von mindestens 4 Kohlenstoffatomen, vorzugsweise einen entsprechenden Alkylrest, und speziell bevorzugt eine Alkylgruppe mit 5 - 15, insbesondere 6 - 12 Kohlenstoffatomen. Von den angeführten Alkylgruppen sind diejenigen bevorzugt, die in ihrer Struktur höchstens drei Verzweigungsstellen, vorzugsweise höchstens zwei Verzweigungsstellen, aufweisen, und speziell bevorzugt sind die entsprechenden geradkettigen Alkylgruppen.

Vorzugsweise besitzen in den entsprechenden nicht cyclischen Carbonsäure-amiden zwei der Reste R₁, R₁' und R₁'' die oben definierten bevorzugten Bedeutungen, und speziell bevorzugt weisen sämtliche der Reste R₁, R₁' und R₁'' die genannten bevorzugten Bedeutungen auf.

In den entsprechenden nicht cyclischen Carbonsäure-amiden der Formel II sind die Reste R₂, R₂' und R₂'' Wasserstoffatome, gegebenenfalls substituierte aliphatische oder cycloaliphatische Reste oder gegebenenfalls substituierte aromatische oder heterocyclische Reste, wobei jedoch Wasserstoffatome und unsubstituierte Alkylreste, und zwar sowohl kurzkettige, wie der Methylrest, als auch die oben erwähnten langkettigen Reste bevorzugt sind.

Die oben gegebenen Erläuterungen für die Reste R₁ und R₂, beziehungsweise R₁', R₁'', sowie R₂' und R₂'', gelten natürlich auch für die entsprechenden Reste der bevorzugten Carbonsäure-amide der Formel VIII, also für die Reste R₁, R₃, R₁', R₃', R₄', R₃'', R₂'' und R₁'' dieser Carbonsäure-amide und auch für die entsprechenden Dicarbonsäurediamidstrukturen der weiter vorne angegebenen Formeln I, I' und I''.

In den entsprechenden monocyclischen, bicyclischen, tricyclischen und polycyclischen Carbonsäure-amiden der Formel II kann der Rest R₁ gemeinsam mit dem Rest R₁' oder gemeinsam mit dem Rest R₁'' einen entsprechenden zweiwertigen organischen Rest darstellen, wobei die entsprechenden aliphatischen Reste, die eine Kettenlänge von mindestens 4 Kohlenstoffatomen aufweisen und allenfalls noch eine oder mehrere Doppelbindungen oder Dreifachbindungen aufweisen, bevorzugt sind. Speziell bevorzugte zweiwertige Reste sind Alkylenreste der Formel

-(CH₂)_{z}- ,

in welcher z eine ganze Zahl im Bereich von 5 - 15, speziell bevorzugt, 6 - 12, ist.

In entsprechenden monocyclischen, bicyclischen oder tricyclischen Carbonsäure-amiden kann der Rest R₁' gemeinsam mit dem Rest R₁'' ebenfalls einen zweiwertigen organischen Rest der oben angegebenen Art darstellen, wobei auch hier entsprechende zweiwertige aliphatische Reste mit einer Kettenlänge von mindestens 4 Kohlenstoffatomen, die allenfalls noch ein oder mehrere Doppelbindungen oder Dreifachbindungen aufweisen, bevorzugt sind, und speziell bevorzugt sind auch hier entsprechende Alkylenketten der Formel

-(CH₂)_{z}- ,

in welcher z die oben angegebenen Bedeutungen besitzt.

In den Carbonsäure-amiden der Formel II, die eine bicyclische, tricyclische oder polycyclische Struktur aufweisen, kann sowohl der Rest R₁ gemeinsam mit dem Rest R₁', als auch der Rest R₂ gemeinsam mit dem Rest R₁'' eine zweiwertige organische Gruppe der oben angegebenen Art darstellen, speziell bevorzugt eine entsprechende aliphatische Gruppe einer Kettenlänge von mindestens 4 Kohlenstoffatomen, die gegebenenfalls noch eine oder mehrere Doppelbindungen oder Dreifachbindungen aufweist. Auch in diesem Falle sind die entsprechenden zweiwertigen organischen Reste vorzugsweise Alkylengruppen der Formel

-(CH₂)_{z}- ,

worin z die weiter oben angegebenen Bedeutungen aufweist.

Analoge Betrachtungen treffen für die entsprechenden monocyclischen, bicyclischen, tricyclischen und polycyclischen Carbonsäure-amide der Formel VIII zu, und in diesen kann der Rest R₁'' gemeinsam mit dem Rest R₁ oder R₃ oder R₁' oder R₃' oder R₄' oder gemeinsam mit dem Rest R₅ oder R₆ oder R₅' oder R₆' einen entsprechenden Zweiwertigen organischen Rest darstellen, wobei auch hier entsprechende aliphatische Reste bevorzugt sind, die eine Kettenlänge von mindestens vier Kohlenstoffatomen aufweisen und allenfalls noch eine oder mehrere Doppelbindungen oder Dreifachbindungen besitzen. Auch hier sind speziell bevorzugte zweiwertige Reste solche Alkylenreste der Formel

-(CH₂)_{z}- ,

in welcher z mindestens 4 ist, und vorzugsweise eine ganze Zahl im Bereich von 5 - 15, speziell bevorzugt 6 - 12, darstellt.

Die oben angeführten zweiwertigen organischen Reste können jedoch allenfalls auch noch solche Substituenten tragen, die keine stark hydrophilen Eigenschaften aufweisen, wie zum Beispiel Halogenatome.

Von den bevorzugten erfindungsgemässen Carbonsäure-amiden der Formel II sind diejenigen Carbonsäureamide speziell bevorzugt, welche die folgende Formel III 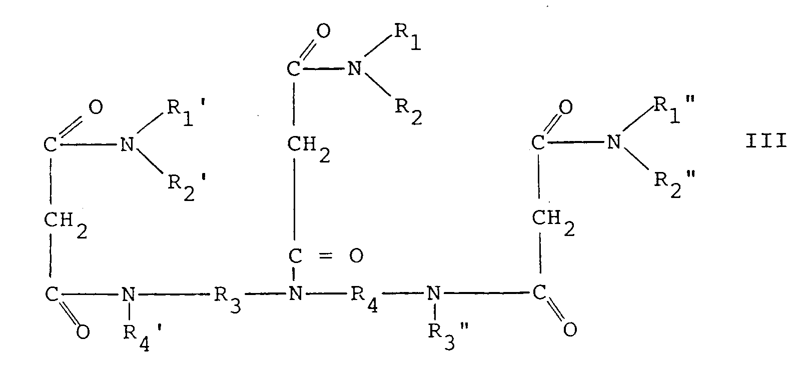
aufweisen, wobei in dieser Formel die Reste
R₃ und R₄ eine Gruppierung der Formel

-(CH₂)_{z}-

darstellen, in welcher z 5 - 9, vorzugsweise 5, 6 oder 7, ist, wobei vorzugsweise in beiden Resten z die gleiche Bedeutung aufweisen. Speziell bevorzugt ist in beiden Gruppierungen der oben angegebenen Formel z 6.

In den bevorzugten Verbindungen der Formel III bedeuten ferner die Reste
R₄' und R₃'' Wasserstoff oder einen Alkylrest mit 1 - 4 Kohlenstoffatomen und in den offenkettigen Verbindungen der Formel III stellen die Reste
R₁, R₁' und R₁'' vorzugsweise Alkylreste mit 5 - 9 Kohlenstoffatomen dar, wobei diese Reste R₁, R₁' und R₁'' vorzugsweise miteinander identisch sind. Speziell bevorzugt sind die entsprechenden geradkettigen Alkylreste und gemäss einem Beispiel für derartige offenkettige Verbindungen der Formel III sind sämtliche Reste R₁, R₁' und R₁'' n-Heptylreste. In den nicht cyclischen Verbindungen der Formel III sind die Reste
R₂, R₂' und R₂'' vorzugsweise Wasserstoffatome oder gegebenenfalls substituententragende Alkylgruppen, Alkenylgruppen oder Alkinylgruppen, insbesondere unsubstituierte längerkettige oder kürzerkettige Alkylgruppen, beispielsweise solche mit 1 - 4 Kohlenstoffatomen. Speziell bevorzugt sind in den nicht cyclischen Verbindungen der Formel III die Reste
R₂, R₂' und R₂'' miteinander identisch. Beispielsweise können sämtliche dieser Reste die Bedeutung von Methylgruppen aufweisen.

In den entsprechenden monocyclischen, bicyclischen, tricyclischen oder polycyclischen Verbindungen der Formel III kann ferner der Rest R₁ gemeinsam mit dem Rest R₁' und/oder der Rest R₂, gemeinsam mit dem Rest R₁'', einen zweiwertigen aliphatischen Rest bilden, wobei dieser vorzugsweise mindestens vier Kohlenstoffatome aufweist und allenfalls ein oder mehr Doppelbindungen oder Dreifachbindungen besitzt. Diese zweiwertigen aliphatischen Reste können gegebenenfalls solche Substituenten tragen, die keine stark hydrophilen Eigenschaften aufweisen, wie zum Beispiel Halogenatome, bevorzugte derartige zweiwertige Reste sind jedoch eine Alkylengruppe der Formel

-(CH₂)_{z}- ,

in welcher z eine ganze Zahl im Bereich von 5 - 15 ist.

Auch in den monocyclischen, bicyclischen und polycyclischen Carbonsäure-amiden der Formel III haben die einwertigen, nicht an ein weiteres Stickstoffatom gebundenen Reste R₂, R₂' und R₂'', vorzugsweise diejenigen Bedeutungen, die weiter oben im Zusammenhang mit den entsprechenden nicht cyclischen Verbindungen der Formel III angeführt wurden.

Von den Carbonsäure-amiden der weiter vorne angegebenen Formel VIII sind diejenigen speziell bevorzugt, welche die folgende Formel IX 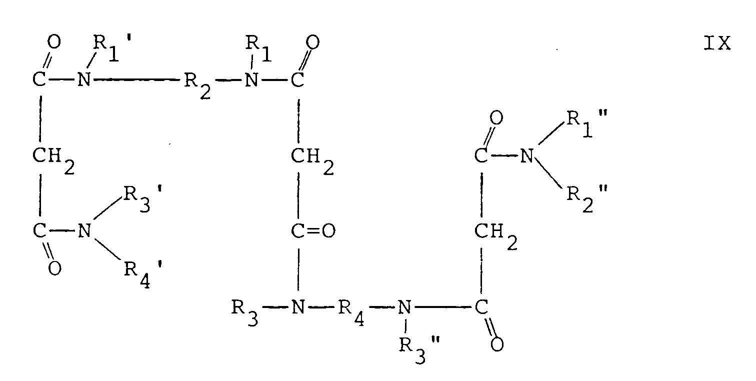
aufweisen.

In dieser Formel IX sind die Reste R₂ und R₄ unabhängig voneinander zweiwertige Gruppierungen, welche die folgende Formel

-(CH₂)_{z}-

aufweisen, in welcher
z eine ganze Zahl im Bereich von 5 - 9 ist,
R₁' und R₃ unabhängig voneinander Wasserstoffatome oder Alkylreste mit 1 - 4 Kohlenstoffatomen bedeuten,
R₃' und R₁'' Alkylreste mit 5 - 9 Kohlenstoffatomen bedeuten und
R₄', R₁, R₃', R₁' und R₂'' unabhängig voneinander Wasserstoffatome oder Alkylgruppen mit 1 - 12 Kohlenstoffatomen darstellen oder
der Rest R₃' zusammen mit dem Reste R₁' eine Alkylengruppe bildet und/oder der Rest R₄' zusammen mit dem Rest R₂'' eine Alkylengruppe darstellt oder der Rest R₃ zusammen mit dem Rest R₁' oder zusammen mit dem Rest R₂'' oder zusammen mit dem Rest R₁'' eine Alkylengruppe bildet, wobei die entsprechenden Alkylengruppen vorzugsweise eine Struktur der Formel

-(CH₂)_{z}- ,

aufweisen, in welcher
z eine ganze Zahl von 5 - 9 ist und wobei
in den Verbindungen der Formel IX verbleibende Reste, die nicht eine zweiwertige Alkylengruppe der angegebenen Formel bilden, Wasserstoffatome oder Alkylreste mit 1 - 12 Kohlenstoffatomen darstellen.

Wie bereits erwähnt wurde, sind bevorzugte erfindungsgemässe lipophile Magnesiumkomplexe 1:1 Komplexe aus einem Mol Mg²⁺ Ionen und einem Mol Hexacarbonsäurehexa-amid. Dementsprechend sind auch die bevorzugten Magnesiumkomplexe der Carbonsäure amide der Formeln II und VIII, beziehungsweise III und IX 1:1 Komplexe aus einem Mol Magnesiumionen und einem Mol des Carbonsäure-amides der angegebenen Formeln.

Die Carbonsäure-amide, die in ihrem Molekül drei Dicarbonsäurediamidgruppierungen der Formeln I, I' und I'' aufweisen und die bevorzugten Carbonsäure-amide der Formeln II und VIII, beziehungsweise III und IX, sind neue chemische Verbindungen. Sie können nach üblichen Arbeitsverfahren hergestellt werden, beispielsweise indem man entsprechende Carbonsäuren oder deren reaktive Derivate, wie zum Beispiel aktive Ester, Carbonsäureanhydride oder Carbonsäurehalogenide mit den entsprechenden Aminen umsetzt.

So können beispielsweise die Carbonsäure-amide der Formel II 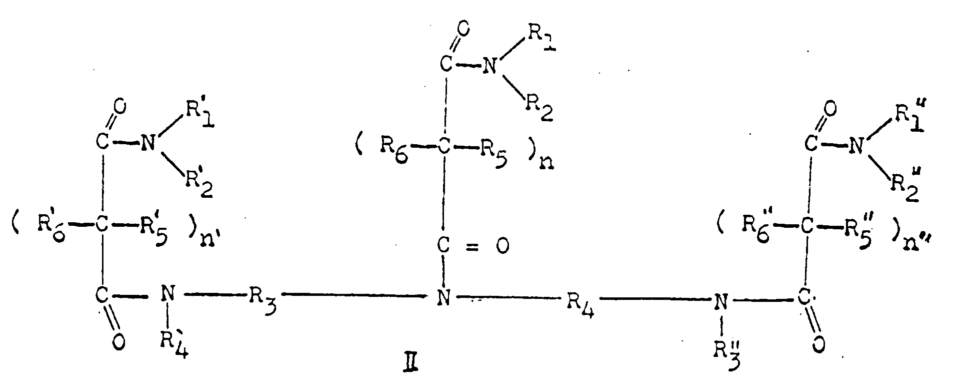
in welchen die Reste
R₃ und R₄ unabhängig voneinander zweiwertige aliphatische, alicyclische, aromatische, heteroaromatische oder eine Kombination aus derartigen Resten darstellen und
R₁, R₂, R₅, R₆, R₁', R₂', R₄', R₅', R₆', R₁'', R₂'', R₃'' R₅'', R₆'', sowie n, n' und n'' die weiter oben angegebene Bedeutung besitzen,
hergestellt werden, indem man ein Triamin der Formel IV 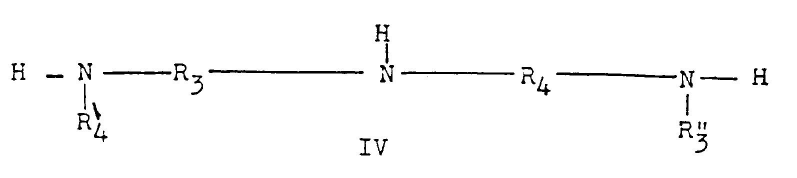
mit Carbonsäuren der folgenden Formeln Va, Vb und Vc 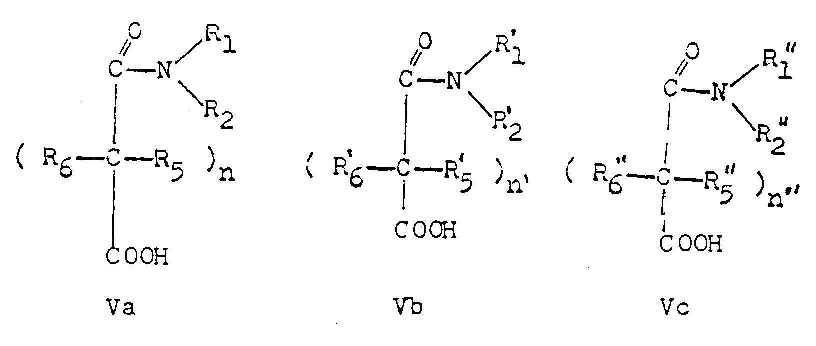
oder reaktiven Derivaten dieser Carbonsäuren zu dem entsprechenden Carbonsäure-amid der Formel II umsetzt,
oder indem man das Amin der Formel IV zunächst mit Dicarbonsäuren der Formeln VIa, VIb, und VIc 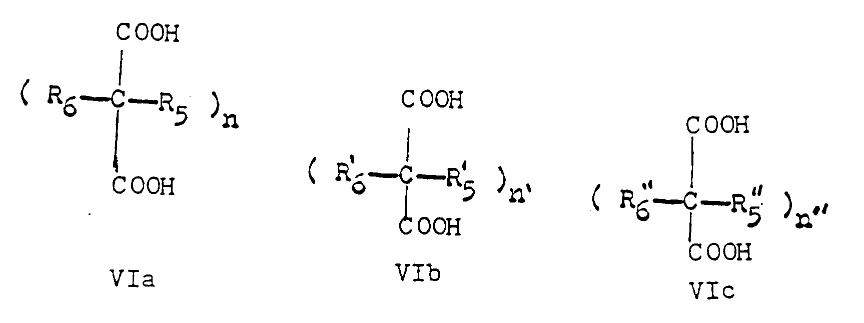
oder resktiven Derivsten dieser Dicarbonsäuren umsetzt, wobei gegebenenfalls die eine der beiden Carboxylgruppen der Dicarbonsäuren, beziehungsweise der resktiven Dicarbonsäurederivate, in geschützter Form vorliegt, und wobei man anschliessend das erhaltene Zwischenprodukt der Formel VII 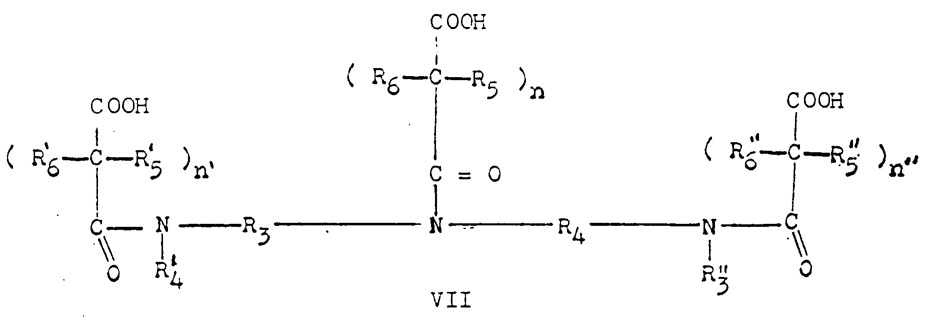
oder ein reaktives Derivat dieses Zwischenproduktes der Formel VII mit den entsprechenden Aminen zu dem gewünschten Endprodukt der Formel II umsetzt.

Bei der Herstellung der speziell bevorzugten Carbonsäure-amide der Formel III nach der zuerst beschriebenen Verfahrensvariante wird das entsprechende Triamin der Formel IV mit einem entsprechenden Monoamid der Malonsäure oder einem reaktiven Derivat derselben, beispielsweise dem Mono-4-nitrophenylester eines Malonsäuremonoamides zu dem gewünschten Endprodukt der Formel III umgesetzt. Die Herstellung kann beispielsweise analog durchgeführt werden, wie dies für die Tetracarbonsäuretetraamide der Formel C in der weiter vorne genannten Veröffentlichung von M. Müller et al. in Mikrochimica Acta, 1988, auf den Seiten 284 und 285 beschrieben ist.

Die bevorzugten Carbonsäure-amide der Formel VIII 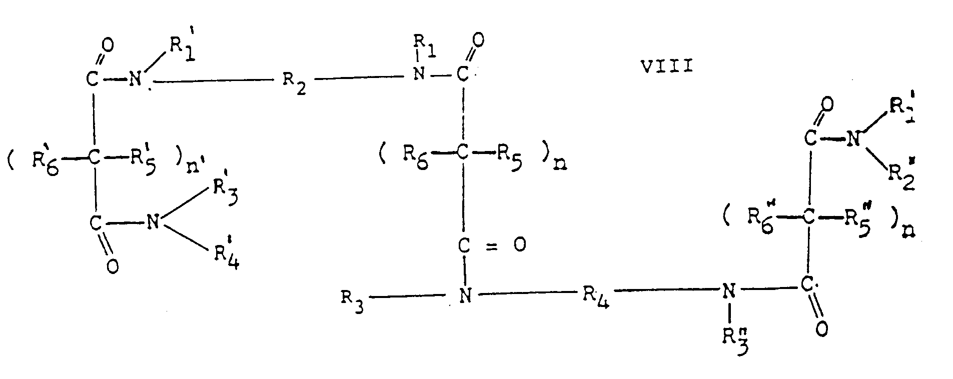
in welchen die Substituenten und die Symbole die weiter vorne angegebenen Bedeutungen aufweisen, werden vorzugsweise hergestellt, indem man zwei Aminoverbindungen der Formeln 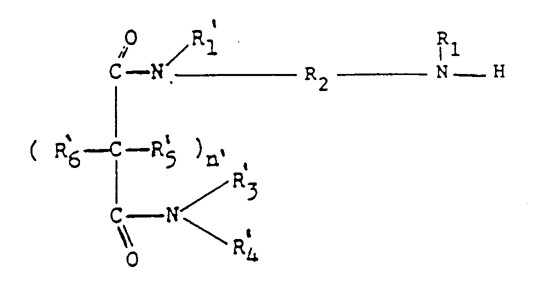
und 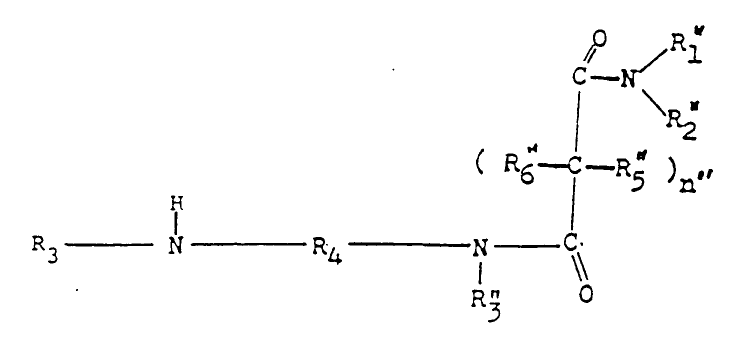
mit einer Dicarbonsäure der Struktur 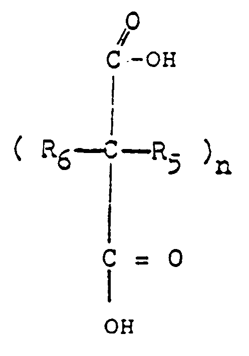
oder einem reaktiven Derivat dieser Dicarbonsäure umsetzt, wobei man das Carbonsäure-amid der Formel VIII erhält. Wenn man bei diesem Herstellungsverfahren als reaktives Derivat der Dicarbonsäure ein entsprechendes Dicarbonsäurehalogenid verwendet, beispielsweise ein Dicarbonsäuredichlorid, dann führt man die Reaktion vorzugsweise in Anwesenheit einer Base durch, welche die bei der Umsetzung frei gesetzte Halogenwasserstoffsäure neutralisiert.

Ein weiterer Gegenstand der vorliegenden Erfindung sind Testvorrichtungen, beispielsweise ionenselektive Teile zur Bestimmung der Aktivität, beziehungswiese Konzentration von Magnesiumionen in flüssigen Proben und diese Testvorrichtungen, beziehungsweise ionenempfindlichen Teile, enthalten erfindungsgemäss als Komponente, welche die Selektivität für die Magnesiumionen aufweist, eine pro Molekül 6 Carbonsäure-amidgruppen aufweisende Verbindung gemäss Anspruch 1
Die erfindungsgemässen Testvorrichtungen, beziehungsweise ionenselektiven Teile enthalten als magnesiumselektive Komponente solche Carbonsäure-amide, die pro Molekül drei Dicarbonsäurediamidgruppierungen der Formel I, also die Dicarbonsäurediamidgruppierungen der weiter vorn angegebenen Strukturen I, I' und I'', enthalten, wobei in diesen Formeln die Substituenten und Symbole die weiter vorne angegebenen Bedeutungen besitzen.

Speziell bevorzugt enthalten die entsprechenden Testvorrichtungen, beziehungsweise ionenselektiven Teile als magnesiumselektive Komponente die Carbonsäure-amide der weiter vorne angegebenen Formel II und der speziell bevorzugten weiter vorne angegebenen Formel III, beziehungsweise Carbonsäure-amide der weiter vorne angegebenen Formel VIII, beziehungsweise der weiter vorne angegebenen bevorzugten Formel IX.

Mit den entsprechenden Testvorrichtungen, beziehungsweise ionenselektiven Teilen können in Proben, beispielsweise wässrigen Proben, die Konzentrationen, beziehungsweise Aktivitäten, der Magnesiumionen bestimmt werden.

Mit Hilfe der erfindungsgemässen Carbonsäure-amide ist es möglich, entsprechende Testvorrichtungen, beziehungsweise ionenselektive Teile herzustellen, die eine ausreichende Selektivität für Magnesiumionen gegenüber Calciumionen und Natriumionen aufweisen, sodass die Bestimmung der Magnesiumkonzentration in biologischen Flüssigkeiten, beispielsweise Körperflüssigkeiten, wie Harn, Blutserum oder Gesamtblut möglich ist.

Das Blutserum des Menschen ist im allgemeinen etwa 1,1 Millimolar an in freier Form vorliegenden Calciumionen und 0,6 Millimolar an in freier Form vorliegenden Magnesiumionen. Weitere Mengen an Calciumionen und Magnesiumionen liegen in gebundener Form vor, und zwar gebunden an Proteinmoleküle des Blutserums. Ferner ändert sich der Anteil an freien Magnesiumionen im Blutserum mit dem pH-Wert des Blutserums, und zwar liegen bei steigendem pH-Wert des Blutserums mehr gebundene Magnesiumionen und weniger freie Magnesiumionen vor.

In der Medizin werden häufig an Patienten Magnesiumpräparate verabreicht um verschiedene krankhafte Zustände zu mildern oder es erfolgt auch eine entsprechende prophylaktische Verabreichung. Beispielsweise werden Magnesiumpräparate gegeben, um Krämpfe zu mildern und das Risiko von Herzinfarkten zu vermindern. Ferner zeigte es sich, dass bei der Verabreichung von magnesiumhaltigen Präperaten an schwangere Frauen die Häufigkeit eines Abortus vermindert werden kann.

Bei der Durchführung einer Magnesiumtherapie ist es wichtig, den Magnesiumgehalt des Blutes zu kontrollieren, um das Ansprechen des Patienten auf die Magnesiumtherapie überprüfen zu können.

Die erfindungsgemässen ionenselektiven Teile, welche als ionenselektive Komponente eine Verbindung mit 6 Carbonsäure-amidgruppen pro Molekül, enthalten, ermoglichen die Bestimmung der Magnesiumkonzentration, beziehungsweise Aktivitäten in Lösungen, die einen relativ niedrigen pH-Wert aufweisen, beispielsweise einen pH-Wert von 5. Mit Hilfe derartiger ionenselektiver Vorrichtungen kann aufgrund der guten Selektivität für Magnesiumionen gegenüber Calciumionen die Magnesiumhärte in Wasserproben bestimmt werden und die Magnesiumkonzentration in biologischem Material, wie Körperflüssigkeiten, gemessen werden.

Die Herstellung ionenselektiver Teile unter Verwendung einer ionenselektiven Komponente, welche für das zu bestimmende Anion oder Kation eine Selektivität besitzt, ist seit langem bekannt. Im allgemeinen enthalten derartige ionenselektive Teile die ionenselektive Komponente, eingebettet in der Matrix eines Polymermaterials, meistens ist ausserdem ein Weichmacher anwesend und allenfalls enthalten entsprechende ionenzelektive Teile ausserdem noch einen Ionenaustauscher. Auch die erfindungsgemässen gegenüber Msgnesiumionen selektiven Teile enthalten das gegenüber Magnesiumionen selektive Carbonsäure-amid, im allgemeinen eingebettet in einem Polymermaterial, wobei als Polymermaterialien Polyvinylhalogenid-homopolymerisate oder -copolymerisate, beziehungsweise Polyvinylidenhalogenid-homopolymerisate oder -copolymerisate bevorzugt sind. Besonders bevorzugte Polymerkomponenten sind Homopolymerisate oder Copolymerisate des Vinylchlorids, beispielsweise ein Polyvinylchloridhomopolymerisat oder ein Copolymerisat aus grösseren Anteilen von Vinylchlorid mit geringeren Anteilen an Vinylalkohol.

Als weitere Komponente enthalten die entsprechenden ionenselektiven Teile im allgemeinen einen Weichmacher, vorzugsweise einen Weichmacher der ausreichend lipophile Eigenschaften aufweist.

Als Beispiele für verwendbare Weichmacher seien Aetherweichmacher, beispielsweise o-Nitrophenyl-octyläther und Esterweichmacher genannt, vorzugsweise solche auf Basis eines Dicarbonsäurediesters oder eines Tetracarbonsäuretetraesters. Als Beispiele für derartige Dicarbonsäurediesterweichmacher seien die entsprechenden Ester der Sebacinsäure oder Adipinsäure genannt, und als Beispiele für Tetracarbonsäuretetraester seien diejenigen der Benzophenontetracarbonsäure und der Benzhydroltetracarbonsäure erwähnt. Die Alkoholkomponante derartiger Esterweichmacher ist im allgemeinen ein längerkettiger aliphatischer Alkohol, beispielsweise einer mit 5 - 15 Kohlenstoffatomen.

Häufig enthalten die erfindungsgemässen ionenselektiven Teile als weitere Komponente noch einen Ionenaustauscher, beispielsweise ein Tetraphenylborat, das in den Benzolkernen gegebenenfalls Substituenten trägt, wie zum Beispiel p-Chlortetraphenylborat oder ein Salz dieser Tetraphenylborate.

Bevorzugte erfindungsgemässe ionenselektive Teile sind ionenselektive Membranen von ionenselektiven Elektroden und diese Membranen haben im allgemeinen die folgende Zusammensetzung:
0,1 - 2 Gew.-Teile der magnesiumselektiven Komponente,
28 - 38 Gew.-Teile Polyvinylchlorid,
61 - 71 Gew.-Teile Weichmacher.

Wenn die entsprechenden ionenselektiven Membranen als weitere Komponente einen Ionenaustauscher enthalten, beispielsweise die oben genannten Tetraphenylborate oder deren Salze, dann sind pro 1OO Mol-% der magnesiumselektiven Komponente vorzugsweise 70 Mol-% bis 170 Mol-% des Ionenaustauschers, speziell bevorzugt 120 bis 160 Mol-% des Ionenaustauschers vorhanden.

Die erfindungsgemässen Testvorrichtungen können beispielsweise auch in Form von Teststreifen vorliegen, welche als magnesiumselektive Komponente die erfindungsgemässen Carbonsäure-amide enthalten, welche pro Molekül drei Dicarbonsäurediamideinheiten der Formel I, I' und I'' enthalten bispielsweise solche der Formel III oder IX. Die entsprechenden Testvorrichtungen, beispielsweise Teststreifen, enthalten das magnesiumselektive Carbonsäure-amid, vorzugsweise eingebettet in einem Trägermaterial, beispielsweise in einem Polymermaterial. Ueblicherweise enthalten entsprechende Testvorrichtungen, beispielsweise Teststreifen, als weitere Komponente einen Indikator, der seine optischen Eigenschaften ändert, wenn das in diesem Teststreifen enthaltene Carbonsäure-amid mit den Magnesiumionen der Testlösung Komplexe bildet, und zwar aufgrund der Freisetzung von Protonen bei der Komplexbildungsreaktion zwischen dem Carbonsäure-amid und dem Magnesiumion. Ueblicherweise sind solche in den Teststreifen enthaltene Indikatoren entsprechende pH-Indikatoren, beispielsweise lipophile Eigenschaften aufweisende pH-Indikatoren. Durch die Aenderung der Farbe des entsprechenden pH-Indikators ist eine quantitative oder halbquantitative Bestimmung der Magnesiumionen in der Probelösung möglich.

Die Erfindung sei nun anhand von Beispielen näher erläutert:

### Beispiel 1

Es wurde ein Carbonsäure-amid der weiter vorne angegebenen Formel III hergestellt, wobei in dieser Formel jeder der Reste
R₃ und R₄ eine Gruppierung der Formel

-(CH₂)ₙ-

war, in welcher n 6 war,
R₄' und R₃'' Wasserstoffatome bedeuten,
jeder der Reste
R₁, R₁' und R₁'' ein n-Heptylrest war und jeder der Reste
R₂, R₂' und R₂'' eine Methylgruppe bedeuteten,
Dieses neue Carbonsäure-amid entsprach also der folgenden Formel IIIa

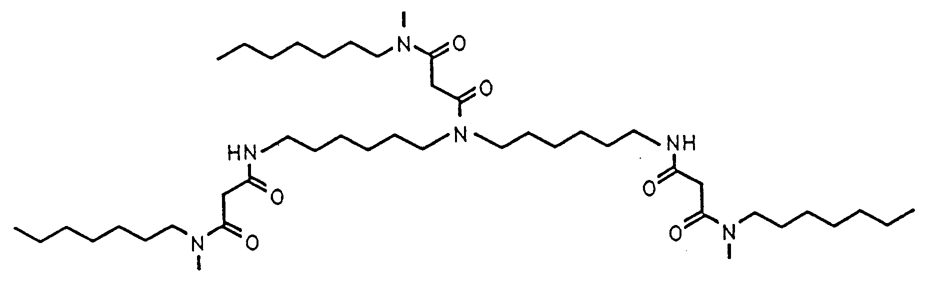

### A) Herstellung des 4-Nitrophenylesters des Malonsäure-amides, in welchem das amidbildende Amin n-Heptylmethyl-amin ist.

Die Herstellung dieses aktiven Esters des Malonsäureamides erfolgte ausgehend von dem Malonsäuremonomethylesterchlorid und dem n-Heptyl-methylamin. Dabei bildete sich als Zwischenprodukt der Methylester des entsprechenden Malonsäureamides, welcher zu der freien Säure verseift und schliesslich mit 4 - Nitrophenol in Anwesenheit von NN-Dicyclohexylcarbodiimid zu dem im Titel genannten aktiven Ester des Malonsäureamides verestert wurde.

Bezüglich näherer Einzelheiten dieses Herstellungsverfahrens sei auf die Seiten 284 und 285 der bereits früher genannten Veröffentlichung von M. Müller, M.V. Rouilly, B. Rusterholz, M. Maj-Żurawska, Z. Hu und W. Simon in Mikrochim. Acta [Wien], III, 1988, verwiesen.

### B) Herstellung des Carbonsäure-amides der Formel IIIa.

Das Triamin der Formel 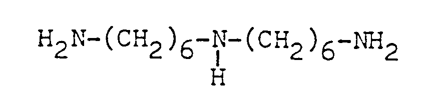
wurde von der Firma Aldrich, USA, bezogen. Dieses Triamin wurde mit dem gemäss Verfahrensschritt A hergestellten aktiven Ester des Malonsäureamides im Molverhältnis 1:3 umgesetzt. Die Umsetzung wurde in Toluol durchgeführt, zunächst rührte man 30 Minuten bei Zimmertemperatur und dann erhitzte man eine Stunde auf 110° C.

Anschliessend wurde die organische Phase mit 0,1 normaler Natronlauge gewaschen, getrocknet und im Vakuum zur Trockene eingedampft. Der verbleibende Rückstand wurde durch eine Flash-Chromatographie auf einer Silikagelsäule gereinigt, wobei als Laufmittel eine Mischung aus Methylenchlorid und Aceton im Volumenverhältnis von 4:1 verwendet wurde.

Die Ausbeute betrug 16% der Theorie.

Die Elementaranalyse des Endproduktes ergab die folgenden Werte:

| | | | |
|---|---|---|---|
| berechnet: | C = 66,96 | H = 10,74 | N = 10,41 |
| gefunden: | C = 66,69 | H = 10,84 | N = 10,39 |

Das Infrarotspektrum, aufgenommen in Chloroform, zeigte Peaks bei 3290, 1662 und 1627 cm⁻¹.

Das Massenspektrum ergab:
807 (6% (M + 1)⁺), 198 (11%), 156 (18%), 128 (18%).

Das kernmagnetische Resonanzspektrum ergab die folgenden Werte:
¹H-NMR (300 MHz, CDCl₃): 0,82-0,94 (m, 9H, 3(CH₂)₆ CH₃); 1,18 - 1,42 (m, 32H, 16 CH₂); 1,42 - 1,64 (m, 14H, 7 NCH₂ CH₂); 2,925, 2,942, 2,944, 3,022, 3,049 (5s, 9H, 3 NCH₃); 3,18 - 3,41 (m, 18H, 2 HNCOCH₂, 2HNCH₂, 2 NCH₂, 3 CH₃NCH₂); 3,45 (s, 2H, NCOCH₂); 7,88 - 7,98 (br, 1H, NH); 7,98 - 8,08 (br, 1H, NH).

Anhand der nachfolgenden Beispiele 2 und 3 werden erfindungsgemässe magnesiumselektive Membranen und die bei deren Verwendung erzielten Ergebnisse erläutert.

### Beispiel 2

### Herstellung von ionenselektiven Membranen

Unter Verwendung des gemäss Beispiel 1 hergestellten Carbonsäure-amides wurden ionenselektive Membranen hergestellt. Die Herstellung der Membranen erfolgte nach dem Verfahren, das in der Veröffentlichung von U. Oesch, Z. Brzozka, A. Xu, B. Rusterholz, G. Suter, H.-V. Pham, D.H. Welti, D. Ammann, E. Pretsch, W. Simon, Anal. Chem. 1986, 58, 2285 beschrieben.

Die ionenselektiven Membranen hatten die folgende Zusammensetzung:

| Bestandteil | Gewichts-% |
|---|---|
| Hexacarbonsäurehexa-amid | 1 |
| Polyvinylchlorid | 33 |
| Weichmacher | 66 |

Als Weichmacher wurde der o-Nitrophenyl-octyläther verwendet.

Die zu Testzwecken hergestellten Membranen enthielten entweder keinen Ionenaustauscher oder sie enthielten als Ionenaustauscher das Kaliumsalz des Tetraphenylborates, bei dem jeder dervier Benzolkerne in der Parastellung mit Chlor substituiert war.

Die entsprechenden getesteten Membranen enthielten pro 100 Mol-% des Carbonsäure-amides 0 Mol-%, beziehungsweise 40 Mol-%, beziehungsweise 70 Mol-%, beziehungsweise 90 Mol-%, beziehungsweise 120 Mol-%, beziehungsweise 150 Mol-%, beziehungsweise 170 Mol-% dieses Ionenaustauschers.

### Beispiel 3

Durchführung von Messungen unter Verwendung der ionenselektiven Membranen des Beispiels 2.

Es wurden entsprechende magnesiumselektive Elektroden unter Verwendung der magnesiumselektiven Membranen hergestellt, wobei die innere Ableitung der Elektrode Silber/Silberchlorid war und die Elektrodenfüllung eine mehrere Salze enthaltende Lösung war. Als Vergleichselektrode diente eine Kalomelelektrode.

Die Bestimmung der EMK (elektromotorische Kraft) wurde also mit einer Zelle des folgenden Typs durchgeführt.
Hg; Hg₂Cl₂, KCl (ges.)/3 M KCl/Probe/Membran// Elektrodenfüllung, AgCl; Ag.

Die Elektrodenfüllung war 0,14 molar an Natriumchlorid, 4 x 10⁻³ molar an Kaliumchlorid,
10⁻³ molar an Calciumchlorid und 6 x 10⁻⁴ molar an Magnesiumchlorid.

Zur Bestimmung der Selektivitätskoeffizienten wurden 10⁻¹ molare wässrige Lösungen der entsprechenden Metallchloride verwendet.

Die Ergebnisse dieser Messungen sind in der Zeichnung veranschaulicht. Auf der Ordinate sind die Selektivitätsfaktoren, ausgedrückt in 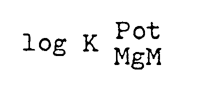
angeführt.

Auf der Abszisse dieser Figur sind die Mol-% en dem Kaliumsalz des Tetrakis-(p-chlorphenyl)-borates pro 100 Mol-% des Carbonsäure-amides ausgedrückt.

Man sieht aus dieser Zeichnung, dass diejenigen Membranen, die 90 Mol-%, beziehungsweise 120 Mol-%, beziehungsweise 150 Mol-% an dem genannten Kaliumsalz des substituierten Tetraphenylborates enthielten, eine höhere Selektivität für Magnesiumionen gegenüber den Erdalkalimetallionen Calcium und Barium, sowie gegenüber sämtlichen Alkalimetallionen und auch gegenüber Ammoniumionen hatten. Besonders gut geeignet, waren die Membranen mit 120 Mol-% und diejenigen mit 150 Mol-% dieses Kationenaustauschers pro 100 Mol-% der ionenselektiven Komponente, denn diese Membranen zeigten nicht nur eine deutlich höhere Selektivität für Magnesiumionen als für Calciumionen, sondern eine extrem höhere Selektivität für Magnesiumionen als für Natriumionen. Diese hohe Selektivität für Magnesium im Vergleich zu Natrium ist ausserordentlich wichtig, weil bekanntlich in biologischen Materialien, wie Körperflüssigkeiten, sehr viel höhere Konzentrationen an Natriumionen vorliegen als an Magnesiumionen.

Wenn man diese Messergebnisse mit denjenigen vergleicht, die mit dem aminosubstituierten Bernsteinsäurediamid erzielt wurden, die auf Seite 2015 der weiter vorne genannten Veröffentlichung von M.V. Rouilly, M. Badertscher, E. Pretsch, G. Suter und W. Simon in Analytical Chemistry, Band 60, 19, Oktober 1988 veranschaulicht sind, dann sieht man, dass die getesteten Carbonsäure-amide eine wesentlich höhere Selektivität für Magnesiumionen im Vergleich zu Natriumionen aufwiesen. Noch bedeutender ist aber, dass mit den erfindungsgemässen Carbonsäure-amiden die Magnesiumbestimmung in neutralen oder schwach sauren Probelösungen durchgeführt werden kann, während die in der genannten Veröffentlichung beschriebenen ionenselektiven Membranen nur zur Bestimmung von Magnesiumionen in Probelösungen eines pH-Wertes von 8 - 9 geeignet waren.

Die Probelösungen, die bei den in der vorliegenden Figur veranschaulichten Ergebnissen eingesetzt wurden, hatten einen pH-Wert von etwa 6 während die Messungen, die auf Seite 2015 der genannten Veröffentlichung veranschaulicht sind, bei einem pH-Wert von 8,8 durchgeführt wurden.

Der in den getesteten Membranen als Weichmacher verwendete p-Nitrophenyl-octyläther ist gut geeignet für eine Verwendung der entsprechenden Membranen zur Bestimmung von Ionenkonzentrationen in Blutserum.

Im Gegensatz dazu, war es bei Verwendung der Tetracarbonsäuretetraamide, die in der weiter vorne genannten Veröffentlichung von M. Müller, M. Rouilly, B. Rusterholz, M. Maj-Żurawska, Z. Hu und W. Simon in Mikrochim. Acta [Wien], III, Seiten 283 - 290, 1988 beschrieben sind, wegen der schlechten Löslichkeit dieser magnesiumselektiven Komponente nötig, in den Membranen als Weichmacher Chlorparaffin zu verwenden. Ionenselektive Membranen, die Chlorparaffine enthalten, sind ungeeignet zur Durchführung von Messungen in Blutserum.

### Beispiel 4

Nach dem in Beispiel 1 beschriebenen Herstellungsverfahren wurden weitere Carbonsäure-amide hergestellt, welche der weiter vorne angegebenen Formel II entsprachen. Diese neuen Carbonsäure-amide wiesen die folgenden Strukturformeln A bis J auf:

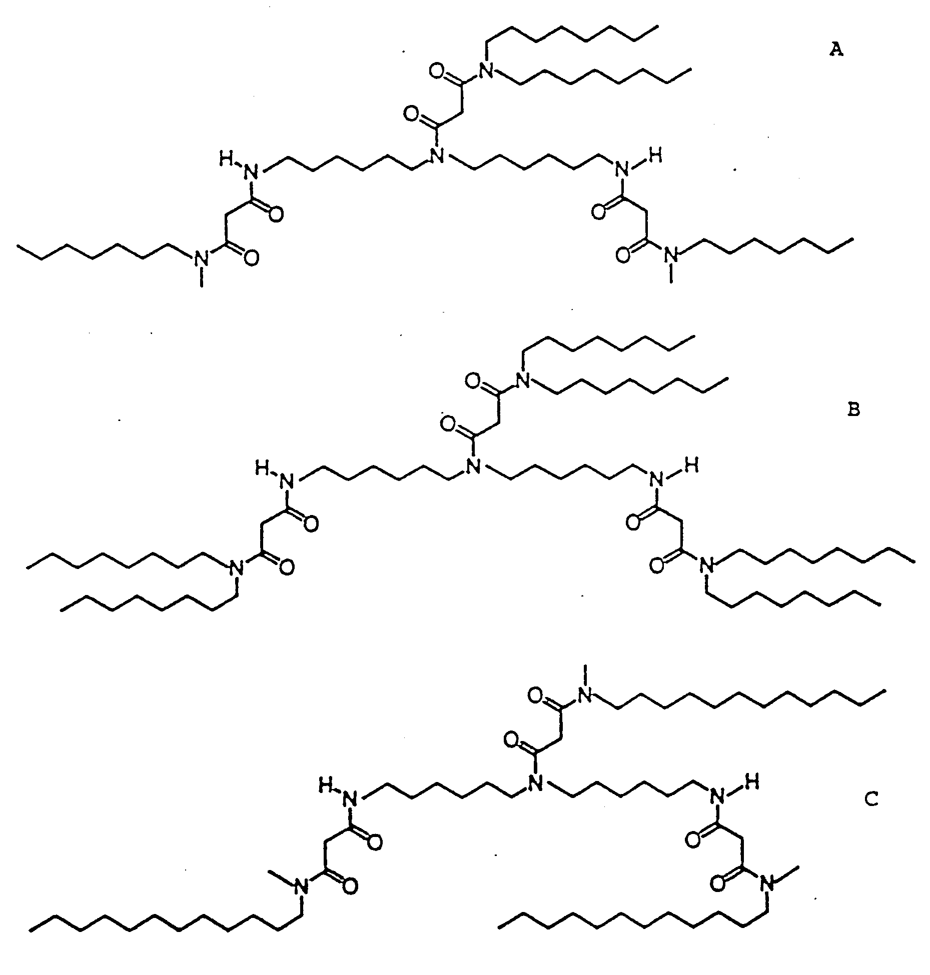

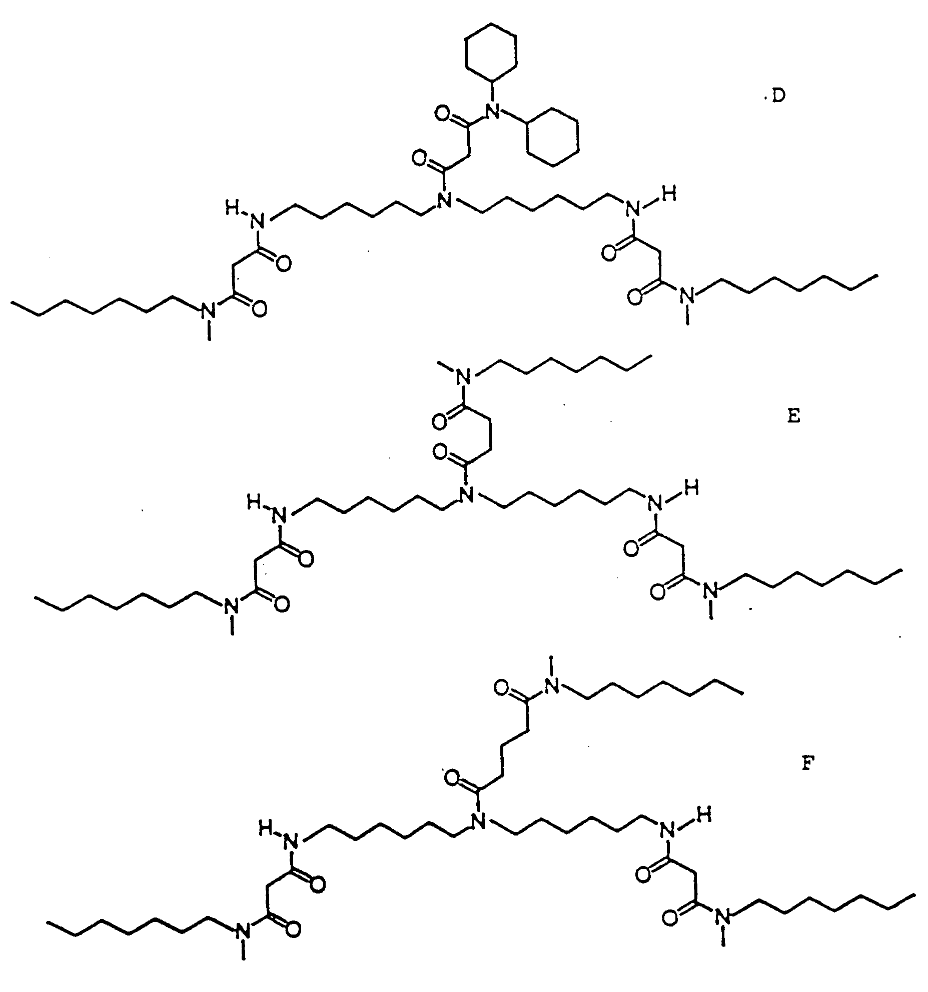

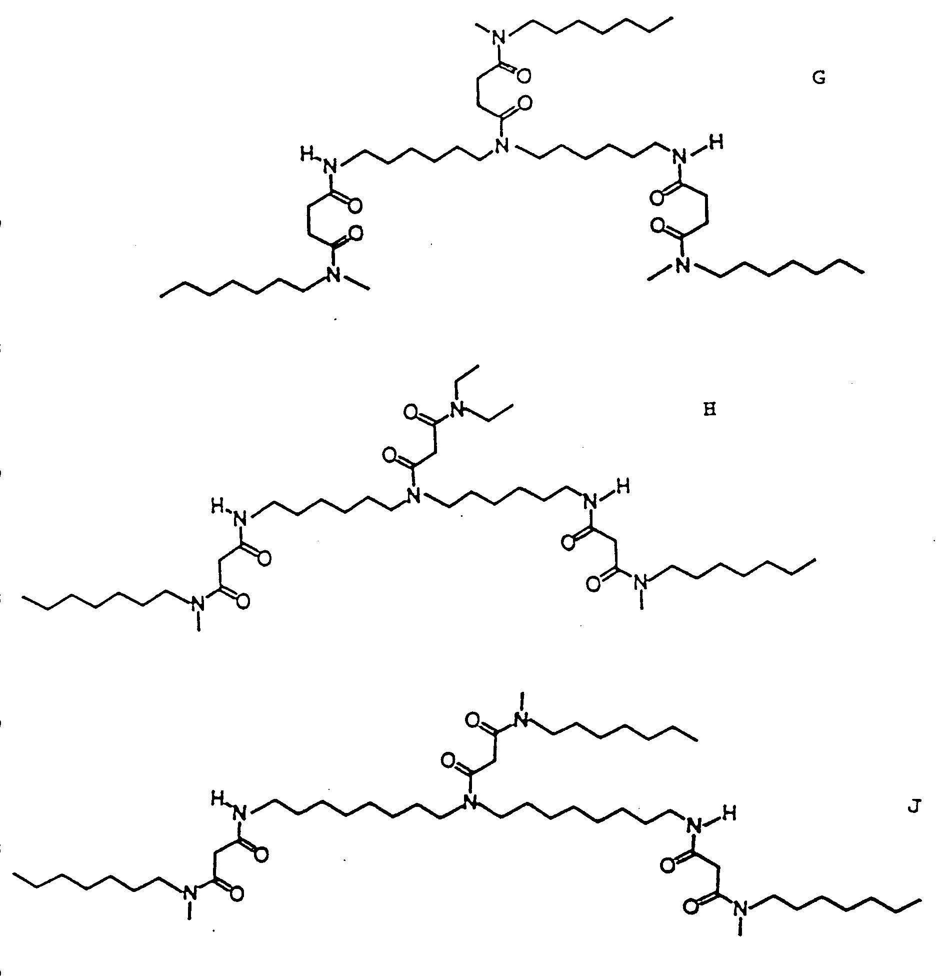

### Beispiel 5

Anhand dieses Beispiels wird die Herstellung von bevorzugten Carbonsäure-amiden erläutert, welche der weiter vorne angegebenen Formel VIII, beziehungsweise IX, entsprechen.

Diese Carbonsäure-amide wiesen die folgenden Strukturformeln K und L auf: 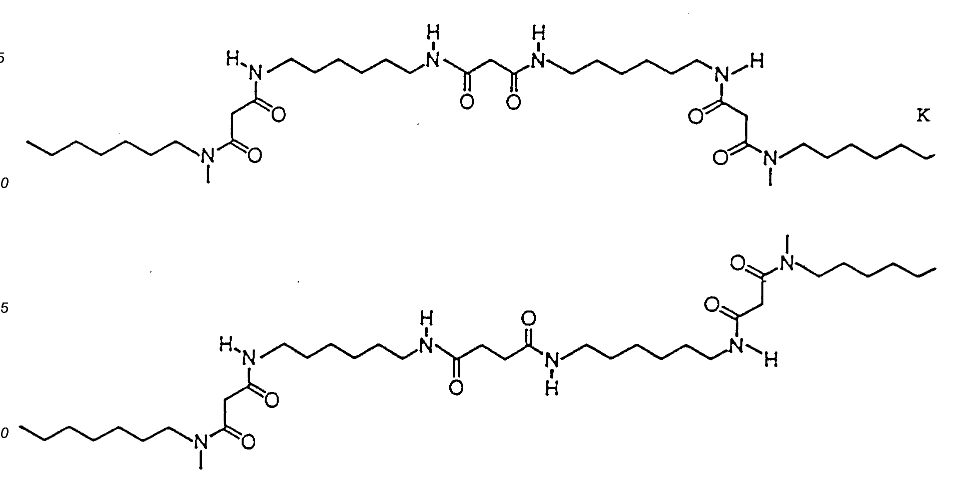
Die Verbindungen der Formeln K und L wurden nach dem weiter vorne beschriebenen Syntheseverfahren hergestellt, d.h. indem man die entsprechenden Amine mit einem reaktiven Dicarbonsäurederivat umsetzte, nämlich im Falle der Herstellung der Verbindung der Formel K mit Malonsäuredichlorid und im Falle der Herstellung der Verbindung der Formel L mit Bernsteinsäuredichlorid.

Die Umsetzung zwischen dem Dicarbonsäuredichlorid und dem Amin wurde in dem Lösungsmittel Methylenchlorid und in Anwesenheit von Triäthylamin als Mittel zur Neutralisierung der abgespaltenen Chlorwasserstoffsäure durchgeführt. Die als Ausgangsmaterial eingesetzten Amine und das Triäthylamin wurden in Methylenchlorid gelöst und zu der entsprechenden gekühlten Lösung setzte man das jeweilige Dicarbonsäuredichlorid, ebenfalls gelöst in Methylenchlorid, zu.

Nach diesem Herstellungsverfahren wurden zwei weitere Carbonsäure-amide hergestellt, die eine sehr ähnliche Struktur aufwiesen, wie die Verbindungen der Formeln K, beziehungsweise L. Diese weiteren Carbonsäure-amide unterschieden sich jedoch von den Verbindungen der Formeln K und L dahingehend, dass das Amin, welches in den beiden Endstellungen der Säureamide der Formeln K und L die Säureamidgruppierungen bildete (dieses war bei den Verbindungen der Formeln K und L das n-Heptyl-methylamin) jetzt das Di-n-octylamin war.

### Beispiel 6

Mit den in den Beispielen 4 und 5 beschriebenen magnesiumselektiven Carbonsäure-amiden wurden ionenselektive Membranen nach dem Verfahren hergestellt, das in Beispiel 2 beschrieben ist.

Mit diesen Membranen wurde die EMK (elektromotorische Kraft) in Zellen desjenigen Typs bestimmt, der in Beispiel 3 beschrieben ist.

Die entsprechenden Tests zeigten, dass die Verbindungen der Strukturformeln A, C, D, E und H, die in Beispiel 4 beschrieben sind und die Verbindung L, die in Beispiel 5 beschrieben ist, eine sehr ähnliche Selektivität für Magnesiumionen gegenüber Calciumionen, sowie für Magnesiumionen gegenüber Natriumionen, aufwiesen, wie die in Beispiel 1 beschriebene Verbindung.

Die höchste Selektivität für Magnesiumionen über Calciumionen zeigte jedoch in diesen Tests die Verbindung der Strukturformel J, die in Beispiel 4 beschrieben ist. Bei einer entsprechenden ionenselektiven Membran, die 150 Mol-% des oben erwähnten Kaliumsalzes des p-chlorsubstituierten Tetraphenylborats pro 100 Mol-% des ionenselektiven Carbonsäure-amides der Formel J enthielt, betrug die entsprechende Ionenselektivität für Magnesiumionen über Calciumionen, also der Wert 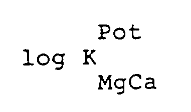
-1,0. Die entsprechende Membran zeigte ferner eine ausserordentlich hohe Selektivität für Magnesiumionen im Vergleich zu Natriumionen, denn der entsprechende Wert von 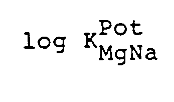
war -2,8.

Entsprechende ionenselektive Membranen, die als ionenselektive Komponente ein Carbonsäure-amid der Formel J gemäss Beispiel 4 enthalten, sind also hervorragend gut geeignet um die Aktivität, beziehungsweise Konzentration von Magnesiumionen in biologischen Materialien zu bestimmen, in denen bekanntlich hohe Konzentrationen an Natriumionen und auch höhere Konzentrationen an Calciumionen als an Magnesiumionen vorliegen.

## Patentansprüche (Patentansprüche für folgende(n) Vertragsstaat(en): AT, BE, CH, DE, DK, FR, GB, IT, LI, LU, NL, SE)

1. Carbonsäureamide, die mit Magnesiumionen lipophile Komplexe bilden, dadurch gekennzeichnet, pro Molekül 6 dass sie Carbonsäure-amidgruppen aufwiesen und dass pro Molekül der Carbonsäureamide drei Dicarbonsäurediamidgruppierungen vorliegen, welche die folgende Formel I 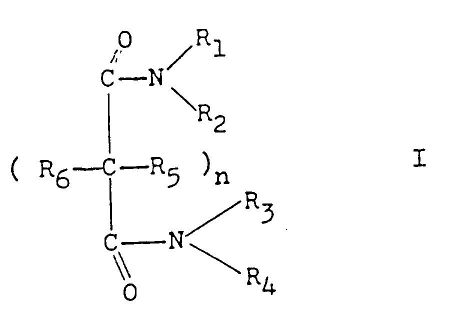 aufweisen, wobei in dieser Formel I die Reste
R₁, R₂, R₃ und R₄ unabhängig voneinander
Wasserstoffatome, gegebenenfalls substituierte Alkyl, Alkenyl oder Alkinyl-Reste gegebenenfalls substituierte Cycloalkylreste oder gegebenenfalls substituierte aromatische oder heterocyclische Reste oder Kombinationen aus derartigen Resten darstellen,
R₅ und R₆ Wasserstoffatome, Alkylgruppen mit 1 - 4 Kohlenstoffatomen oder Halogenatome sind und
n 0 oder eine ganze Zahl im Bereich von 1 - 3 ist,
wobei jedoch mindestens einer der Reste R₁, R₂, R₃, R₄, R₅ und R₆ gemeinsam mit zwei entsprechenden Resten R₁', R₂', R₃', R₄', R₅', R₆', R₁'', R₂'', R₃'', R₄'', R₅'' oder R₆'' der beiden weiteren Dicarbonsäurediamidstrukturen der entsprechenden Formel I', beziehungsweise I'' einen mehrwertigen aliphatischen, alicyclischen, aromatischen oder heteroaromatischen oder eine Kombination aus derartigen Resten darstellt, durch welchen die Bindung der Dicarbonsäurediamidgruppierung der Formel I über ein Stickstoffatom derselben oder ein Kohlenstoffatom derselben an ein Stickstoffatom oder ein Kohlenstoffatom einer weiteren Dicarbonsäurediamidstruktur einer entsprechenden Formel I', beziehungsweise I'', der verbindung mit 6 unter Ausbildung Carbonsäure-amidgruppen pro Moleküle erreicht wird und wobei die Gesamtstruktur der Verbindung mit 6 Carbonsäure-amidegruppen offenkettig oder cyclisch ist, und wobei
gegebenenfalls entweder der Rest R₁ und R₂ gemeinsam mit dem Stickstoffatom, an welches die beiden Reste gebunden sind, oder der Rest R₃ und R₄ gemeinsam mit dem Stickstoffatom, an welches die beiden Reste R₃ und R₄ gebunden sind, einen gegebenenfalls noch weitere Heteroatome aufweisenden heterocyclischen Rest bilden oder gegebenenfalls der Rest R₁ oder der Rest R₂ des einen der beiden Stickstoffatome der Dicarbonsäurediamidgruppierung der Formel I gemeinsam mit dem Rest R₃ oder R₄ des anderen der beiden Stickstoffatome der Dicarbonsäuredismidgruppierung der Formel I diese Dicarbonsäurediamidgruppierung unter Ausbildung einer cyclischen Struktur ringschliesst.

2. Carbonsäureamide gemäss Anspruch 1, dadurch gekennzeichnet, dass zwei der Reste R₁, R₂, R₃ und R₄ gemeinsam mit einem entsprechenden Rest R₁', R₂', R₃' und R₄' und gemeinsam mit einem Rest R₁'', R₂'', R₃'' und R₄'' der beiden weiteren Dicarbonsäurediamidstrukturen der entsprechenden Formel I', beziehungsweise I'', einen zweiwertigen, aliphatischen, alicyclischen, aromatischen oder heteroaromatischen oder eine Kombination aus derartigen Resten darstellen, durch welche die Bindung der Dicarbonsäurediamidgruppierung der Formel I über ein Stickstoffatom derselben an eine weitere Dicarbonsäurediamidstruktur der entsprechenden Formeln I' und I'' unter Ausbildung der Verbindung mit sechs Carbonsäureamidgruppen pro Molekül erreicht wird wobei die Gesamtstruktur der Verbindung mit sechs Carbonsäureamidgruppen offenkettig oder cyclisch ist.

3. Carbonsäureamide gemäss Anspruch 1 oder 2, dadurch gekennzeichnet, dass sie die folgende Formel II 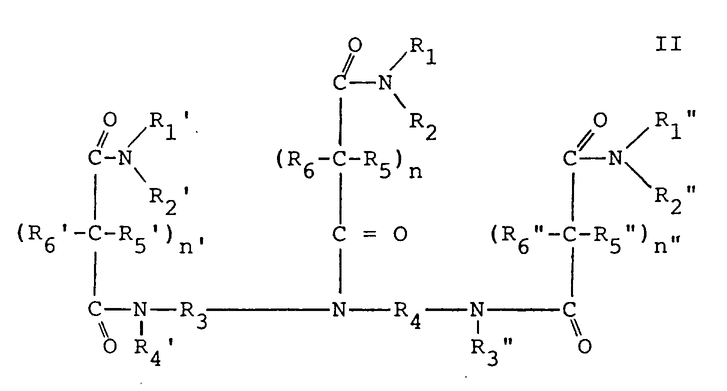 aufweisen, wobei in dieser Formel die Reste
R₃ und R₄ unabhängig voneinander zweiwertige aliphatische, alicyclische, aromatische, heteroaromatische oder eine Kombination aus derartigen Reste darstellen, die Reste und Symbole R₁', R₂', R₄', R₅', R₆', R₁'', R₂'', R₃'', R₅'', R₆'', n' und n'' die gleiche Bedeutung aufweisen, wie die Reste und Symbole R₁, R₂, R₃, R₄, R₅, R₆ und n in den Dicarbonsäurediamidgruppierungen der Formel I und wobei gegebenenfalls der Rest R₂ und/oder der Rest R₁, zusammen mit dem Rest R₁' oder R₂' oder R₄' oder R₃'' oder R₂'' oder R₁'' einen zweiwertigen Rest darstellt und/oder der Rest R₁' und/oder der Rest R₂' gemeinsam mit dem Rest R₃'' oder dem RestR₂'' oder dem Rest R₁'' einen zweiwertigen Rest darstellt und/oder
der Rest R₁'' oder der Rest R₂'' gemeinsam mit dem Rest R₄' einen zweiwertigen Rest darstellt und wobei
die genannten zweiwertigen Reste aliphatische, alicyclische, aromatische oder heteroaromatische Reste oder eine Kombination aus derartigen Resten darstellen, welche die Carbonsäureamide der Formel II zu einer monocyclischen, bicyclischen, tricyclischen oder polycyclischen Verbindung ringschliessen.

4. Carbonsäureamide gemäss Anspruch 1 oder 2, dadurch gekennzeichnet, dass diese Carbonsäureamide die folgende Formel VIII 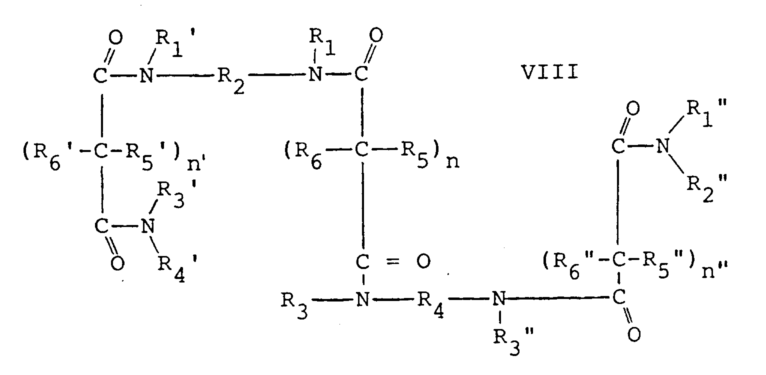 aufweisen, in welcher die Reste
R₂ und R₄ unabhängig voneinander zweiwertige aliphatische, alicyclische, aromatische oder heteroaromatische Reste oder Kombinationen aus derartigen Resten darstellen und die Reste
R₁, R₁', R₁'', R₂'', R₃, R₃', R₄' und R₃'' die gleiche Bedeutung aufweisen, wie in Anspruch 1 im Zusammenhang mit den entsprechenden Dicarbonsäurediamideinheiten der Formel I, beziehungsweise den entsprechenden Einheiten der Formeln I' und I'', definiert wurde, oder
der Rest R₁'', zusammen mit dem Rest R₂'' und/oder
der Rest R₃', zusammen mit dem Rest R₄' und/oder
der Rest R₁, zusammen mit dem Rest R₃ und den entsprechenden Stickstoffatomen, an welche diese Reste gebunden sind, einen heterocyclischen Ring darstellen, R₅, R₆, R₅', R₆', R₅'' und R₆'' die gleiche Bedeutung aufweisen, wie in Anspruch 1 im Zusammenhang mit den Dicarbonsäurediamideinheiten der Formel I, beziehungsweise den entsprechenden Einheiten der Formeln I' und I'', definiert wurde, oder
der Rest R₃ und/oder der Rest R₁ zusammen mit dem Rest R₁', R₃', R₄', R₃'', R₂'' oder R₁'' einen zweiwertigen Rest darstellt und/oder
der Rest R₃' und/oder der Rest R₄' zusammen mit dem Rest R₃'' oder R₂'' oder R₁'' einen zweiwertigen Rest bildet und/oder
der Rest R₁'' oder der Rest R₂'' zusammen mit dem Rest R₄' einen zweiwertigen Rest darstellt und
wobei die genannten zweiwertigen Reste aliphatische, alicyclische, aromatische oder heteroaromatische Reste oder eine Kombination aus zwei oder mehr derartigen Resten darstellen, durch welche die Carbonsäure-amide der Formel VIII ringgeschlossen sind, wobei sich ein entsprechendes Carbonsäure-amid bildet, welches eine monocyclische, bicyclische, tricyclische oder polycyclische Struktur aufweist.

5. Carbonsäureamide gemäss einem der Ansprüche 1 bis 4, dadurch gekennzeichnet, dass in den drei Dicarbonsäurediamidgruppierungen der Formel I, beziehungsweise I', beziehungsweise I'', jeweils n, n', beziehungsweise n'' die Zahl 1 oder 2 bedeutet und dass die Reste R₅, R₆, R₅', R₆', R₅'' und R₆'' vorzugsweise die Bedeutung von Wasserstoffatomen oder Methylgruppen aufweisen, speziell bevorzugt die Bedeutung von Wasserstoffatomen.

6. Carbonsäureamide gemäss einem der Ansprüche 1 bis 5, dadurch gekennzeichnet, dass sie in ihrem Molekül mindestens einen gegebenenfalls substituierten Alkyl, Alkenyl oder Alkinyl-Rest aufweisen, der mindestens 4, vorzugsweise 5 - 15, und speziell bevorzugt, 6 - 12 Kohlenstoffatome aufweist und/oder dass die Stickstoffatome zweier unterschiedlicher Carbonsäureamidgruppen dieser Carbonsäure-amide miteinander durch eine gegebenenfalls substituierte zweiwertige aliphatische Gruppe, die gegebenenfalls eine oder zwei Doppelbindungen oder Dreifachbindungen aufweist, verbunden sind, wobei diese aliphatische Gruppe eine Kettenlänge von mindestens 4 Kohlenstoffatomen, vorzugsweise eine Kettenlänge von 5 - 15 Kohlenstoffatomen, speziell bevorzugt 6 - 12 Kohlenstoffatomen, aufweist.

7. Carbonsäureamide gemäss einem der Ansprüche 3 - 6, dadurch gekennzeichnet, dass in der Formel II die Reste R₃ und R₄, beziehungsweise in der Formel VIII die Reste R₂ und R₄ Alkylengruppen einer Kettenlänge von mindestens 4 Kohlenstoffatomen sind, vorzugsweise unverzweigte Reste der Formel
-(CH₂)_{z}- ,
in welchen z eine ganze Zahl im Bereich von 5 - 15, speziell bevorzugt, 6 - 12, ist,
und dass die Carbonsäure-amide der Formel II, beziehungsweise VIII, verzugsweise nicht cyclische Verbindungen sind, wobei in den entsprechenden Verbindungen der Formel II, sowohl der Rest R₁ als auch der Rest R₁' und auch der Rest R₁'' eine Alkylgruppe mit einer Kettenlänge von mindestens 4 Kohlenstoffatomen, vorzugsweise eine Alkylkette mit 5 - 15 Kohlenstoffatomen, speziell bevorzugt mit 6 - 12 Kohlenstoffatomen ist und dass in den nicht cyclischen Verbindungen der Formel VIII sowohl der Rest R₃' als auch der Rest R₁'' eine Alkylgruppe mit einer Kettenlänge von mindestens 4 Kohlenstoffatomen, vorzugsweise eine Alkylgruppe mit 5 - 15 Kohlenstoffatomen, speziell bevorzugt mit 6 - 12 Kohlenstoffatomen, ist.

8. Carbonsäureamide gemäss einem der Ansprüche 3, beziehungsweise 5 bis 7, dadurch gekennzeichnet, dass sie die folgende Formel III 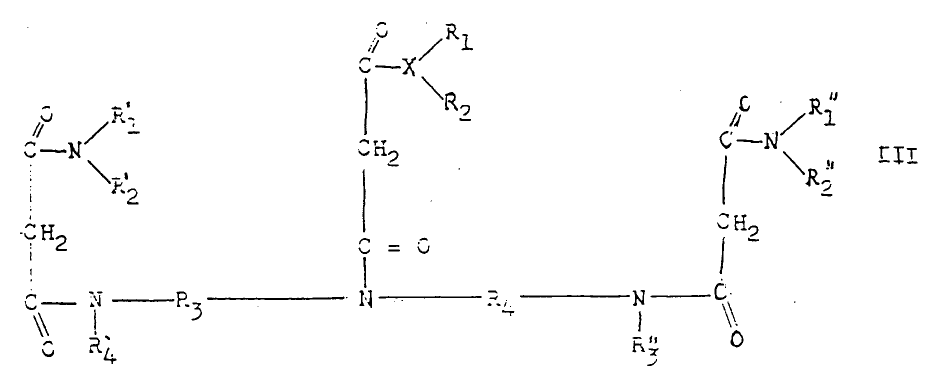 aufweisen, wobei in dieser Formel die Reste
R₃ und R₄ eine Gruppierung der Formel
-(CH₂)_{z}-
bedeuten, in welcher z 5, 6, oder 7 ist, wobei vorzugsweise in beiden Resten z die gleiche Bedeutung aufweisen und speziell bevorzugt, 6 sind,
R₄' und R₃'' Wasserstoff oder einen Alkylrest mit 1 - 4 Kohlenstoffatomen bedeuten,
die Reste
R₁, R₁' und R₁'' Alkylreste mit 5 - 9 Kohlenstoffatomen darstellen, wobei die Reste R₁, R₁' und R₁'' vorzugsweise miteinander identisch sind und speziell bevorzugt entsprechende geradkettige Alkylreste darstellen und die Reste
R₂, R₂' und R₂'' vorzugsweise miteinander identisch sind und speziell bevorzugt die
Bedeutung eines Wasserstoffatomes oder einer Alkylgruppe mit 1 - 4 Kohlenstoffatomen, insbesondere die Bedeutung einer Methylgruppe aufweisen.

9. Carbonsäureamide gemäss einem der Ansprüche 4 - 7, dadurch gekennzeichnet, dass sie die folgende Formel IX 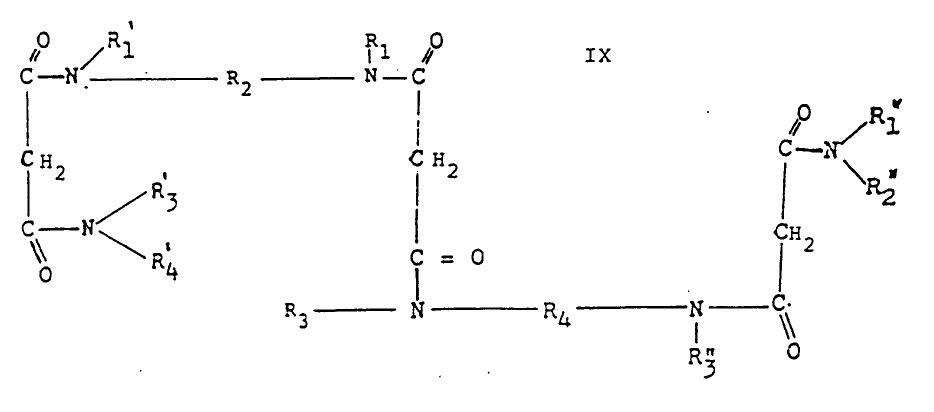 aufweisen, wobei in dieser Formel IX die Reste
R₂ und R₄ unabhängig voneinander Gruppen der Formel
-(CH₂)_{z}-
darstellen, in welcher
z eine ganze Zahl im Bereich von 5 - 9 ist,
und die Reste
R₄', R₁, R₃ R₃', R₁' R₁'' R₃'' und R₂'' unabhängig voneinander Wasserstoffatome oder Alkylgruppen mit 1 - 12 Kohlenstoffatomen darstellen.

10. Lipophile Komplexe aus Magnesiumionen und den pro Molekül 6 Carbonsäure-amidgruppen aufweisenden Verbindungen gemäss einem der Ansprüche 1 - 9, vorzugsweise lipophile Komplexe aus einem Mol Magnesiumionen und einem Mol der Carbonsäure-amide.

11. Verfahren zur Herstellung eines lipophilen Komplexes aus Magnesiumionen dadurch gekennzeichnet, dass man die Magnesiumionen mit den pro Molekül 6 Carbonsäure-amidgruppen aufweisenden Verbindungen gemäss einem der Ansprüche 1 bis 9 in Berührung bringt, wobei sich die entsprechenden lipophilen Magnesiumkomplexe bilden, vorzugsweise 1:1 Komplexe aus einem Mol Magnesiumionen und einem Mol Carbonsäure-amid.

12. Testvorrichtung zur Bestimmung der Aktivität, beziehungsweise Konzentration, von Magnesiumionen in flüssigen Proben, dadurch gekennzeichnet, dass sie als magnesiumselektive Komponente eine pro Molekül 6 Carbonsäure-amidgruppen aufweisende Verbindung, gemäss einem der Ansprüche 1 - 9 enthält.

13. Testvorrichtung gemäss Anspruch 12, dadurch gekennzeichnet, dass sie die magnesiumselektive Verbindung in einem oder auf einem inerten Trägermaterial enthält, vorzugsweise in einem Polymermaterial.

14. Testvorrichtung gemäss Anspruch 12 oder 13, dadurch gekennzeichnet, dass sie ein ionenselektiver Teil ist, der das Carbonsäure-amid, eingebettet in einem Polymermaterial, enthält, vorzugsweise einem Polyvinylhalogenidhomopolymerisat oder Polyvinylhalogenidcopolymerisat.

15. Testvorrichtung gemäss Anspruch 14, dadurch gekennzeichnet, dass sie als weitere Komponente einen Weichmacher enthält, vorzugsweise einen Aetherweichmacher, beispielsweise o-Nitrophenyl-octyläther, oder einen Esterweichmacher, vorzugsweise auf Basis eines Dicarbonsäurediesters oder eines Tetracarbonsäuretetraesters, beispielsweise eines Benzophenontetracarbonsäuretetraesters oder Benzhydroltetracarbonsäuretetraesters und dass der ionenselektive Teil gegebenenfalls noch eine als Ionenaustauscher wirkende Komponente enthält, beispielsweise Tetraphenylborat, in welchem die Benzolkerne gegebenenfalls Substituenten tragen oder Salze dieser Borate.

16. Verfahren zur Herstellung der Carbonsäureamide gemäss Anspruch 3, welche pro Molekül sechs Carbonsäureamidgruppen aufweisen und die folgende Formel II 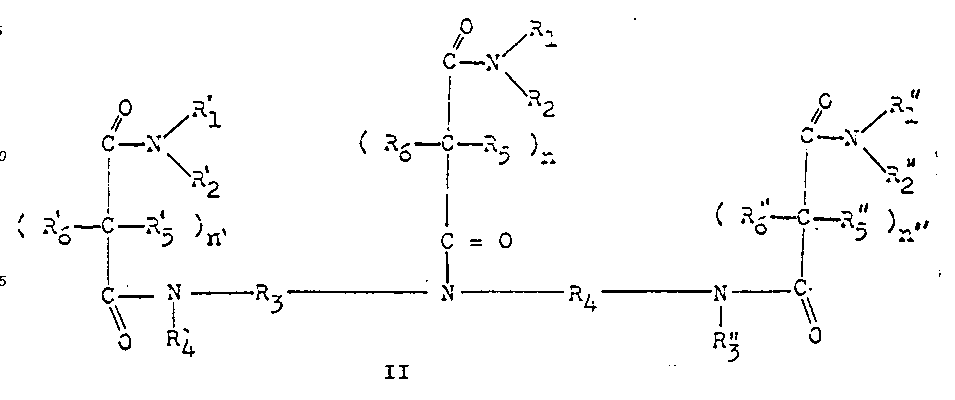 besitzen, wobei in dieser Formel II die Reste
R₃ und R₄, sowie die Reste R₁', R₂', R₄', R₅', R₆', R₁, R₂, R₅, R₆, R₁'', R₂'', R₃'', R₅'', R₆'' und die Symbole n, n' und n'' die gleiche Bedeutung aufweisen, wie im Anspruch 3, indem man ein Triamin der Formel IV 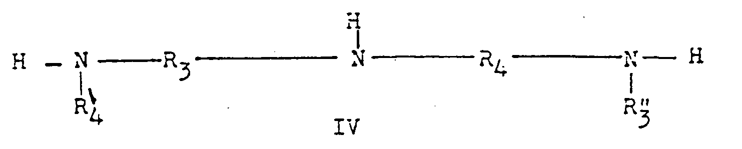 mit Carbonsäuren der folgenden Formeln Va, Vb und Vc 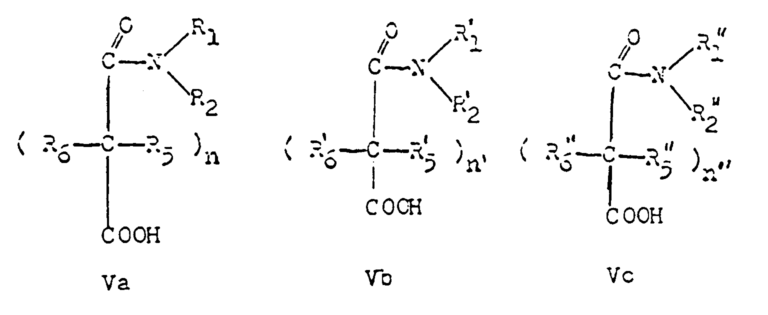 oder reaktiven Derivaten dieser Carbonsäuren zu dem entsprechenden Carbonsäureamid der Formel II umsetzt, oder indem man das Amin der Formel IV zunächst mit Dicarbonsäuren der Formeln VIa, VIb und VIc 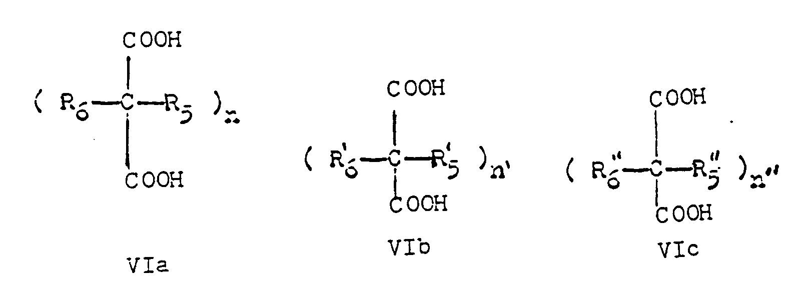 oder reaktiven Derivaten dieser Dicarbonsäurenumsetzt, wobei gegebenenfalls die eine der beiden Carboxylgruppen der Dicarbonsäuren, beziehungsweise der reaktiven Dicarbonsäurederivate, in geschützter Form vorliegt, und wobei man anschliessend das erhaltene Zwischenprodukt der Formel VII 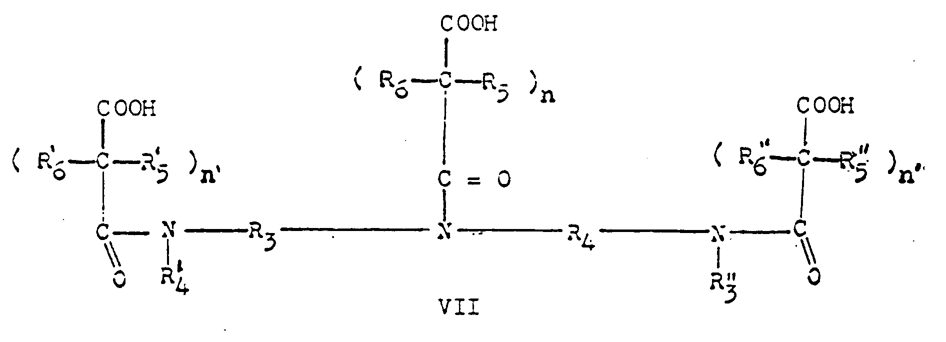 oder ein reaktives Derivat dieses Zwischenproduktes der Formel VII mit den entsprechenden Aminen zu dem gewünschten Endprodukt der Formel II umsetzt.

17. Verfahren zur Herstellung der Carbonsäureamide gemäss Anspruch 4, welche pro Molekül sechs Carbonsäureamidgruppen aufweisen und die folgende Formel VIII 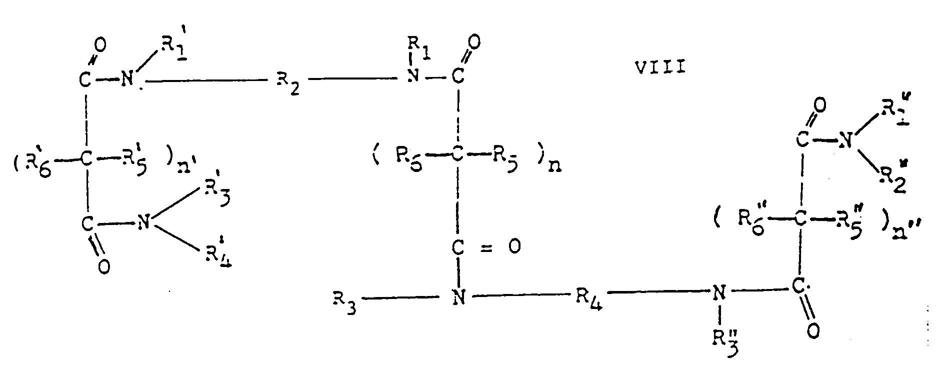 besitzen, wobei in dieser Formel VIII
R₁', R₃', R₄', R₅', R₆', R₁, R₃, R₅, R₆, R₁'', R₂'', R₃'', R₅'' und R₆'', sowie die Symbole n, n' und n'' die gleiche Bedeutung haben, wie im Anspruch 4,
indem man zwei Aminoverbindungen 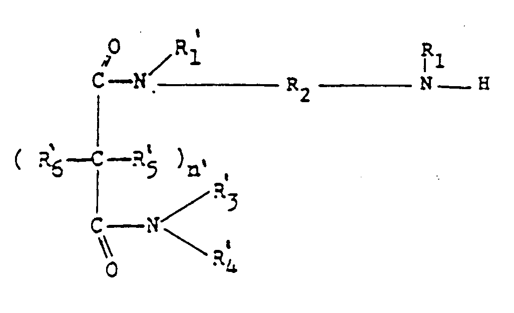 und 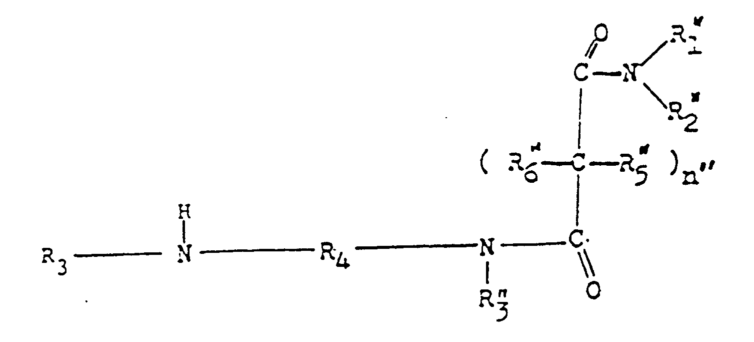 mit einer Dicarbonsäure der Struktur 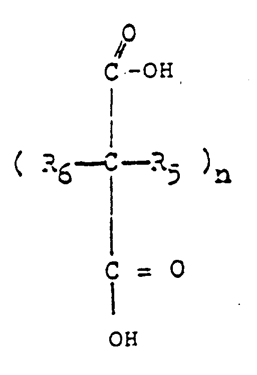 oder einem reaktiven Derivat dieser Dicarbonsäure umsetzt, wobei man das Carbonsäureamid der Formel VIII erhält.

## Patentansprüche (Patentansprüche für folgende(n) Vertragsstaat(en): ES)

1. Verfahren zur Herstellung von lipophilen Magnesiumkomplexen, dadurch gekennzeichnet, dass man Magnesiumionen mit Carbonsäureamiden umsetzt, die pro Molekül sechs Carbonsäureamidgruppen aufweisen und in denen pro Molekül dieser Carbonsäureamide drei Dicarbonsäurediamidgruppierungen vorliegen, welche die folgende Formel I 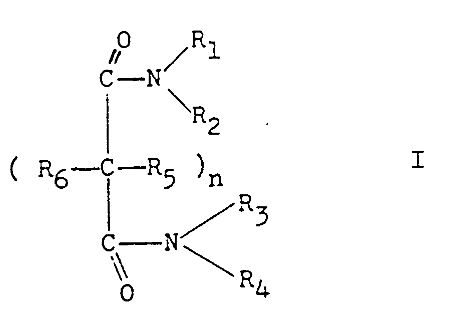 aufweisen, wobei in dieser Formel I die Reste
R₁, R₂, R₃ und R₄ unabhängig voneinander
Wasserstoffatome, gegebenenfalls substituierte Alkyl, Alkenyl oder Alkinyl-Reste gegebenenfalls substituierte Cycloalkylreste oder gegebenenfalls substituierte aromatische oder heterocyclische Reste oder Kombinationen aus derartigen Resten darstellen,
R₅ und R₆ Wasserstoffatome, Alkylgruppen mit 1 - 4 Kohlenstoffatomen oder Halogenatome sind und
n 0 oder eine ganze Zahl im Bereich von 1 - 3 ist,
wobei jedoch mindestens einer der Reste R₁, R₂, R₃, R₄, R₅ und R₆ gemeinsam mit zwei entsprechenden Resten R₁', R₂', R₃', R₄', R₅', R₆', R₁'', R₂'', R₃'', R₄'', R₅'' oder R₆'' der beiden weiteren Dicarbonsäurediamidstrukturen der entsprechenden Formel I', beziehungsweise I'' einen mehrwertigen aliphatischen, alicyclischen, aromatischen oder heteroaromatischen oder eine Kombination aus derartigen Resten darstellt, durch welchen die Bindung der Dicarbonsäureamidgruppierung der Formel I über ein Stickstoffatom derselben oder ein Kohlenstoffatom derselben an ein Stickstoffatom oder ein Kohlenstoffatom einer weiteren Dicarbonsäurediamidstruktur einer entsprechenden Formel I', beziehungsweise I'', unter Ausbildung der Verbindung mit 6 Carbonsäureamidgruppen pro Molekül erreicht wird, und wobei die Gesamtstruktur der Verbindung mit 6 Carbonsäureamidgruppen offenkettig oder cyclisch ist, und wobei
gegebenenfalls entweder der Rest R₁ oder R₂ gemeinsam mit dem Stickstoffatom, an welches die beiden Reste gebunden sind, oder der Rest R₃ und R₄ gemeinsam mit dem Stickstoffatom, an welches die beiden Reste R₃ und R₄ gebunden sind, einen gegebenenfalls noch weitere Heteroatome aufweisenden heterocyclischen Rest bilden oder gegebenenfalls der Rest R₁ oder der Rest R₂ des einen der beiden Stickstoffatome der Dicarbonsäurediamidgruppierung der Formel I gemeinsam mit dem Reste R₃ oder R₄ des anderen der beiden Stickstoffatome der Dicarbonsäurediamidgruppierung der Formel I diese Dicarbonsäurediamidgruppierung unter Ausbildung einer cyclischen Struktur ringschliesst.

2. Verfahren nach Anspruch 1, dadurch gekennzeichnet, dass in den Carbonsäureamiden, die pro Molekül drei Dicarbonsäurediamidgruppierungen der Formel I aufweisen, zwei der Reste R₁, R₂, R₃ und R₄ gemeinsam mit einem entsprechenden Rest R₁', R₂', R₃' und R₄' und gemeinsam mit einem Rest R₁'', R₂'', R₃'' und R₄'' der beiden weiteren Dicarbonsäurediamidstrukturen der entsprechenden Formel I', beziehungsweise I'', einen zweiwertigen, aliphatischen, alicyclischen, aromatischen oder heteroaromatischen oder eine Kombination aus derartigen Resten darstellen, durch welchen die Bindung der Dicarbonsäurediamidgruppierung der Formel I über ein Stickstoffatom derselben an eine weitere Dicarbonsäurediamidstruktur der entsprechenden Formeln I' und I'' unter Ausbildung der Verbindung mit sechs Carbonsäureamidgruppen pro Molekül erreicht wird, wobei die Gesamtstruktur der Verbindung mit sechs Carbonsäureamidgruppen offenkettig oder cyclisch ist.

3. Verfahren nach Anspruch 1 oder 2, dadurch gekennzeichnet, dass man die Umsetzung mit Carbonsäureamiden durchführt, welche die folgende Formel II 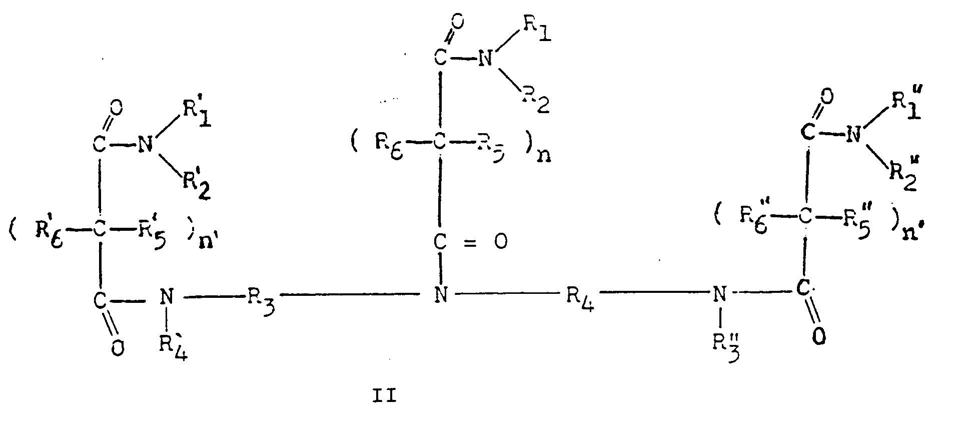 aufweisen, wobei in dieser Formel II die Reste
R₃ und R₄ unabhängig voneinander zweiwertige aliphatische, alicyclische, aromatische, heteroaromatische oder eine Kombination aus derartigen Resten darstellen, die Reste und Symbole R₁', R₂', R₄', R₅', R₆', R₁'', R₂'', R₃'', R₅'', R₆'', n' und n'' die gleiche Bedeutung aufweisen, wie die Reste und Symbole R₁, R₂, R₃, R₄, R₅, R₆ und n in den Dicarbonsäurediamidgruppierungen der Formel I, und wobei gegebenenfalls der Rest R₂ und/oder der Rest R₁, zusammen mit dem Rest R₁' oder R₂' oder R₄' oder R₃'' oder R₂'' oder R₁'' einen zweiwertigen Rest darstellt und/oder der Rest R₁' und/oder der Rest R₂', gemeinsam mit dem Rest R₃'' oder dem Rest R₂'' oder dem Rest R₁'' einen zweiwertigen Rest darstellt und/oder
der Rest R₁'' oder der Rest R₂'' gemeinsam mit dem Rest R₄' einen zweiwertigen Rest darstellt und wobei die genannten zweiwertigen Reste aliphatische, alicyclische, aromatische oder heteroaromatische Reste oder eine Kombination aus derartigen Resten darstellen, welche die Carbonsäureamide der Formel II zu einer monocyclischen, bicyclischen, tricyclischen oder polycyclischen Verbindung ringschliessen.

4. Verfahren nach Anspruch 1 oder 2, dadurch gekennzeichnet, dass man die Umsetzung mit Carbonsäureamiden durchführt, welche die folgende Formel VIII 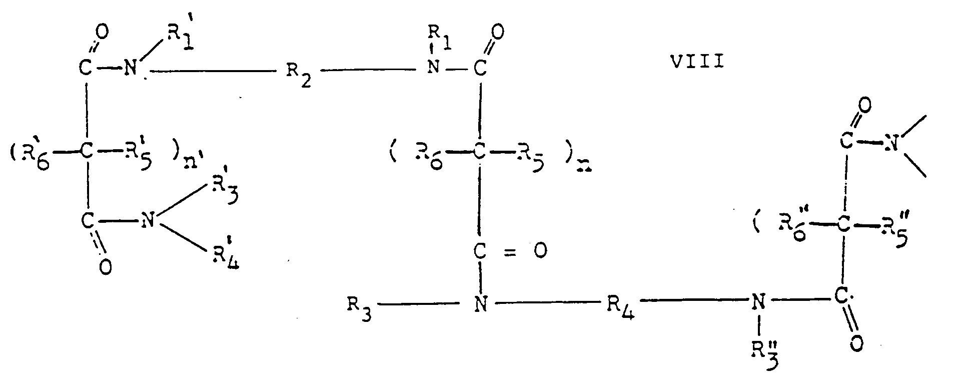 aufweisen, in welcher die Reste
R₂ und R₄ unabhängig voneinander zweiwertige aliphatische, alicyclische, aromatische oder heteroaromatische Reste oder Kombinationen aus derartigen Resten darstellen und die Reste
R₁, R₁', R₁'', R₂'', R₃, R₃', R₄' und R₃'' die gleiche bedeutung aufweisen, wie in Anspruch 1, im Zusammenhang mit den entsprechenden Dicarbonsäurediamideinheiten
der Formel I, beziehungsweise den entsprechenden Einheiten der Formeln I' und I'', definiert wurde, oder
der Rest R₁'', zusammen mit dem Rest R₂'', und/oder
der Rest R₃', zusammen mit dem Rest R₄', und/oder
der Rest R₁', zusammen mit dem Rest R₃ und den entsprechenden Stickstoffatomen, an welche diese Reste gebunden sind, einen heterocyclischen Ring darstellen,
R₅, R₆, R₅', R₆', R₅'' und R₆'' die gleiche Bedeutung aufweisen, wie in Anspruch 1, im Zusammenhang mit den Dicarbonsäurediamideinheiten der Formel I, beziehungsweise den entsprechenden Einheiten der Formeln I' und I'', definiert wurde, oder
der Rest R₃ und/oder der Rest R₁, zusammen mit dem Rest R₁', R₃', R₄', R₃'', R₂'' oder R₁'' einen zweiwertigen Rest darstellt und/oder
der Rest R₃' und/oder der Rest R₄', zusammen mit dem Rest R₃'' oder R₂'' oder R₁'', einen zweiwertigen Rest bildet und/oder
der Rest R₁'' oder der Rest R₂'', zusammen mit dem Rest R₄', einen zweiwertigen Rest darstellt, und
wobei die genannten zweiwertigen Reste aliphatische, alicyclische, aromatische oder heteroaromatische Reste oder eine Kombination aus zwei oder mehr derartigen Resten darstellen, durch welche die Carbonsäureamide der Formel VIII ringgeschlossen sind, wobei sich ein entsprechendes Carbonsäureamid bildet, welches eine monocyclische, bicyclische, tricyclische oder polycyclische Struktur aufweist.

5. Verfahren nach einem der Ansprüche 1 bis 4, dadurch gekennzeichnet, dass in den verwendeten Carbonsäureamiden in den drei Dicarbonsäurediamidgruppierungen der Formel I, beziehungsweise I', beziehungsweise I'', jeweils n, n', beziehungsweise n'', die Zahl 1 oder 2 bedeutet, und dass die Reste R₅, R₆, R₅', R₆', R₅'' und R₆'' vorzugsweise die Bedeutung von Wasserstoffatomen oder Methylgruppen aufweisen, speziell bevorzugt die Bedeutung von Wasserstoffatomen.

6. Verfahren nach einem der Ansprüche 1 bis 5, dadurch gekennzeichnet, dass man Carbonsäure-amide verwendet, die in ihrem Molekül mindestens einen gegebenenfalls substituierten Alkyl, Alkenyl oder Alkinyl-Rest aufweisen, der mindestens 4, vorzugsweise 5 - 15, und speziell bevorzugt, 6 - 12 Kohlenstoffatome aufweist, und/oder dass die Stickstoffatome zweier unterschiedlicher Carbonsäureamidgruppen dieser Carbonsäureamide miteinander durch eine gegebenenfalls substituierte zweiwertige aliphatische Gruppe, die gegebenenfalls eine oder zwei Doppelbindungen oder Dreifachbindungen aufweist, verbunden sind, wobei diese aliphatische Gruppe eine Kettenlänge von mindestens 4 Kohlenstoffatomen, vorzugsweise eine Kettenlänge von 5 - 15 Kohlenstoffatomen, speziell bevorzugt 6 - 12 Kohlenstoffatomen, aufweist.

7. Verfahren nach einem der Ansprüche 3 bis 6, dadurch gekennzeichnet, dass man Carbonsäureamide der Formel II oder Carbonsäureamide der Formel VIII verwendet, wobei in der Formel II die Reste R₃ und R₄, beziehungsweise in der Formel VIII die Reste R₂ und R₄ Alkylengruppen einer Kettenlänge von mindestens 4 Kohlenstoffatomen sind, vorzugsweise unverzweigte Reste der Formel
-(CH₂)_{z}- ,
in welchen z eine ganze Zahl im Bereich von 5 - 15, speziell bevorzugt, 6 - 12, ist,
und dass die verwendeten Carbonsäureamide der Formel II, beziehungsweise VIII, vorzugsweise nicht cyclische Verbindungen sind, wobei in den entsprechenden Verbindungen der Formel II, sowohl der Rest R₁ als auch der Rest
R₁' und auch der Rest R₁'' eine Alkylgruppe mit einer Kettenlänge von mindestens 4 Kohlenstoffatomen, vorzugsweise eine Alkylkette mit 5 - 15 Kohlenstoffatomen, speziell bevorzugt mit 6 - 12 Kohlenstoffatomen ist,
und dass in den nicht cyclischen Verbindungen der Formel VIII sowohl der Rest R₃' als auch der Rest R₁'' eine Alkylgruppe mit einer Kettenlänge von mindestens vier Kohlenstoffatomen, vorzugsweise eine Alkylgruppe mit 5 - 15 Kohlenstoffatomen, speziell bevorzugt mit 6 - 12 Kohlenstoffatomen, ist.

8. Verfahren gemäss einem der Ansprüche 3, beziehungsweise 5 bis 7, dadurch gekennzeichnet,
dass man Carbonsäureamide verwendet, welche die folgende Formel III 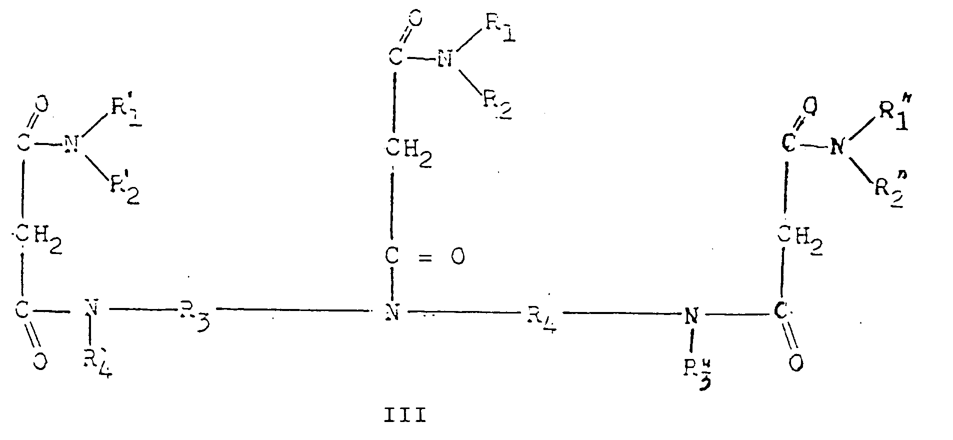 aufweisen, wobei in dieser Formel die Reste
R₃ und R₄ eine Gruppierung der Formel
-(CH₂)_{z}-
bedeuten, in welcher z 5, 6 oder 7 ist, wobei vorzugsweise in beiden Resten z die gleiche Bedeutung aufweisen und speziell bevorzugt, 6 sind,
R₄' und R₃'' Wasserstoff oder einen Alkylrest mit
1 - 4 Kohlenstoffatomen bedeuten,
die Reste
R₁, R₁' und R₁'' Alkylreste mit 5 - 9 Kohlenstoffatomen darstellen, wobei die Reste R₁, R₁' und R₁'' vorzugsweise miteinander identisch sind und speziell bevorzugt entsprechende geradkettige Alkylreste darstellen und die Reste
R₂, R₂' und R₂'' vorzugsweise miteinander identisch sind und speziell bevorzugt die Bedeutung eines Wasserstoffatomes oder einer Alkylgruppe mit 1 - 4 Kohlenstoffatomen, insbesondere die Bedeutung einer Methylgruppe aufweisen.

9. Verfahren gemäss einem der Ansprüche 4 - 7, dadurch gekennzeichnet, dass man Carbonsäureamide verwendet, welche die folgende Formel IX 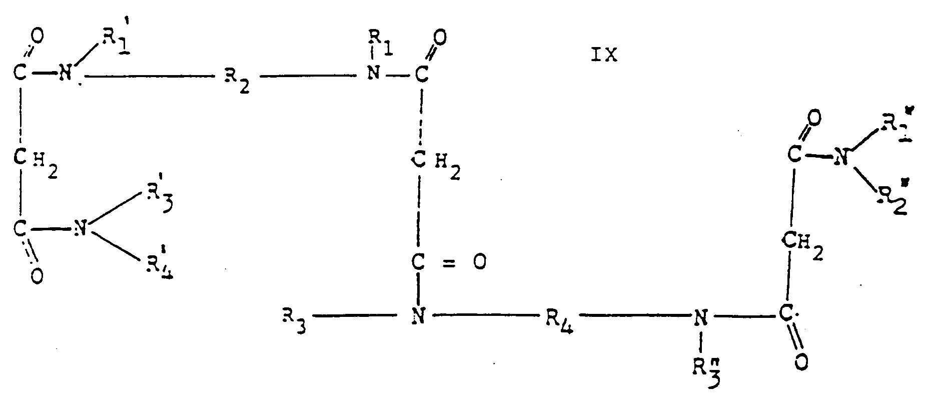 aufweisen, wobei in dieser Formel IX die Reste
R₂ und R₄ unabhängig voneinander Gruppen der Formel
-(CH₂)_{z}-
darstellen, in welcher
z eine ganze Zahl im Bereich von 5 - 9 ist,
und die Reste
R₄', R₁', R₃, R₃', R₁, R₁'', R₃'' und R₂'' unabhängig voneinander Wasserstoffatome oder Alkylgruppen mit
1 - 12 Kohlenstoffatomen darstellen.

10. Verfahren gemäss einem der Ansprüche 1 bis 9, dadurch gekennzeichnet, dass sich bei der Umsetzung der Magnesiumionen mit der Verbindung, die pro Molekül 6 Carbonsäureamidgruppen aufweist, entsprechende lipophile Magnesiumkomplexe bilden, die 1:1 Komplexe aus einem Mol Magnesiumionen und einem Mol Carbonsäureamid sind.

11. Verfahren gemäss einem der Ansprüche 1 bis 10, dadurch gekennzeichnet, dass die lipophilen Magnesiumkomplexe hergestellt werden, indem man ein Carbonsäureamid, das pro Molekül 6 Carbonsäureamidgruppen aufweist und das sich in einem oder auf einem Trägermaterial befindet, mit einem flüssigen Material in Berührung bringt, das die Magnesiumionen enthält.

12. Testvorrichtung zur Bestimmung der Aktivität, beziehungsweise Konzentration, von Magnesiumionen in flüssigen Proben, dadurch gekennzeichnet, dass sie als magnesiumselektive Komponente ein Carbonsäureamid enthält, das pro Molekül 6 Carbonsäureamidgruppen aufweist und die, Struktur besitzt, die für die entsprechenden Carbonsäureamide in einem der Verfahrensansprüche 1 - 9 definiert ist

13. Testvorrichtung gemäss Anspruch 12, dadurch gekennzeichnet, dass sie die magnesiumselektive Verbindung in einem oder auf einem inerten Trägermaterial enthält, vorzugsweise in einem Polymermaterial.

14. Testvorrichtung gemäss Anspruch 12 oder 13, dadurch gekennzeichnet, dass sie ein ionenselektiver Teil ist, der das Carbonsäureamid, eingebettet in einem Polymermatierial, enthält, vorzugsweise einem Polyvinylhalogenidhomopolymerisat oder Polyvinylhalogenidcopolymerisat.

15. Testvorrichtung gemäss Anspruch 14 dadurch gekennzeichnet, dass sie als weitere Komponente einen Weichmacher enthält, vorzugsweise einen Aetherweichmacher, beispielsweise o-Nitrophenyl-octyläther, oder einen Esterweichmacher, vorzugsweise auf Basis eines Dicarbonsäurediesters oder eines Tetracarbonsäuretetraesters, beispielsweise eines Benzophenontetracarbonsäuretetraesters oder Benzhydroltetracarbonsäuretetraesters und dass der ionenselektive Teil gegebenenfalls noch eine als Ionenaustauscher wirkende Komponente enthält, beispielsweise Tetraphenylborat, in welchem die Benzolkerne gegebenenfalls Substituenten tragen, oder Salze dieser Borate.

16. Verfahren zur Herstellung von Carbonsäureamiden, die als Ausgangsmaterial in dem Verfahren gemäss Anspruch 3 verwendbar sind, dadurch gekennzeichnet, dass man entsprechende Carbonsäureamide herstellt, welche die folgende Formel II 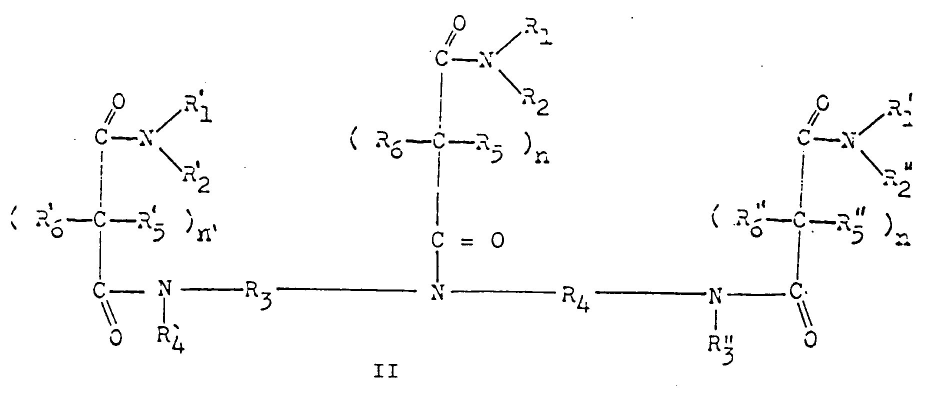 besitzen, wobei in dieser Formel II die Reste
R₃ und R₄, sowie die Reste R₁', R₂', R₄', R₅', R₆', R₁, R₂, R₅, R₆, R₁'', R₂'', R₃'', R₅'', R₆'', und die Symbole n, n' und n'' die gleiche Bedeutung aufweisen, wie im Anspruch 3, indem man ein Triamin der Formel IV 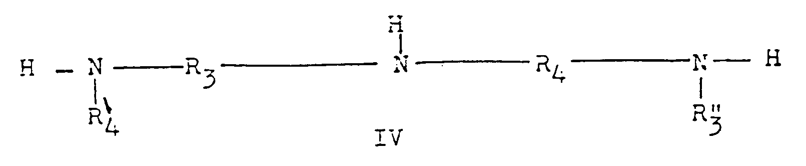 mit Carbonsäuren der folgenden Formeln Va, Vb und Vc 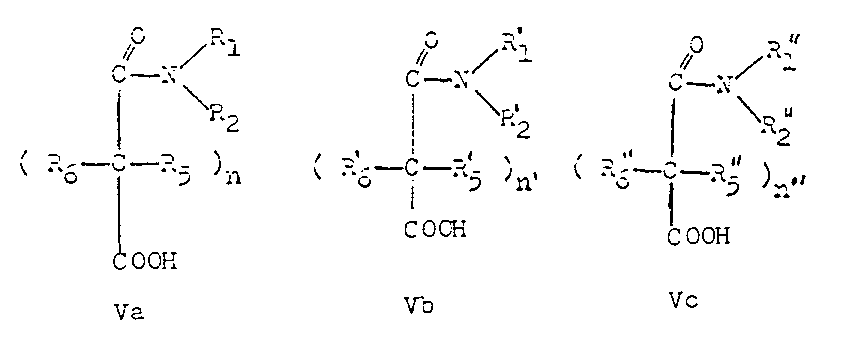 oder reaktiven Derivaten dieser Carbonsäuren zu dem entsprechenden Carbonsäureamid der Formel II umsetzt, oder indem man das Amin der Formel IV zunächst mit Dicarbonsäuren der Formeln VIa, VIb und VIc 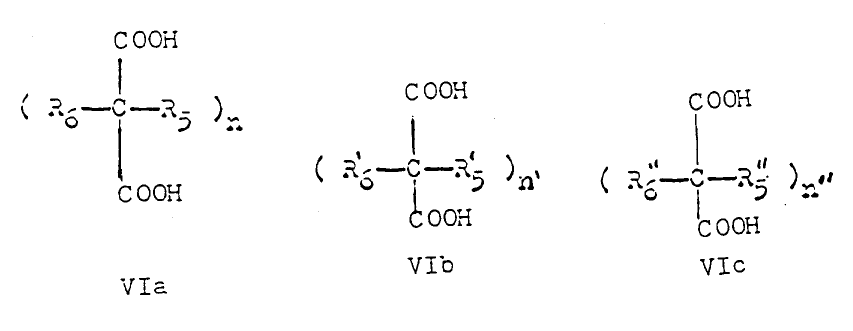 oder reaktiven Derivaten dieser Dicarbonsäuren umsetzt, wobei gegebenenfalls die eine der beiden Carboxylgruppen der Dicarbonsäuren, beziehungsweise der reaktiven Dicarbonsäurederivate, in geschützter Form vorliegt, und wobei man anschliessend das erhaltene Zwischenprodukt der Formel VII 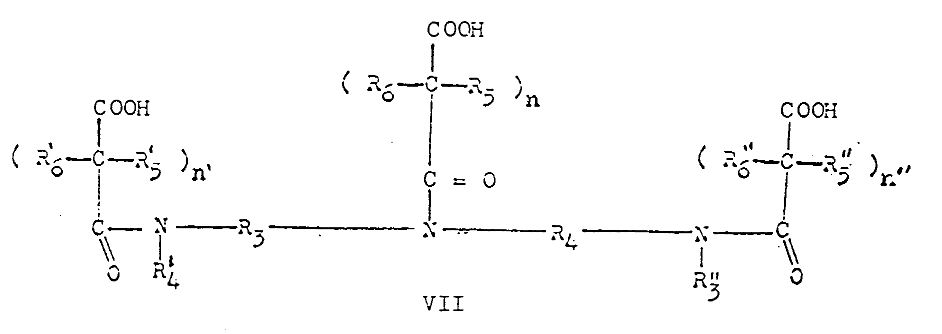 oder ein reaktives Derivat dieses Zwischenproduktes der Formel VII mit den entsprechenden Aminen zu dem gewünschten Endprodukt der Formel II umsetzt.

17. Verfahren nach Anspruch 16, dadurch gekennzeichnet, dass man Carbonsäureamide der Formel II herstellt, welche die in einem der Verfahrensansprüche 5 bis 8 definierte Struktur besitzen.

18. Verfahren zur Herstellung von Carbonsäureamiden, die als Ausgangsmaterial in dem Verfahren gemäss Anspruch 4 verwendbar sind, dadurch gekennzeichnet, dass man entsprechende Carbonsäureamide herstellt, welche die folgende Formel VIII 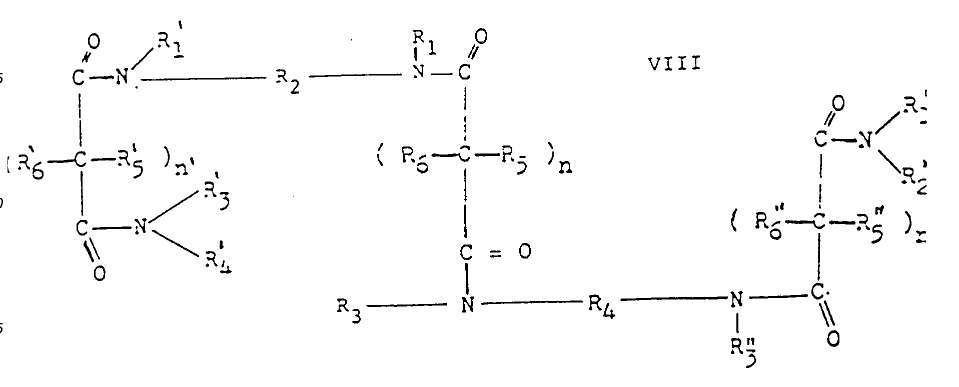 besitzen, wobei in dieser Formel VIII
R₁', R₃', R₄', R₅', R₆', R₁, R₃, R₅, R₆, R₁'', R₂'', R₃'', R₅'' und R₆'', sowie die Symbole n, n' und n'' die gleiche Bedeutung haben, wie im Anspruch 4,
indem man zwei Aminoverbindungen 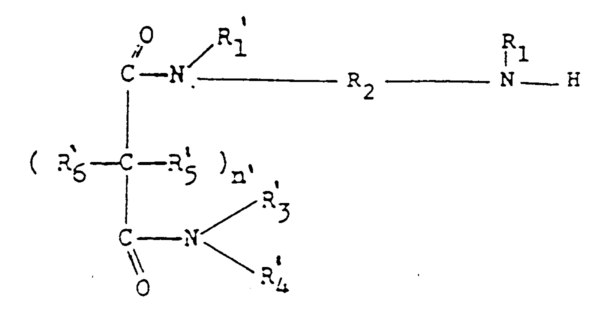 und 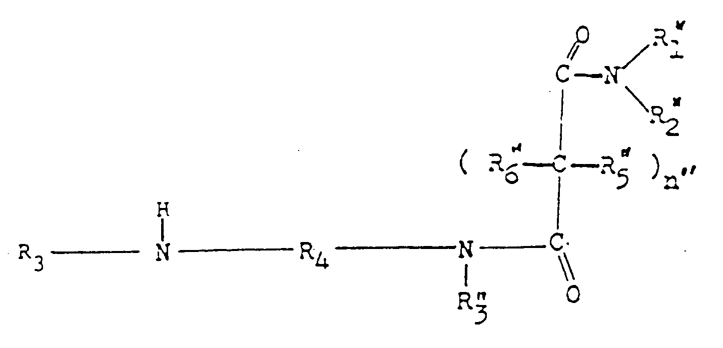 mit einer Dicarbonsäure der Struktur 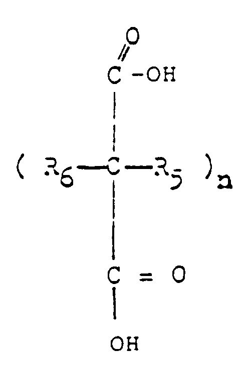 oder einem reaktiven Derivat dieser Dicarbonsäure umsetzt, wobei man das Carbonsäureamid der Formel VIII erhält.

19. Verfahren nach Anspruch 18, dadurch gekennzeichnet, dass man Carbonsäureamide der Formel VIII herstellt, welche die in den Verfahrensansprüchen 5 bis 7 oder 9 angegebene Struktur besitzen.

## Claims (Claims for the following Contracting State(s): AT, BE, CH, DE, DK, FR, GB, IT, LI, LU, NL, SE)

1. Carboxamides which form lipophilic complexes with magnesium ions, characterized in that they have 6 carboxamide groups per molecule and in that each molecule of the carboxamides contains three dicarboxylic acid diamide groupings of the following formula I: 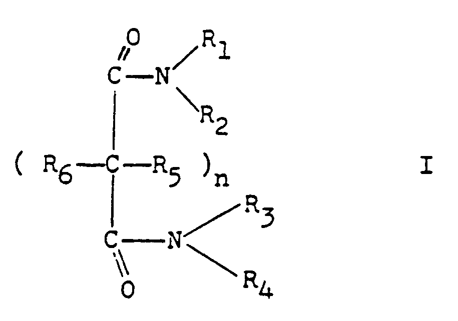 in which the radicals R₁, R₂, R₃ and R₄ independently of one another are hydrogen atoms, optionally substituted alkyl, alkenyl or alkynyl radicals, optionally substituted cycloalkyl radicals or optionally substituted aromatic or heterocyclic radicals, or combinations of such radicals,
R₅ and R₆ are hydrogen atoms, alkyl groups having 1 - 4 carbon atoms or halogen atoms, and
n is 0 or an integer in the range 1 - 3,
except that at least one of the radicals R₁, R₂, R₃, R₄, R₅ and R₆, together with two corresponding radicals R₁', R₂', R₃', R₄', R₅', R₆', R₁'', R₂'', R₃'', R₄'', R₅'' or R₆'' of the other two dicarboxylic acid diamide structures of the corresponding formula I' or I'', is a polyvalent aliphatic, alicyclic, aromatic or heteroaromatic radical, or a combination of such radicals, which bonds the dicarboxylic acid diamide grouping of formula I, *via* a nitrogen atom thereof or a carbon atom thereof, to a nitrogen atom or a carbon atom of another dicarboxylic acid diamide structure of a corresponding formula I' or I'' to form the compound with 6 carboxamide groups per molecule, the overall structure of the compound with 6 carboxamide groups being open-chain or cyclic and, if appropriate, either the radicals R₁ and R₂, together with the nitrogen atom to which these two radicals are bonded, or the radicals R₃ and R₄, together with the nitrogen atom to which these two radicals R₃ and R₄ are bonded, forming a heterocyclic radical optionally containing yet further heteroatoms, or, if appropriate, the radical R₁ or the radical R₂ on one of the two nitrogen atoms of the dicarboxylic acid diamide grouping of formula I, together with the radical R₃ or R₄ on the other of the two nitrogen atoms of the dicarboxylic acid diamide grouping of formula I, cyclizing this dicarboxylic acid diamide grouping to form a cyclic structure.

2. Carboxamides according to Claim 1, characterized in that two of the radicals R₁, R₂, R₃ and R₄, together with a corresponding radical R₁', R₂', R₃' and R₄' and together with a radical R₁'', R₂'', R₃'' and R₄'' of the other two dicarboxylic acid diamide structures of the corresponding formula I' or I'', are a divalent aliphatic, alicyclic, aromatic or heteroaromatic radical, or a combination of such radicals, which bond the dicarboxylic acid diamide grouping of formula I, *via* a nitrogen atom thereof, to another dicarboxylic acid diamide structure of the corresponding formulae I' and I'' to form the compound with six carboxamide groups per molecule, the overall structure of the compound with six carboxamide groups being open-chain or cyclic.

3. Carboxamides according to Claim 1 or 2, characterized in that they have the following formula II: 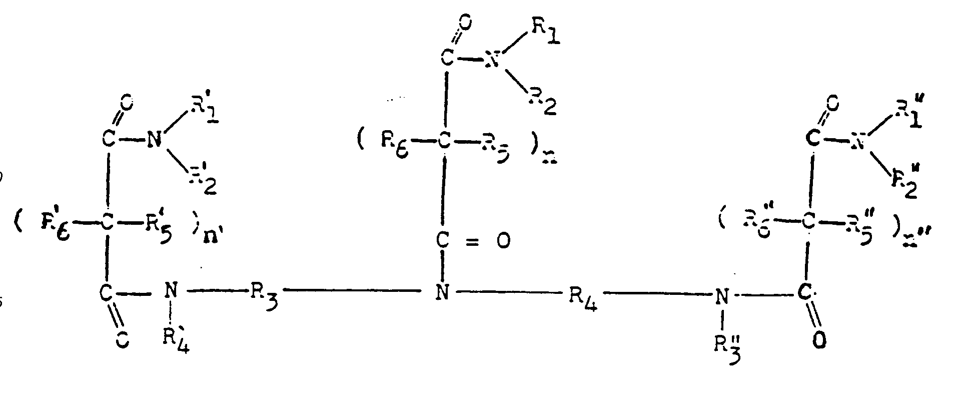 in which the radicals R₃ and R₄ independently of one another are divalent aliphatic, alicyclic, aromatic or heteroaromatic radicals, or a combination of such radicals, the radicals and symbols R₁', R₂', R₄', R₅', R₆', R₁'', R₂'', R₃'', R₅'', R₆'', n' and n'' being defined in the same way as the radicals and symbols R₁, R₂, R₃, R₄, R₅, R₆ and n in the dicarboxylic acid diamide groupings of formula I and, if appropriate, the radical R₂ and/or the radical R₁, together with the radical R₁', R₂', R₄', R₃'', R₂'' or R₁'', is a divalent radical, and/or the radical R₁' and/or the radical R₂', together with the radical R₃'', the radical R₂'' or the radical R₁'', is a divalent radical, and/or the radical R₁'' or the radical R₂'', together with the radical R₄', is a divalent radical, said divalent radicals being aliphatic, alicyclic, aromatic or heteroaromatic radicals, or a combination of such radicals, which cyclize the carboxamides of formula II to form a monocyclic, bicyclic, tricyclic or polycyclic compound.

4. Carboxamides according to Claim 1 or 2, characterized in that these carboxamides have the following formula VIII: 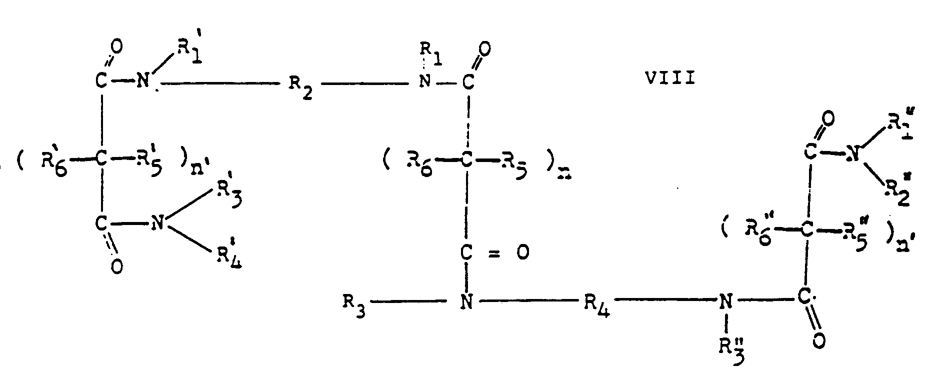 in which the radicals R₂ and R₄ independently of one another are divalent aliphatic, alicyclic, aromatic or heteroaromatic radicals, or combinations of such radicals, the radicals R₁, R₁', R₁'', R₂'', R₃, R₃', R₄' and R₃'' are defined in the same way as in Claim 1 in connection with the corresponding dicarboxylic acid diamide units of formula I or the corresponding units of formulae I' and I'', or the radical R₁'', together with the radical R₂'', and/or the radical R₃', together with the radical R₄', and/or the radical R₁, together with the radical R₃, and together with the corresponding nitrogen atoms to which these radicals are bonded, are a heterocyclic ring, and R₅, R₆, R₅', R₆', R₅'' and R₆'' are defined in the same way as in Claim 1 in connection with the dicarboxylic acid diamide units of formula I or the corresponding units of formulae I' and I'', or the radical R₃ and/or the radical R₁, together with the radical R₁', R₃', R₄', R₃'', R₂'' or R₁'', are a divalent radical, and/or the radical R₃' and/or the radical R₄', together with the radical R₃'', R₂'' or R₁'', form a divalent radical, and/or the radical R₁'' or the radical R₂'', together with the radical R₄', is a divalent radical, said divalent radicals being aliphatic, alicyclic, aromatic or heteroaromatic radicals, or a combination of two or more such radicals, by which the carboxamides of formula VIII are cyclized to form a corresponding carboxamide having a monocyclic, bicyclic, tricyclic or polycyclic structure.

5. Carboxamides according to one of Claims 1 to 4, characterized in that, in the three dicarboxylic acid diamide groupings of formulae I, I' and I'', n, n' and n'' respectively are the number 1 or 2 and the radicals R₅, R₆, R₅', R₆', R₅'' and R₆'' are preferably hydrogen atoms or methyl groups and particularly preferably hydrogen atoms.

6. Carboxamides according to one of Claims 1 to 5, characterized in that they contain in their molecule at least one optionally substituted alkyl, alkenyl or alkynyl radical having at least 4, preferably 5 - 15 and particularly preferably 6 - 12 carbon atoms, and/or in that the nitrogen atoms of two different carboxamide groups of these carboxamides are bonded together by an optionally substituted divalent aliphatic group which optionally has one or two double bonds or triple bonds, this aliphatic group having a chain length of at least 4 carbon atoms, preferably a chain length of 5 - 15 carbon atoms and particularly preferably 6 - 12 carbon atoms.

7. Carboxamides according to one of Claims 3 - 6, characterized in that the radicals R₃ and R₄ in formula II, or the radicals R₂ and R₄ in formula VIII, are alkylene groups having a chain length of at least 4 carbon atoms, preferably unbranched radicals of the formula
-(CH₂)_{z}-
in which z is an integer in the range 5 - 15 and particularly preferably 6 - 12, and in that the carboxamides of formula II or VIII are preferably non-cyclic compounds, the radical R₁, the radical R₁' and the radical R₁'' in the corresponding compounds of formula II being an alkyl group having a chain length of at least 4 carbon atoms, preferably an alkyl chain having 5 - 15 carbon atoms and particularly preferably 6 - 12 carbon atoms, and in that the radical R₃' and the radical R₁'' in the non-cyclic compounds of formula VIII are an alkyl group having a chain length of at least 4 carbon atoms, preferably an alkyl group having 5 - 15 carbon atoms and particularly preferably 6 - 12 carbon atoms.

8. Carboxamides according to one of Claims 3 and 5 to 7, characterized in that they have the following formula III: 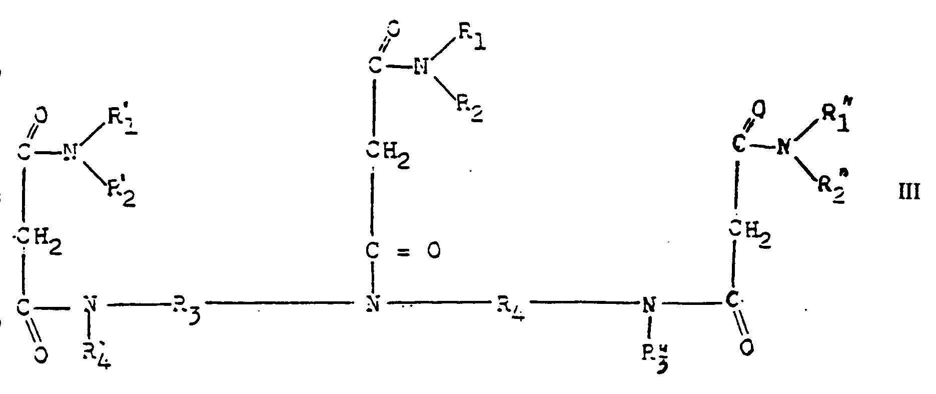 in which the radicals R₃ and R₄ are a grouping of the formula
-(CH₂)_{z}-
in which z is 5, 6 or 7, z preferably being the same in both radicals and particularly preferably 6, R₄' and R₃'' are hydrogen or an alkyl radical having 1 - 4 carbon atoms, the radicals R₁, R₁' and R₁'' are alkyl radicals having 5 - 9 carbon atoms, the radicals R₁, R₁' and R₁'' preferably being identical to one another and particularly preferably being corresponding linear alkyl radicals, and the radicals R₂, R₂' and R₂'' are preferably identical to one another and are particularly preferably a hydrogen atom or an alkyl group having 1 - 4 carbon atoms, especially a methyl group.

9. Carboxamides according to one of Claims 4 - 7, characterized in that they have the following formula IX: 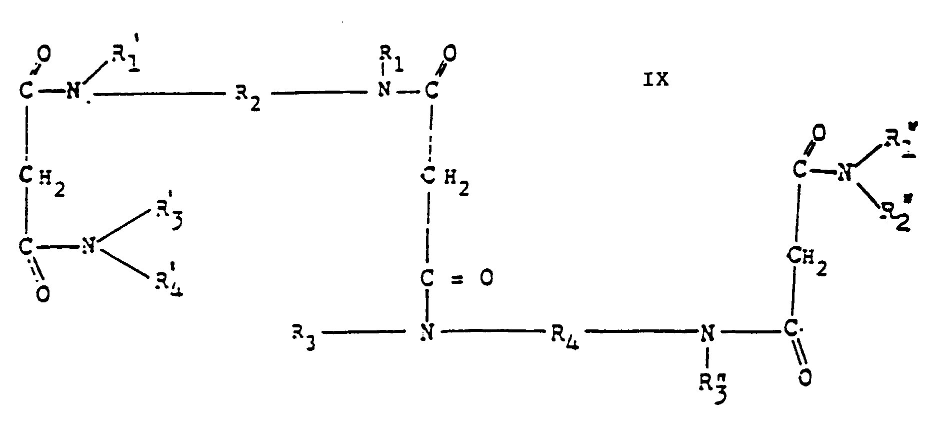 in which the radicals R₂ and R₄ independently of one another are groups of the formula
-(CH₂)_{z}-
in which z is an integer in the range 5 - 9, and the radicals R₄', R₁, R₃, R₃', R₁', R₁'', R₃'' and R₂'' independently of one another are hydrogen atoms or alkyl groups having 1 - 12 carbon atoms.

10. Lipophilic complexes of magnesium ions and the compounds according to one of Claims 1 - 9, having 6 carboxamide groups per molecule, preferably lipophilic complexes of one mol of magnesium ions and one mol of the carboxamides.

11. Process for the preparation of a lipophilic complex of magnesium ions, characterized in that the magnesium ions are brought into contact with the compounds according to one of Claims 1 to 9, having 6 carboxamide groups per molecule, to form the corresponding lipophilic magnesium complexes, preferably 1:1 complexes of one mol of magnesium ions and one mol of carboxamide.

12. Testing device for determining the activity or concentration of magnesium ions in liquid samples, characterized in that it contains, as the magnesium-selective component, a compound according to one of Claims 1 - 9, having 6 carboxamide groups per molecule.

13. Testing device according to Claim 12, characterized in that it contains the magnesium-selective compound in or on an inert carrier material, preferably in a polymer material.

14. Testing device according to Claim 12 or 13, characterized in that it is an ion-selective moiety containing the carboxamide embedded in a polymer material, preferably a polyvinyl halide homopolymer or polyvinyl halide copolymer.

15. Testing device according to Claim 14, characterized in that it contains, as a further component, a plasticizer, preferably an ether plasticizer, for example o-nitrophenyl octyl ether, or an ester plasticizer, preferably based on a dicarboxylic acid diester or a tetracarboxylic acid tetraester, for example a benzophenonetetracarboxylic acid tetraester or benzohydroltetracarboxylic acid tetraester, and in that the ion-selective moiety optionally also contains a component acting as an ion exchanger, for example tetraphenyl borate, in which the benzene rings are optionally substituted, or salts of these borates.

16. Process for the preparation of the carboxamides according to Claim 3 which contain six carboxamide groups per molecule and have the following formula II: 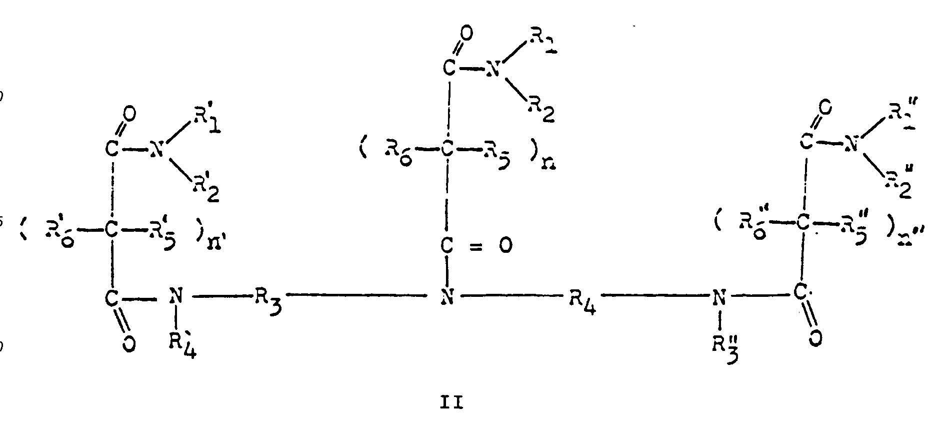 in which the radicals R₃ and R₄, the radicals R₁', R₂', R₄', R₅', R₆', R₁, R₂, R₅, R₆, R₁'', R₂'', R₃'', R₅'' and R₆'' and the symbols n, n' and n'' are defined in the same way as in Claim 3, by reacting a triamine of formula IV: 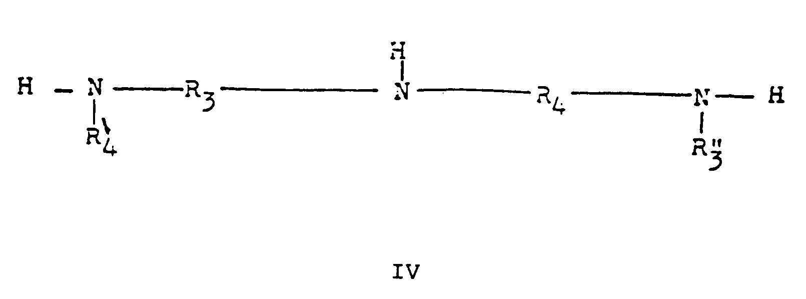 with carboxylic acids of the following formulae Va, Vb and Vc: 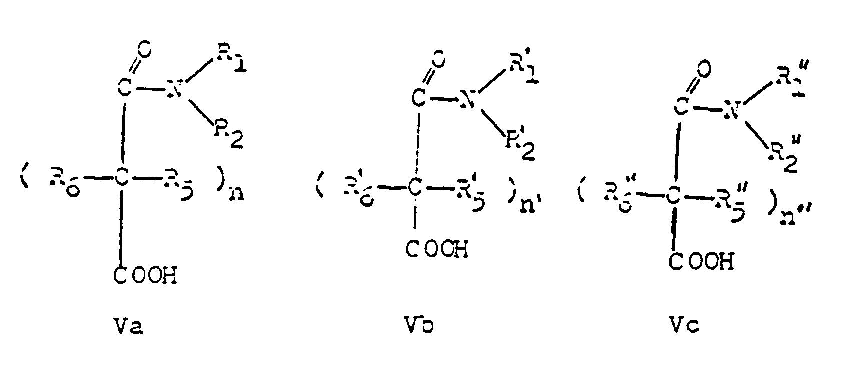 or with reactive derivatives of these carboxylic acids to give the corresponding carboxamide of formula II, or by first reacting the amine of formula IV with dicarboxylic acids of formulae VIa, VIb and VIc: 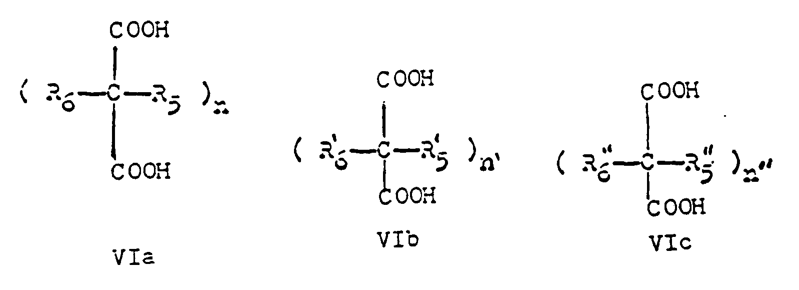 or with reactive derivatives of these dicarboxylic acids, one of the two carboxyl groups of the dicarboxylic acids or of the reactive dicarboxylic acid derivatives optionally being in protected form, and then reacting the resulting intermediate of formula VII: 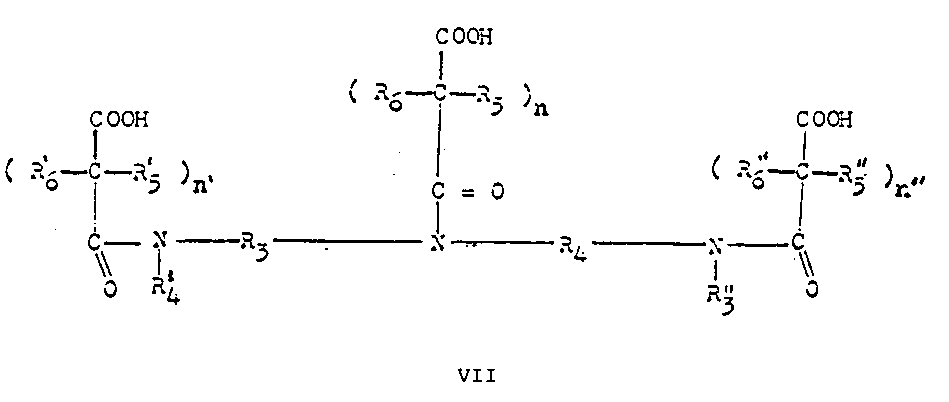 or a reactive derivative of this intermediate of formula VII with the appropriate amines to give the desired end product of formula II.

17. Process for the preparation of the carboxamides according to Claim 4 which contain six carboxamide groups per molecule and have the following formula VIII: 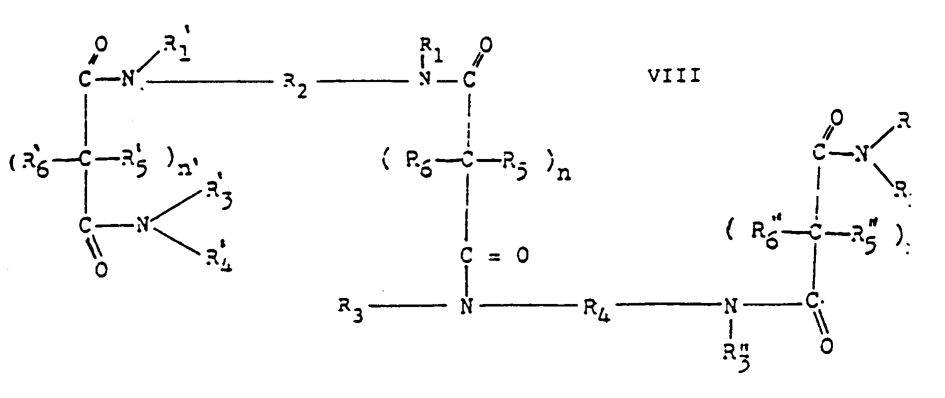 in which R₁', R₃', R₄', R₅', R₆', R₁, R₃, R₅, R₆, R₁'' R₂'', R₃'', R₅'' and R₆'' and the symbols n, n' and n'' are defined in the same way as in Claim 4, by reacting two amino compounds 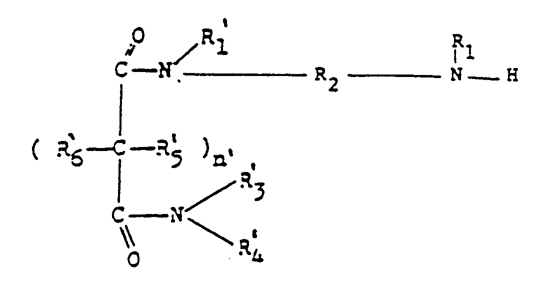 and 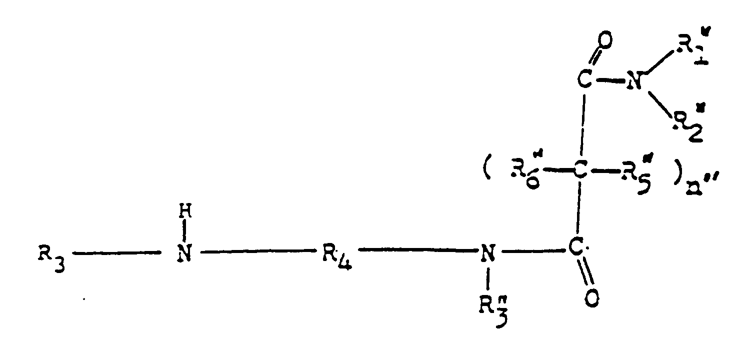 with a dicarboxylic acid of the structure 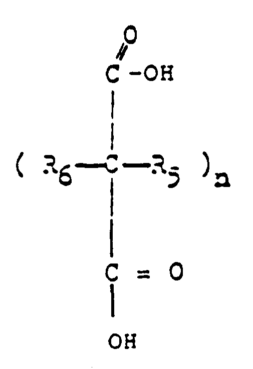 or with a reactive derivative of this dicarboxylic acid to give the carboxamide of formula VIII.

## Claims (Claims for the following Contracting State(s): ES)

1. Process for the preparation of lipophilic magnesium complexes, characterized in that magnesium ions are reacted with carboxamides which have six carboxamide groups per molecule and in which each molecule of these carboxamides contains three dicarboxylic acid diamide groupings of the following formula I: 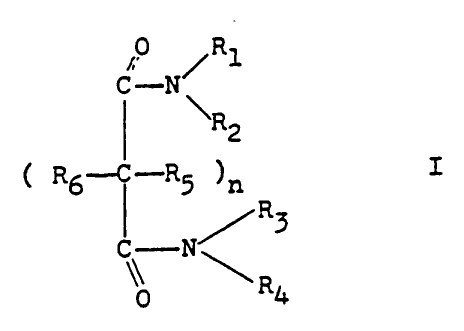 in which the radicals R₁, R₂, R₃ and R₄ independently of one another are hydrogen atoms, optionally substituted alkyl, alkenyl or alkynyl radicals, optionally substituted cycloalkyl radicals or optionally substituted aromatic or heterocyclic radicals, or combinations of such radicals,
R₅ and R₆ are hydrogen atoms, alkyl groups having 1 - 4 carbon atoms or halogen atoms, and
n is 0 or an integer in the range 1 - 3,
except that at least one of the radicals R₁, R₂, R₃, R₄, R₅ and R₆, together with two corresponding radicals R₁', R₂', R₃', R₄', R₅', R₆', R₁'', R₂'', R₃'', R₄'', R₅'' or R₆'' of the other two dicarboxylic acid diamide structures of the corresponding formula I' or I'', is a polyvalent aliphatic, alicyclic, aromatic or heteroaromatic radical, or a combination of such radicals, which bonds the dicarboxylic acid diamide grouping of formula I, *via* a nitrogen atom thereof or a carbon atom thereof, to a nitrogen atom or a carbon atom of another dicarboxylic acid diamide structure of a corresponding formula I' or I'' to form the compound with 6 carboxamide groups per molecule, the overall structure of the compound with 6 carboxamide groups being open-chain or cyclic and, if appropriate, either the radical R₁ or R₂, together with the nitrogen atom to which these two radicals are bonded, or the radicals R₃ and R₄, together with the nitrogen atom to which these two radicals R₃ and R₄ are bonded, forming a heterocyclic radical optionally containing yet further heteroatoms, or, if appropriate, the radical R₁ or the radical R₂ on one of the two nitrogen atoms of the dicarboxylic acid diamide grouping of formula I, together with the radical R₃ or R₄ on the other of the two nitrogen atoms of the dicarboxylic acid diamide grouping of formula I, cyclizing this dicarboxylic acid diamide grouping to form a cyclic structure.

2. Process according to Claim 1, characterized in that, in the carboxamides which have three dicarboxylic acid diamide groupings of formula I per molecule, two of the radicals R₁, R₂, R₃ and R₄, together with a corresponding radical R₁', R₂', R₃' and R₄' and together with a radical R₁'', R₂'', R₃'' and R₄'' of the other two dicarboxylic acid diamide structures of the corresponding formula I' or I'', are a divalent aliphatic, alicyclic, aromatic or heteroaromatic radical, or a combination of such radicals, which bond the dicarboxylic acid diamide grouping of formula I, *via* a nitrogen atom thereof, to another dicarboxylic acid diamide structure of the corresponding formulae I' and I'' to form the compound with six carboxamide groups per molecule, the overall structure of the compound with six carboxamide groups being open-chain or cyclic.

3. Process according to Claim 1 or 2, characterized in that the reaction is carried out with carboxamides of the following formula II: 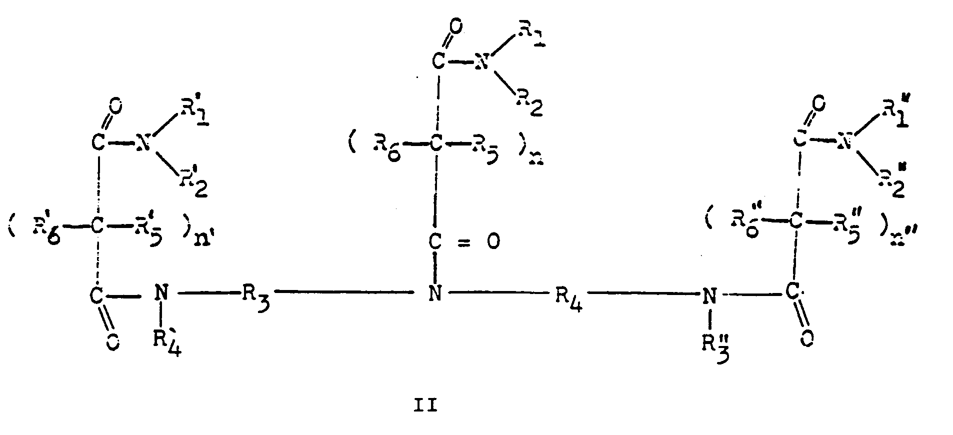 in which the radicals R₃ and R₄ independently of one another are divalent aliphatic, alicyclic, aromatic or heteroaromatic radicals, or a combination of such radicals, the radicals and symbols R₁', R₂', R₄', R₅', R₆', R₁'', R₂'', R₃'', R₅'', R₆'', n' and n'' being defined in the same way as the radicals and symbols R₁, R₂, R₃, R₄, R₅, R₆ and n in the dicarboxylic acid diamide groupings of formula I and, if appropriate, the radical R₂ and/or the radical R₁, together with the radical R₁', R₂', R₄', R₃'', R₂'' or R₁'', is a divalent radical, and/or the radical R₁' and/or the radical R₂', together with the radical R₃'', the radical R₂'' or the radical R₁'', is a divalent radical, and/or the radical R₁'' or the radical R₂'', together with the radical R₄', is a divalent radical, said divalent radicals being aliphatic, alicyclic, aromatic or heteroaromatic radicals, or a combination of such radicals, which cyclize the carboxamides of formula II to form a monocyclic, bicyclic, tricyclic or polycyclic compound.

4. Process according to Claim 1 or 2, characterized in that the reaction is carried out with carboxamides of the following formula VIII: 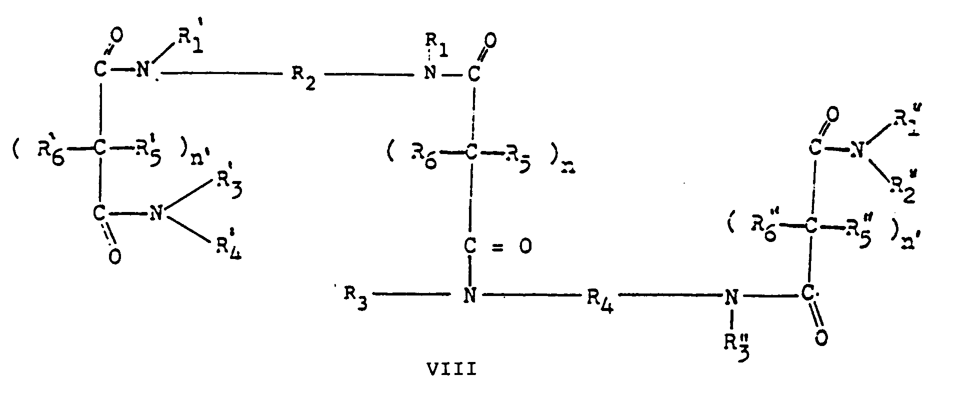 in which the radicals R₂ and R₄ independently of one another are divalent aliphatic, alicyclic, aromatic or heteroaromatic radicals, or combinations of such radicals, the radicals R₁, R₁', R₁'', R₂'', R₃, R₃', R₄' and R₃'' are defined in the same way as in Claim 1 in connection with the corresponding dicarboxylic acid diamide units of formula I or the corresponding units of formulae I' and I'', or the radical R₁'', together with the radical R₂'', and/or the radical R₃', together with the radical R₄', and/or the radical R₁', together with the radical R₃, and together with the corresponding nitrogen atoms to which these radicals are bonded, are a heterocyclic ring, and R₅, R₆, R₅', R₆', R₅'' and R₆'' are defined in the same way as in Claim 1 in connection with the dicarboxylic acid diamide units of formula I or the corresponding units of formulae I' and I'', or the radical R₃ and/or the radical R₁, together with the radical R₁', R₃', R₄', R₃'', R₂'' or R₁'', are a divalent radical, and/or the radical R₃' and/or the radical R₄', together with the radical R₃'', R₂'' or R₁'', form a divalent radical, and/or the radical R₁'' or the radical R₂'', together with the radical R₄', is a divalent radical, said divalent radicals being aliphatic, alicyclic, aromatic or heteroaromatic radicals, or a combination of two or more of such radicals, by which the carboxamides of formula VIII are cyclized to form a corresponding carboxamide having a monocyclic, bicyclic, tricyclic or polycyclic structure.

5. Process according to one of Claims 1 to 4, characterized in that, in the three dicarboxylic acid diamide groupings of formulae I, I' and I'' of the carboxamides used, n, n' and n'' respectively are the number 1 or 2 and the radicals R₅, R₆, R₅', R₆', R₅'' and R₆'' are preferably hydrogen atoms or methyl groups and particularly preferably hydrogen atoms.

6. Process according to one of Claims 1 to 5, characterized in that carboxamides are used which contain in their molecule at least one optionally substituted alkyl, alkenyl or alkynyl radical having at least 4, preferably 5 - 15 and particularly preferably 6 - 12 carbon atoms, and/or in that the nitrogen atoms of two different carboxamide groups of these carboxamides are bonded together by an optionally substituted divalent aliphatic group which optionally has one or two double bonds or triple bonds, this aliphatic group having a chain length of at least 4 carbon atoms, preferably a chain length of 5 - 15 carbon atoms and particularly preferably 6 - 12 carbon atoms.

7. Process according to one of Claims 3 to 6, characterized in that carboxamides of formula II or carboxamides of formula VIII are used in which the radicals R₃ and R₄ in formula II, or the radicals R₂ and R₄ in formula VIII, are alkylene groups having a chain length of at least 4 carbon atoms, preferably unbranched radicals of the formula
-(CH₂)_{z}-
in which z is an integer in the range 5 - 15 and particularly preferably 6 - 12, and in that the carboxamides of formula II or VIII used are preferably noncyclic compounds, the radical R₁, the radical R₁' and the radical R₁'' in the corresponding compounds of formula II being an alkyl group having a chain length of at least 4 carbon atoms, preferably an alkyl chain having 5 - 15 carbon atoms and particularly preferably 6 - 12 carbon atoms, and in that the radical R₃' and the radical R₁'' in the non-cyclic compounds of formula VIII are an alkyl group having a chain length of at least four carbon atoms, preferably an alkyl group having 5 - 15 carbon atoms and particularly preferably 6 - 12 carbon atoms.

8. Process according to one of Claims 3 and 5 to 7, characterized in that carboxamides are used which have the following formula III: 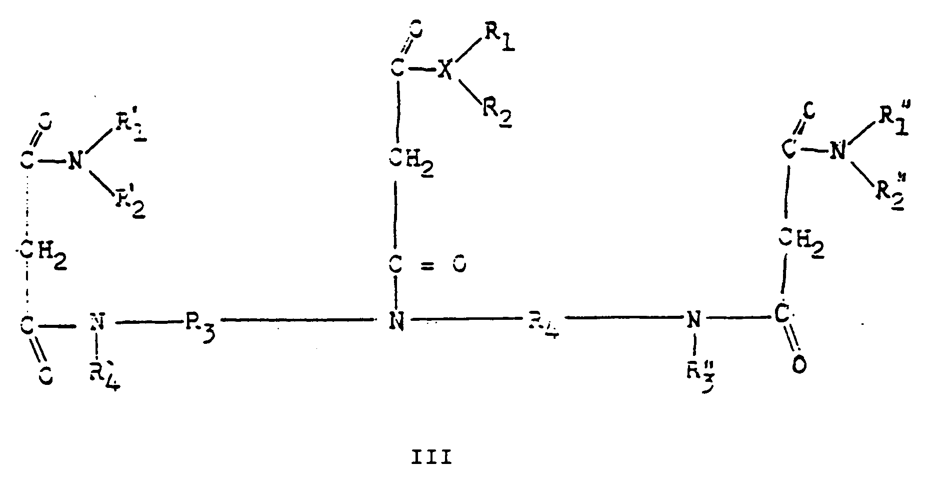 in which the radicals R₃ and R₄ are a grouping of the formula
-(CH₂)_{z}-
in which z is 5, 6 or 7, z preferably being the same in both radicals and particularly preferably 6, R₄' and R₃'' are hydrogen or an alkyl radical having 1 - 4 carbon atoms, the radicals R₁, R₁' and R₁'' are alkyl radicals having 5 - 9 carbon atoms, the radicals R₁, R₁' and R₁'' preferably being identical to one another and particularly preferably being corresponding linear alkyl radicals, and the radicals R₂, R₂' and R₂'' are preferably identical to one another and are particularly preferably a hydrogen atom or an alkyl group having 1 - 4 carbon atoms, especially a methyl group.

9. Process according to one of Claims 4 - 7, characterized in that carboxamides are used which have the following formula IX: 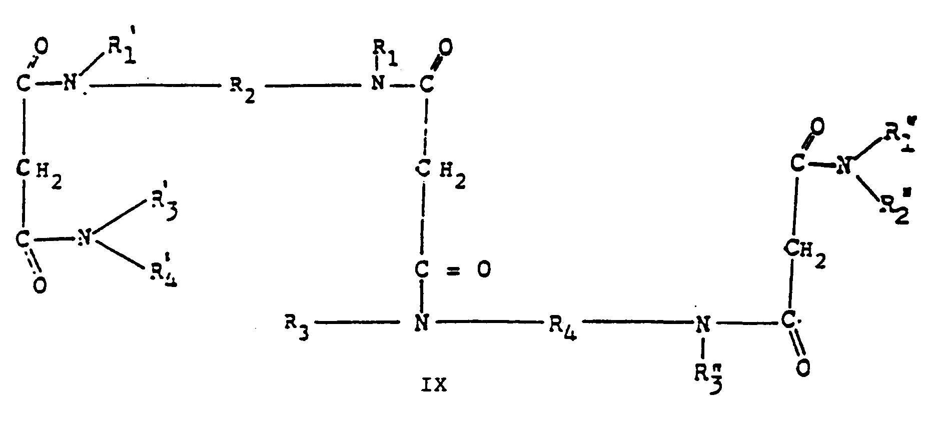 in which the radicals R₂ and R₄ independently of one another are groups of the formula
-(CH₂)_{z}-
in which z is an integer in the range 5 - 9, and the radicals R₄', R₁', R₃, R₃', R₁, R₁'', R₃'' and R₂'' independently of one another are hydrogen atoms or alkyl groups having 1 - 12 carbon atoms.

10. Process according to one of Claims 1 to 9, characterized in that, in the reaction of the magnesium ions with the compound having 6 carboxamide groups per molecule, corresponding lipophilic magnesium complexes are formed which are 1:1 complexes of one mol of magnesium ions and one mol of carboxamide.

11. Process according to one of Claims 1 to 10, characterized in that the lipophilic magnesium complexes are prepared by bringing a carboxamide having 6 carboxamide groups per molecule, which is present in or on a carrier material, into contact with a liquid material containing the magnesium ions.

12. Testing device for determining the activity or concentration of magnesium ions in liquid samples, characterized in that it contains, as the magnesium-selective component, a carboxamide having 6 carboxamide groups per molecule and possessing the structure defined for the corresponding carboxamides in one of Process Claims 1 - 9.

13. Testing device according to Claim 12, characterized in that it contains the magnesium-selective compound in or on an inert carrier material, preferably in a polymer material.

14. Testing device according to Claim 12 or 13, characterized in that it is an ion-selective moiety containing the carboxamide embedded in a polymer material, preferably a polyvinyl halide homopolymer or polyvinyl halide copolymer.

15. Testing device according to Claim 14, characterized in that it contains, as a further component, a plasticizer, preferably an ether plasticizer, for example o-nitrophenyl octyl ether, or an ester plasticizer, preferably based on a dicarboxylic acid diester or a tetracarboxylic acid tetraester, for example a benzophenonetetracarboxylic acid tetraester or benzohydroltetracarboxylic acid tetraester, and in that the ion-selective moiety optionally also contains a component acting as an ion exchanger, for example tetraphenyl borate in which the benzene rings are optionally substituted, or salts of these borates.

16. Process for the preparation of carboxamides which can be used as starting materials in the process according to Claim 3, characterized in that corresponding carboxamides are prepared which have the following formula II: 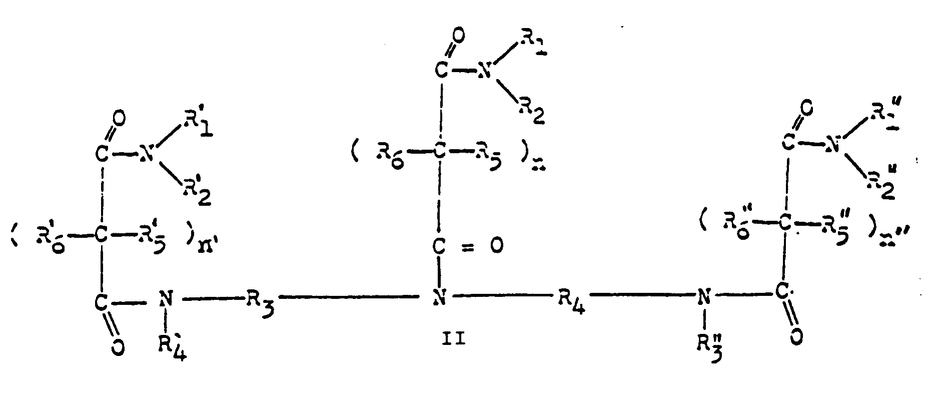 in which the radicals R₃ and R₄, the radicals R₁', R₂', R₄', R₅', R₆', R₁, R₂, R₅, R₆, R₁'', R₂'', R₃'', R₅'' and R₆'' and the symbols n, n' and n'' are defined in the same way as in Claim 3, by reacting a triamine of formula IV: 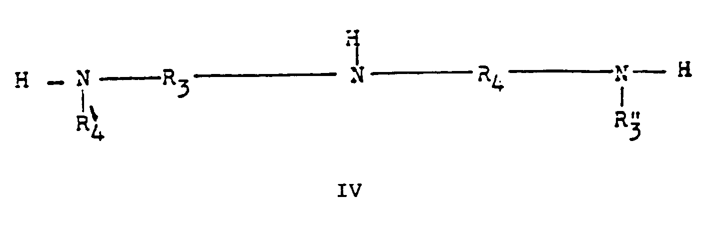 with carboxylic acids of the following formulae Va, Vb and Vc: 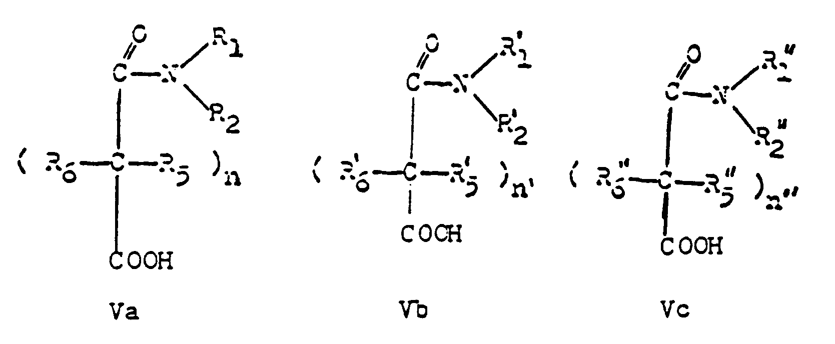 or with reactive derivatives of these carboxylic acids to give the corresponding carboxamide of formula II, or by first reacting the amine of formula IV with dicarboxylic acids of formulae VIa, VIb and VIc: 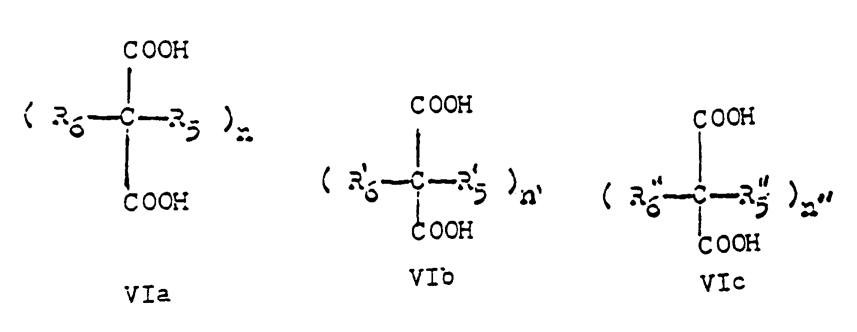 or with reactive derivatives of these dicarboxylic acids, one of the two carboxyl groups of the dicarboxylic acids or of the reactive dicarboxylic acid derivatives optionally being in protected form, and then reacting the resulting intermediate of formula VII: 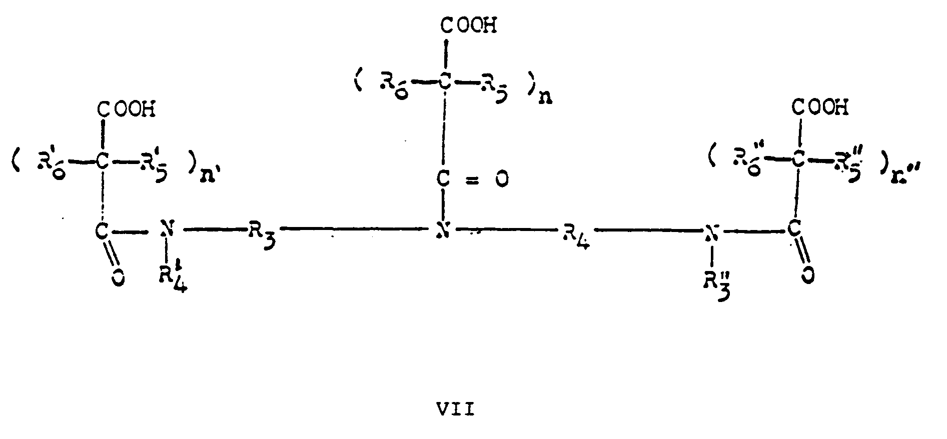 or a reactive derivative of this intermediate of formula VII with the appropriate amines to give the desired end product of formula II.

17. Process according to Claim 16, characterized in that carboxamides of formula II are prepared which possess the structure defined in one of Process Claims 5 to 8.

18. Process for the preparation of carboxamides which can be used as starting materials in the process according to Claim 4, characterized in that corresponding carboxamides are prepared which have the following formula VIII: 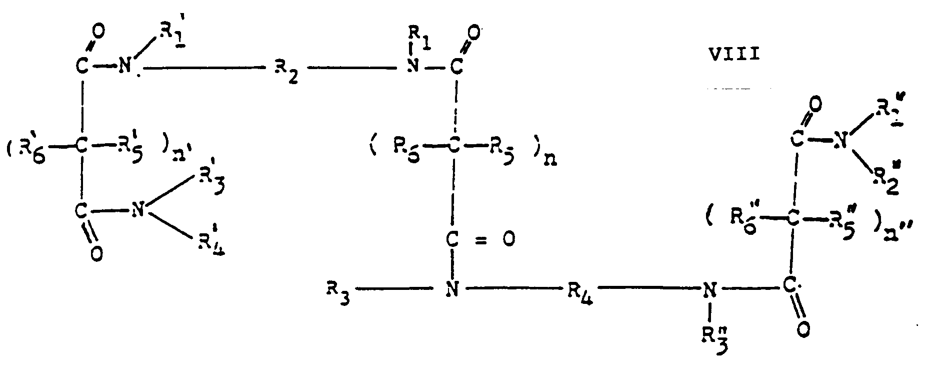 in which R₁', R₃', R₄', R₅', R₆', R₁, R₃, R₅, R₆, R₁'', R₂'', R₃'', R₅'' and R₆'' and the symbols n, n' and n'' are defined in the same way as in Claim 4, by reacting two amino compounds 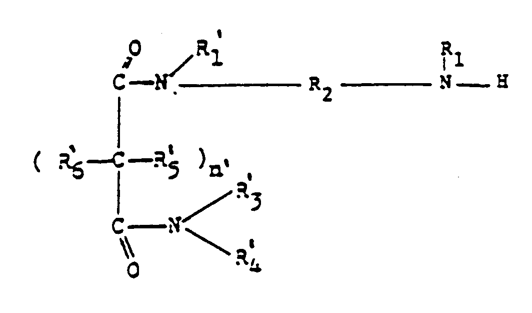 and 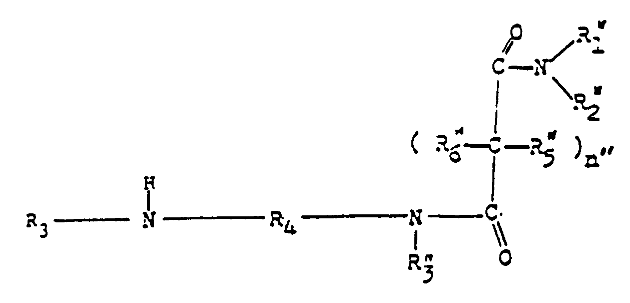 with a dicarboxylic acid of the structure 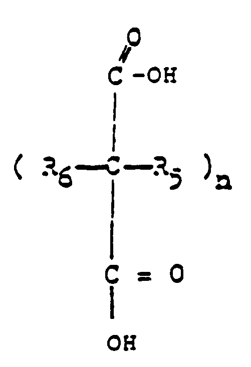 or with a reactive derivative of this dicarboxylic acid to give the carboxamide of formula VIII.

19. Process according to Claim 18, characterized in that carboxamides of formula VIII are prepared which possess the structure indicated in Process Claims 5 to 7 or 9.

## Revendications (Revendications pour l'(les) Etat(s) contractant(s) suivant(s): AT, BE, CH, DE, DK, FR, GB, IT, LI, LU, NL, SE)

1. Amides d'acide carborylique qui forment des complexes lipophiles avec des ions magnésium et sont caractérisés en ce qu'ils présentent 6 groupes amide d'acide carboxylique par molécule et possèdent par molécule d'amide d'acide carboxylique trois groupes diamide d'acide dicarboxylique de formule I 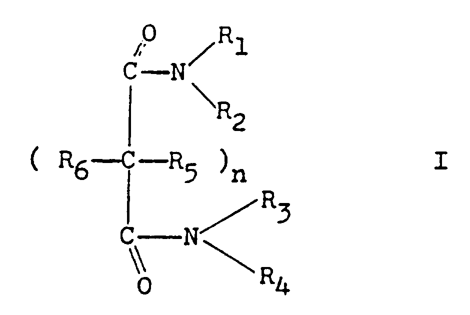 dans laquelle, les radicaux R₁, R₂, R₃ et R₄ désignent, indépendamment l'un de l'autre, des atomes d'hydrogène, des radicaux alkyle, alcényle ou alcynyle éventuellement substitués, des radicaux cycloalkyle éventuellement substitués, des radicaux aromatiques ou hétérocycliques éventuellement substitués ou des combinaisons de radicaux de ce type, les radicaux R₅ et R₆ désignent des atomes d'hydrogène et des radicaux alkyle contenant 1 à 4 atomes de carbone ou des atomes d'halogène et n est égal à 0 ou à un nombre entier de l'ordre de 1 à 3,
au moins un des radicaux R₁, R₂, R₃, R₄, R₅ et R₆ désignant toutefois avec deux des radicaux correspondants R₁', R₂', R₃', R₄', R₅', R₆', R₁'', R₂'', R₃'', R₄'', R₅'' ou R₆'' des deux autres structures de diamide d'acide dicarboxylique,
respectivement, de formule I' ou I'' correspondante, un radical aliphatique, alicyclique, aromatique ou hétéroaromatique polyvalent ou une combinaison de tels radicaux qui lie le groupe diamide d'acide dicarboxylique en question de formule I à un atome d'azote ou un atome de carbone d'une autre structure correspondante de diamide d'acide dicarboxylique de formule I', ou I'', de l'amide d'acide carboxylique en formant le composé avec 6 groupes amides d'acide carboxylique par molécule, l'ensemble de la structure de ce composé à 6 groupes amide d'acide carboxylique par molécule étant à chaîne ouverte ou cyclique et,
soit le radical R₁ et R₂ avec l'atome d'azote auquel sont liés les deux radicaux, soit le radical R₃ et R₄ avec l'atome d'azote auquel sont liés les deux radicaux R₃ et R₄ forment un radical hétérocyclique présentant éventuellement d'autres hétéroatomes ou le radical R₁ ou le radical R₂ d'un des deux atomes d'azote du groupe diamide d'acide dicarboxylique de formule I assurant avec le radical R₃ ou R₄ de l'autre des deux atomes d'azote du groupe diamide d'acide dicarboxylique de formule I la cyclisation de ce groupe diamide d'acide dicarboxylique par formation d'une structure cyclique.

2. Amides d'acide carboxylique selon la revendication 1, caractérisés en ce que deux des radicaux R₁, R₂, R₃ et R₄ désignent conjointement avec un radical correspondant R₁', R₂', R₃' et R₄' et avec un radical R₁'', R₂'', R₃'' et R₄'' des deux autres structures de diamide d'acide dicarboxylique de formule I' ou II'' correspondante, un radical divalent aliphatique, alicyclique, aromatique, hétéroaromatique ou une combinaison de tels radicaux qui assurent la liaison du groupe diamide d'acide dicarboxylique de formule I par un atome d'azote de celui-ci à une autre structure de diamides d'acide dicarboxylique des formules I' et I'' correspondantes en formant le composé avec six groupes amide d'acide carboxylique par molécule, toute la structure du composé étant à chaîne ouverte ou cyclique avec six groupes amide d'acide carboxylique.

3. Amides d'acide carboxylique selon la revendication 1 ou 2, caractérisés en ce qu'ils présentent la formule II suivante 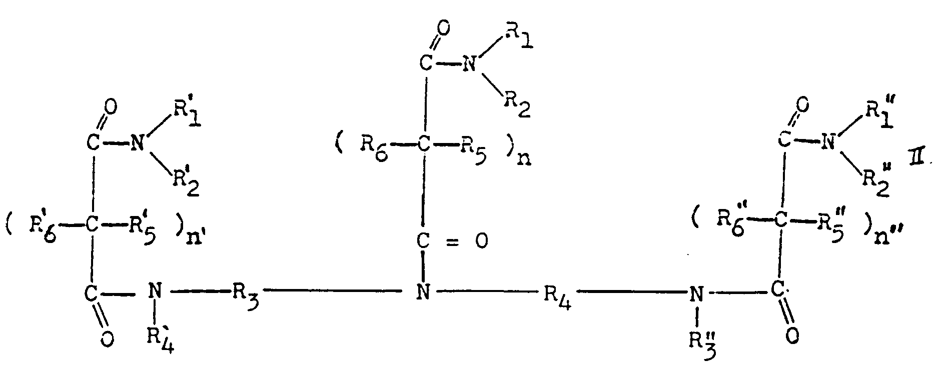 dans laquelle les radicaux R₃ et R₄ désignent indépendamment l'un de l'autre des radicaux aliphatiques, alicycliques, aromatiques ou hétéroaromatiques divalents ou une combinaison de tels radicaux et les radicaux R₁', R₂', R₄', R₅', R₆', R₁'', R₂'' R₃'', R₅'', R₆'' et les symboles n' et n'' ont la même signification que les radicaux R₁, R₂, R₃, R₄, R₅, R₆ et le symbole n dans les groupes diamide d' acide dicarboxylique de formule I, le radical R₂ et/ou le radical R₁ représentant éventuellement avec le radical R₁' ou R₂' ou R₄' ou R₃'' ou R₂'' ou R₁'' un radical divalent et/ou le radical R₁' et/ou le radical R₂' représentant avec le radical R₃'' ou le radical R₂'' ou le radical R₁'' un radical divalent et/ou
le radical R₁'' ou le radical R₂'' représentant avec le radical R₄' un radical divalent et les radicaux divalents cités constituant des radicaux aliphatiques, alicycliques, aromatiques ou hétéroaromatiques ou une combinaison de tels radicaux dont les amides d'acide carboxylique de formule II procèdent à la cyclisation en un composé monocyclique, bicyclique, tricyclique ou polycyclique.

4. Amides d'acide carboxylique selon la revendication 1 ou 2, caractérisé en ce qu'ils présentent des amides d'acide carboxylique de formule VIII suivante 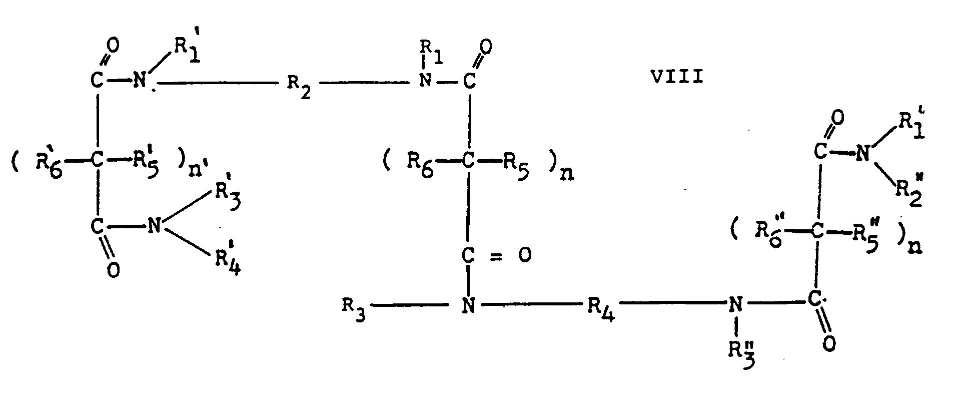 dans laquelle, les radicaux R₂ et R₄ désignent, indépendamment l'un de l'autre, des radicaux aliphatiques,alicycliques, aromatiques ou hétéroaromatiques divalents ou des combinaisons de ces radicaux et les radicaux R₁, R₁', R₁'', R₂'', R₃, R₃', R₄' et R₃'' ont la même signification que celle définie dans la revendication 1 dans le cadre des groupes correspondants diamide d'acide dicarboxylique de formule I ou les unités correspondantes de formule I' et I'' ou
le radical R₁'' avec le radical R₂'' et/ou
le radical R₃' avec le radical R₄' et/ou
le radical R₁ avec le radical R₃ et les atomes d'azote correspondants auxquels sont liés ces radicaux forment un cycle hétérocyclique et les radicaux R₅, R₆, R₅', R₆', R₅'' et R₆'' ont les mêmes significations que celles définies dans la revendication 1 dans le cadre des groupes diamide d'acide dicarboxylique correspondantes de formule I ou les groupes correspondants de formules I' et I'' ou
le radical R₃ et/ou le radical R₁ forme avec le radical R₁', R₃', R₄', R₃'', R₂'' ou R₁'' un radical divalent et/ou
le radical R₃' et/ou le radical R₄' forme avec le radical R₃'' ou R₂'' ou R₁'' un radical divalent et/ou
le radical R₁'' ou le radical R₂'' forme avec le radical R₄' un radical divalent et
les radicaux divalents susmentionnés étant des radicaux aliphatiques, alicycliques, aromatiques ou hétéroaromatiques ou une combinaison de deux ou plusieurs radicaux de ce type et les amides d'acide carboxylique de formule VIII étant cyclisé en amides d'acide carboxylique à structure monocyclique, bicyclique, tricyclique ou polycyclique étant ainsi formé.

5. Amides d'acide carboxylique selon une des revendications 1 à 4, caractérisés en ce que, dans les 3 groupes diamides d'acide dicarboxylique de formule I ou I' ou I'', n, n' ou n'' désignent le nombre 1 ou 2 et en ce que les radicaux R₅, R₆, R₅', R₆', R₅'' et R₆'' désignent de préférence des atomes d'hydrogène ou des radicaux méthyle, les atomes d'hydrogène étant tout particulièrement recommandés.

6. Amides d'acide carboxylique selon une des revendications 1 à 5, caractérisés en ce qu'ils comportent dans leur molécule au moins un radical alkyle, alcényle ou alcynyle éventuellement substitué qui contient au moins 4, de préférence 5 à 15, et tout particulièrement 6 à 12 atomes de carbone et/ou en ce que les atomes d'azote de deux groupes amide d'acide carboxylique différents de cet amide d'acide carboxylique sont reliés l'un à l'autre par un radical aliphatique divalent éventuellement substitué qui comporte éventuellement une ou deux liaisons doubles ou triples, ce radical aliphatique présentant une longueur de chaîne d'au moins 4 atomes de carbone, de préférence de 5 à 15 atomes de carbone et tout particulièrement de 6 à 12 atomes de carbone.

7. Amides d'acide carborylique selon une des revendications 3 à 6, caractérisés en ce que, dans la formule II, les radicaux R₃ et R₄ ou, dans la formule VIII, les radicaux R₂ et R₄ désignent des radicaux alkylène d'une longueur de chaîne d'au moins 4 atomes de carbone, de préférence des radicaux non ramifiés de formule
-(CH₂)_{z}-
dans laquelle z désigne un nombre entier de l'ordre de 5 à 15, tout particulièrement de 6 à 12, et
en ce que les amides d'acide carboxylique de formule II ou VIII sont de préférence des composés non cycliques,
le radical R₁ ainsi que les radicaux R₁' et R₁'', dans les composés correspondants de formule II, désignant un radical alkyle avec une longueur de chaîne d'au moins 4 atomes de carbone, de préférence une chaîne alkyle avec 5 à 15 atomes de carbone, tout particulièrement 6 à 12 atomes de carbone et en ce que, dans les composés non cycliques de formule VIII, tant le radical R₃' que le radical R₁'' désignent un radical alkyle avec une longueur de chaîne d'au moins 4 atomes de carbone, de préférence un radical alkyle avec 5 à 15 atomes de carbone et, tout particulièrement, avec 6 à 12 atomes de carbone.

8. Amides d'acide carboxylique selon une des revendications 3 ou 5 à 7, caractérisés en ce qu'ils présentent la formule III suivante 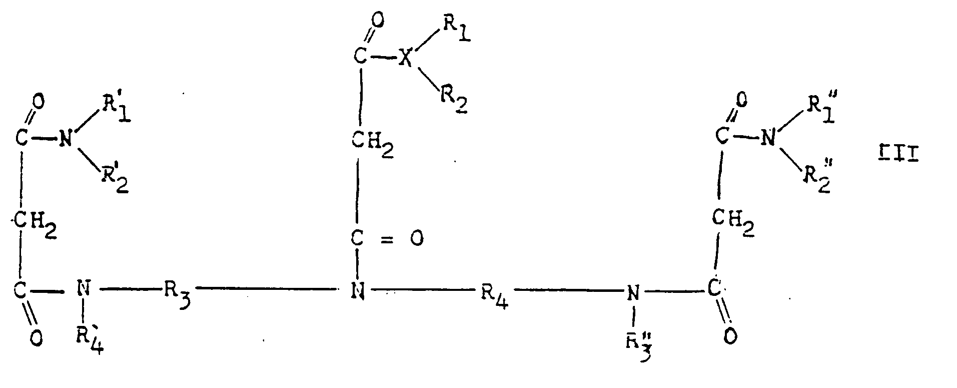 dans laquelle les radicaux R₃ et R₄ désignent un groupe de formule
-(CH₂)_{z}-
dans laquelle z désigne 5, 6 ou 7, z ayant de préférence la même signification dans les deux radicaux et, tout particulièrement, étant égal à 6,
en ce que R₄' et R₃'' désignent l'hydrogène ou un radical alkyle avec 1 à 4 atomes de carbone,
les radicaux R₁, R₁' et R₁'' désignant des radicaux alkyle avec 5 à 9 atomes de carbone, les radicaux R₁, R₁' et R₁'' étant de préférence identiques l'un à l'autre et désignant tout particulièrement des radicaux alkyle à chaîne droite correspondants et
en ce que les radicaux R₂, R₂' et R₂'' sont de préférence identiques l'un à l'autre et désignent tout particulièrement un atome d'hydrogène ou un radical alkyle avec 1 à 4 atomes de carbone, et plus spécifiquement un radical méthyle.

9. Amides d'acide carboxylique selon une des revendications 4 à 7, caractérisés en ce qu'ils présentent la formule IX suivante 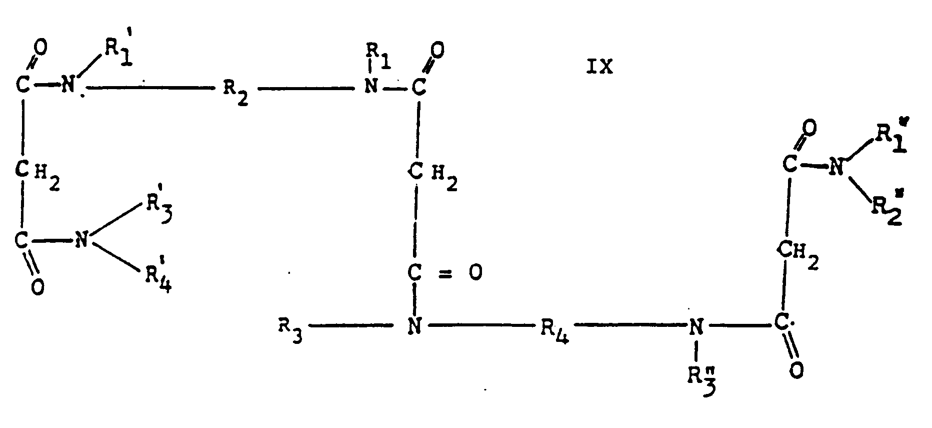 dans laquelle les radicaux R₂ et R₄ désignent indépendamment l'un de l'autre des radicaux de formule
-(CH₂)_{z}-
dans laquelle z désigne un nombre entier de l'ordre de 5 à 9 et les radicaux R₄', R₁, R₃, R₃', R₁', R₁'', R₃'', R₂'' désignent, indépendamment l'un de l'autre, des atomes d'hydrogène ou des radicaux alkyle avec 1 à 12 atomes de carbone.

10. Complexes lipophiles à partir d'ions magnésium et de composés présentant par molécule 6 groupes amide d'acide carboxylique selon une des revendications 1 à 9, de préférence des complexes lipophiles à partir d'une mole d'ions magnésium et d'une mole d' amides d'acide carboxylique.

11. Procédé de préparation d'un complexe lipophile à partir d'ions magnésium, caractérisé en ce que les ions magnésium sont mis en contact avec les composés présentant 6 groupes amide d'acide carboxylique par molécule selon une des revendications 1 à 9, les complexes de magnésium lipophiles correspondants se formant, de préférence des complexes 1 : 1 à partir d'une mole d'ions magnésium et d'une mole d'amide d'acide carboxylique.

12. Dispositif de test de détermination de l'activité ou de la concentration d'ions magnésium dans des échantillons liquides, caractérisé en ce qu'il contient comme composant sélecteur de magnésium un composé présentant 6 groupes amide d'acide carboxylique par molécule selon une des revendications 1 à 9.

13. Dispositif de test selon la revendication 12, caractérisé en ce qu'il contient le composé sélecteur de magnésium dans un ou sur un support inerte, de préférence dans un matériau polymère.

14. Dispositif de test selon la revendication 12 ou 13, caractérisé en ce qu'il est une partie sélectrice d'ions qui contient l'amide d'acide carboxylique incorporé dans un matériau polymère de préférence un homopolymère d'halogénure de polyvinyle ou un copolymère d'halogénure de polyvinyle.

15. Dispositif de test selon la revendication 14, caractérisé en ce qu'il contient comme autre composant un plastifiant, de préférence un plastifiant d'éther, par exemple le o-nitrophényl-octyléther ou un plastifiant d'ester de préférence à base d'un diester d'acide dicarboxylique ou d'un tétraester d'acide tétracarboxylique, par exemple un tétraester d'acide benzophénonetétracarboxylique ou un tétraester d'acide benzhydroltétracarboxylique et en ce que la partie sélectrice d'ions contient éventuellement un composant faisant office d'échangeur d'ions, par exemple le tétraphénylborate dans lequel les noyaux benzène portent éventuellement des substituants ou des sels de ces borates.

16. Procédé de préparation d'amides d'acide carboxylique selon la revendication 3 qui présentent par molécule six groupes amide d'acide carboxylique et possèdent la formule II suivante 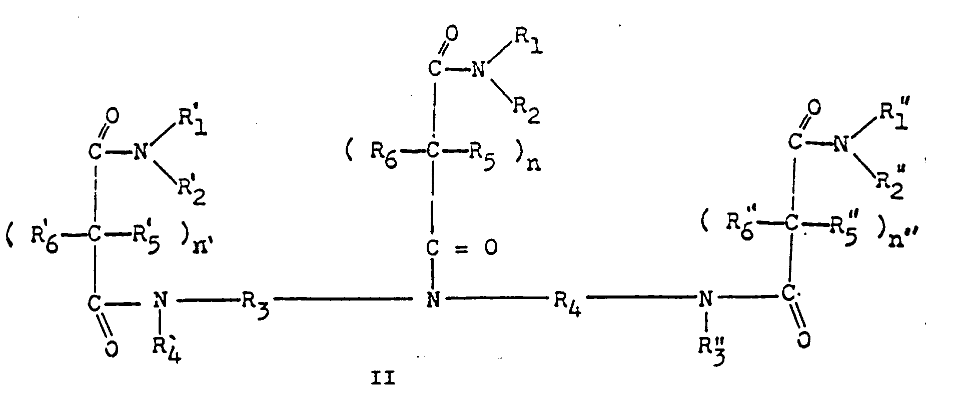 dans laquelle les radicaux R₃ et R₄ ainsi que les radicaux R₁', R₂', R₄', R₅', R₆', R₁, R₂, R₅, R₆, R₁'', R₂'', R₃'', R₅'' R₆'' et les symboles n et n' et n'' présentent la même signification que dans la revendication 3 en faisant réagir une triamine de formule IV 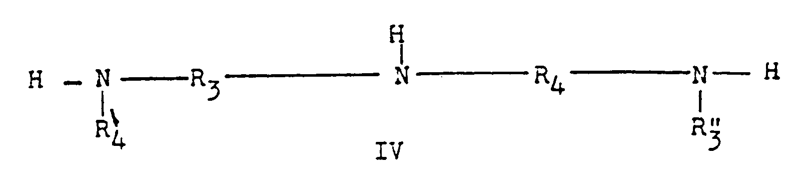 avec les acides carboxyliques des formules Va, Vb, Vc suivantes 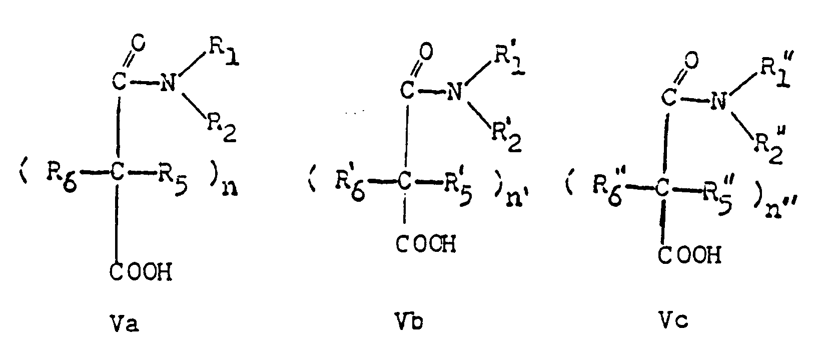 ou des dérivés réactifs de ces acides carboxyliques pour former l'amide d'acide carboxylique correspondant de formule II ou
en faisant d'abord réagir l'amine de formule IV avec des acides dicarboxyliques des formules VIa, VIb, et VIc 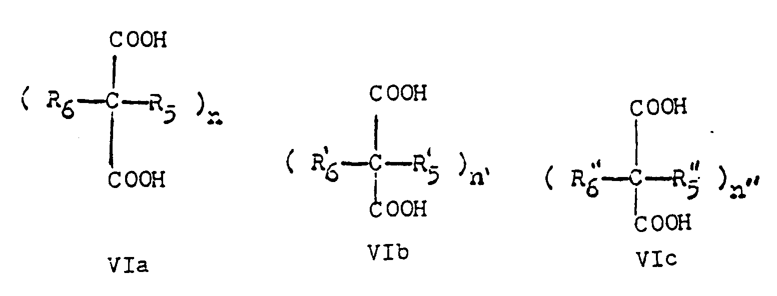 ou des dérivés réactifs de ces acides dicarboxyliques, l'un des deux radicaux carboxyle de l'acide dicarboxylique ou du dérivé d'acide réactif de l'acide dicarboxylique se présentant éventuellement sous forme protégée et en faisant ensuite réagir le produit intermédiaire obtenu de formule VII 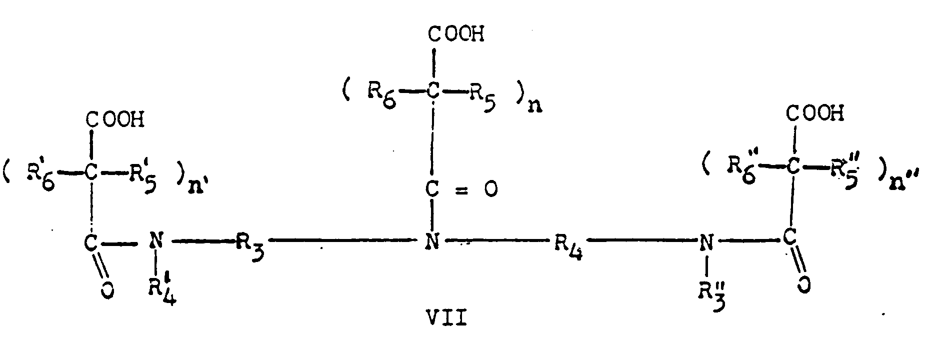 ou un dérivé réactif de ce produit intermédiaire de formule VII avec les amines correspondantes pour former le produit final souhaité de formule II.

17. Procédé de préparation des amides d'acide carboxylique selon la revendication 4 qui contiennent par molécule six groupes amide d'acide carboxylique et possèdent la formule suivante VIII 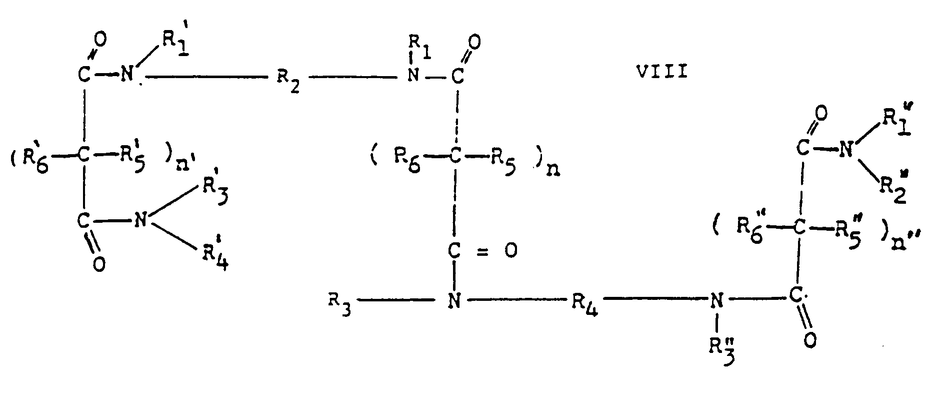 dans laquelle R₁', R₃', R₄', R₅', R₆', R₁, R₃, R₅, R₆, R₁'', R₂'', R₃'', R₅'' et R₆'' ainsi que les symboles n, n' et n'' ont la même signification que dans la revendication 4 en faisant réagir deux composés aminés 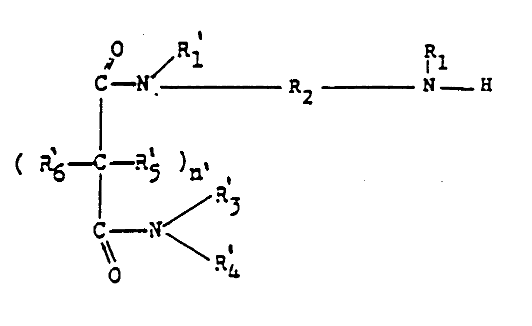 et 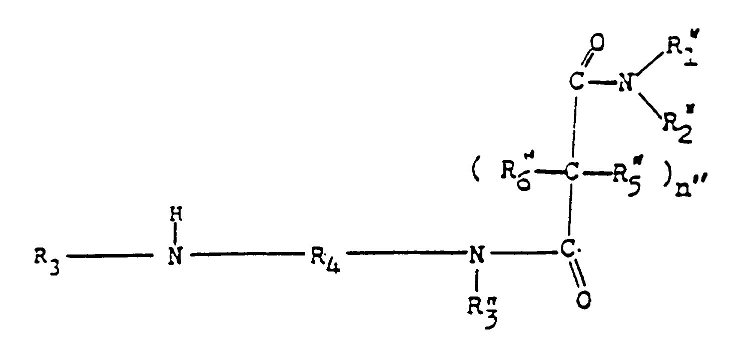 avec un acide dicarboxylique de structure 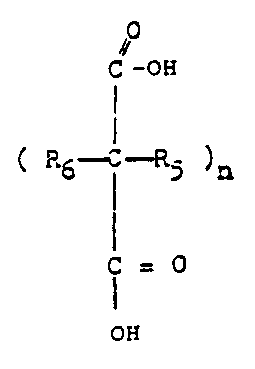 ou avec un dérivé réactif de cet acide dicarboxylique, l'amide d'acide carboxylique de formule VIII étant ainsi obtenu.

## Revendications (Revendications pour l'(les) Etat(s) contractant(s) suivant(s): ES)

1. Procédé de préparation de complexes de magnésium lipophiles, caractérisé en ce que des ions magnésium sont amenés à réagir avec des amides d'acide carboxylique qui présentent six groupes amide d'acide carboxylique par molécule et possèdent par molécule d'amide d'acide carboxylique trois groupes diamide d'acide dicarboxylique qui possèdent la formule I suivante 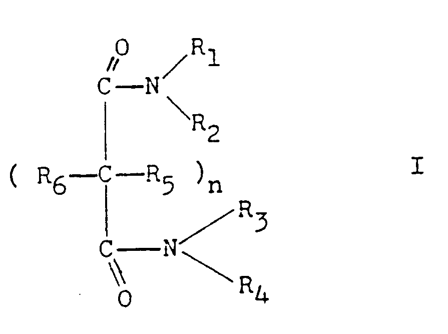 dans laquelle, les radicaux R₁, R₂, R₃ et R₄ désignent, indépendamment l'un de l'autre, des atomes d'hydrogène, des radicaux alkyle, alcényle ou alcynyle éventuellement substitués, des radicaux cycloalkyle éventuellement substitués, des radicaux aromatiques ou hétérocycliques éventuellement substitués ou des combinaisons de ce type, les radicaux R₅ et R₆ désignent des atomes d'hydrogène et des radicaux alkyle contenant 1 à 4 atomes de carbone ou des atomes d'halogène et n est égal à 0 ou à un nombre entier de l'ordre de 1 à 3,
au moins un des radicaux R₁, R₂, R₃, R₄, R₅ et R₆ désignant toutefois avec deux des radicaux correspondants R₁', R₂', R₃', R₄', R₅', R₆', R₁'', R₂'', R₃'', R₄'', R₅'' ou R₆'' des deux autres structures de diamide d'acide dicarboxylique,
respectivement, de formule I' ou I" correspondante, un radical aliphatique, alicyclique, aromatique ou hétéroaro-matique polyvalent ou une combinaison de tels radicaux qui lie le groupe amide d'acide dicarboxylique en question de formule I à un atome d'azote ou un atome de carbone d'une autre structure correspondante de diamide d'acide dicarboxylique de formule I', ou I'', l' ensemble de la structure de ce composé à 6 groupes amide d'acide carboxylique par molécule étant à chaîne ouverte ou cyclique et,
soit le radical R₁ et R₂ avec l'atome d'azote auquel sont liés les deux radicaux, soit le radical R₃ et R₄ avec l'atome d'azote auquel sont liés les deux radicaux R₃ et R₄ forment un radical hétérocyclique présentant éventuellement d'autres hétéroatomes ou le radical R₁ ou le radical R₂ d'un des deux atomes d'azote du groupe diamide d'acide dicarboxylique de formule I assurant avec le radical R₃ ou R₄ de l'autre des deux atomes d'azote du groupe diamide d'acide dicarboxylique de formule I la cyclisation de ce groupe diamide d'acide dicarboxylique par formation d'une structure cyclique.

2. Procédé selon la revendication 1, caractérisé en ce que, dans les amides d'acide carboxylique qui présentent par molécule trois groupes diamide d'acide dicarboxylique de formule I, deux des radicaux R₁, R₂, R₃ et R₄ désignent conjointement avec un radical correspondant R₁', R₂', R₃' et R₄' et avec un radical R₁'', R₂'', R₃'',et R₄'' des deux autres structures de diamide d'acide dicarboxylique de formule I' ou II'' correspondante, un radical divalent aliphatique, alicyclique, aromatique, hétéroaromatique ou une combinaison de tels radicaux qui assurent la liaison du groupe diamide d'acide dicarboxylique de formule I par un atome d'azote de celui-ci à une autre structure de diamides d'acide dicarboxylique des formules I' et I'' correspondantes en formant le composé avec six groupes amide d'acide carboxylique par molécule, toute la structure du composé étant à chaîne ouverte ou cyclique avec six groupes amide d'acide carboxylique.

3. Procédé selon la revendication 1 ou 2 caractérisé en ce qu'il est procédé à une réaction avec des amides d'acide carboxylique qui présentent la formule II suivante 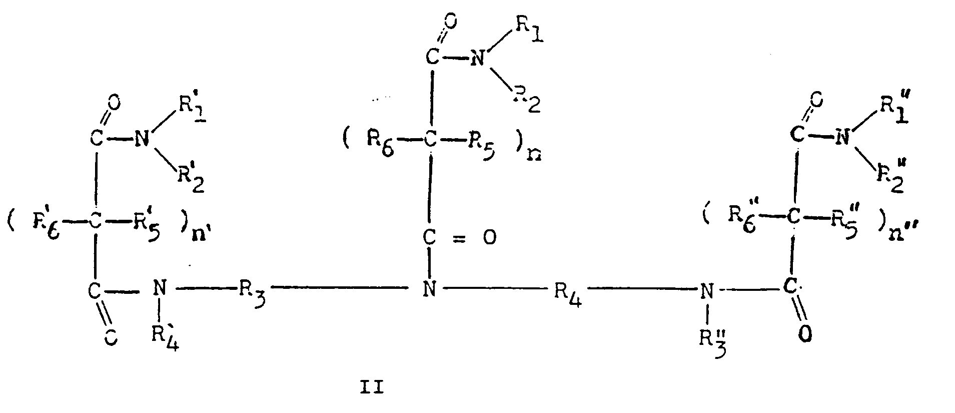 dans laquelle les radicaux R₃ et R₄ désignent indépendamment l'un de l'autre des radicaux aliphatiques, alicycliques, aromatiques ou hétéroaromatiques divalents ou une combinaison de tels radicaux, les radicaux R₁', R₂', R₄', R₅', R₆', R₁'', R₂'', R₃'', R₅'', R₆'' et les symboles n' et n'' ont la même signification que les radicaux R₁, R₂, R₃, R₄, R₅, R₆ et le symbole n dans les groupes diamide d'acide dicarboxylique de formule I, le radical R₂ et/ou le radical R₁ représentant éventuellement avec le radical R₁' ou R₂' ou R₄' ou R₃'' ou R₂'' ou R₁'' un radical divalent et/ou le radical R₁' et/ou le radical R₂' représentant avec le radical R₃'' ou le radical R₂'' ou le radical R₁'' un radical divalent et/ou
le radical R₁'' ou le radical R₂'' représentant avec le radical R₄' un radical divalent et les radicaux divalents cités constituant des radicaux aliphatiques, alicycliques, aromatiques ou hétéroaromatiques ou une combinaison de tels radicaux dont les amides d'acide carboxylique de formule II procèdent à la cyclisation en un composé monocyclique, bicyclique, tricyclique ou polycyclique.

4. Procédé selon la revendication 1 ou 2, caractérisé en ce que la réaction se déroule avec des amides d'acide carboxylique qui répondent à la formule VIII suivante 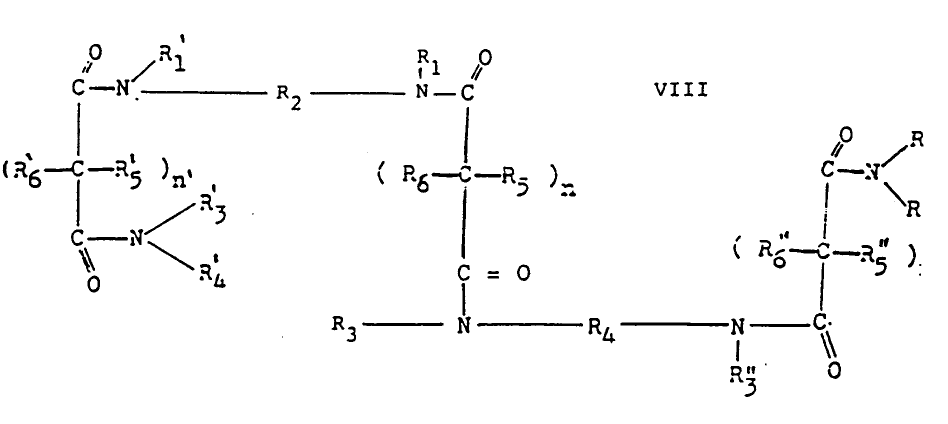 dans laquelle, les radicaux R₂ et R₄ désignent, indépendamment l'un de l'autre, des radicaux aliphatiques, alicycliques, aromatiques ou hétéroaromatiques divalents ou des combinaisons de tels radicaux et les radicaux R₁, R₁', R₁'', R₂'', R₃, R₃', R₄' et R₃'' ont la même signification que celle définie dans la revendication 1 dans le cadre des groupes correspondants diamide d'acide dicarboxylique de formule I ou les unités correspondantes de formule I' et I'' ou
le radical R₁'' avec le radical R₂'' et/ou
le radical R₃' avec le radical R₄' et/ou
le radical R₁ avec le radical R₃ et les atomes d'azote correspondants auxquels sont liés ces radicaux forment un cycle hétérocyclique et les radicaux R₅, R₆, R₅', R₆', R₅'' et R₆'' ont les mêmes significations que celles définies dans la revendication 1 dans le cadre des groupes diamide d'acide dicarboxylique correspondants de formule I ou les groupes correspondants de formules I' et I'' ou
le radical R₃ et/ou le radical R₁ forme avec le radical R₁', R₃', R₄', R₃'', R₂'' ou R₁'' un radical divalent et/ou
le radical R₃' et/ou le radical R₄' forme avec le radical R₃'' ou R₂'' ou R₁'' un radical divalent et/ou
le radical R₁'' ou le radical R₂'' forme avec le radical R₄' un radical divalent,
les radicaux divalents susmentionnés étant des radicaux aliphatiques, alicycliques, aromatiques ou hétéroaromatiques ou une combinaison de deux ou plusieurs radicaux de ce type et les amides d'acide carboxylique de formule VIII, par lesquels les amides d'acide carboxyliques correspondants sont cyclisés, un amide d'acide carboxylique correspondant présentant une structure globale monocyclique, bicyclique, tricyclique ou polycyclique étant ainsi formée.

5. Procédé selon une des revendications 1 à 4, caractérisé en ce que, dans les 3 groupes diamide d'acide dicarboxylique de formule I ou I' ou I'' des amides d'acide carboxylique utilisés, n, n' ou n'' désignent le nombre 1 ou 2 et en ce que les radicaux R₅, R₆, R₅', R₆', R₅'' et R₆'' désignent de préférence des atomes d'hydrogène ou des radicaux méthyle, les atomes d'hydrogène étant tout particulièrement recommandés.

6. Procédé selon une des revendications 1 à 5, caractérisé en ce que les amides d'acide carboxylique utilisés comportent dans leur molécule au moins un radical alkyle, alcényle ou alcynyle éventuellement substitué qui contient au moins 4, de préférence 5 à 15, et tout particulièrement 6 à 12 atomes de carbone et/ou en ce que les atomes d'azote de deux groupes amide d'acide carboxylique différents de cet amide d'acide carboxylique sont reliés l'un à l'autre par un radical aliphatique divalent éventuellement substitué qui comporte éventuellement une ou deux liaisons doubles ou triples, ce radical aliphatique présentant une longueur de chaîne d'au moins 4 atomes de carbone, de préférence de 5 à 15 atomes de carbone et tout particulièrement de 6 à 12 atomes de carbone.

7. Procédé selon une des revendications 3 à 6, caractérisé en ce que des amides d'acide carboxylique de formule II ou des amides d'acide carboxylique de formule VIII sont utilisés, les radicaux R₃ et R₄ ou, dans la formule VIII, les radicaux R₂ et R₄ désignent des radicaux alkylène d'une longueur de chaîne d'au moins 4 atomes de carbone, de préférence des radicaux non ramifiés de formule
-(CH₂)_{z}-
dans laquelle z désigne un nombre entier de l'ordre de 5 à 15, tout particulièrement de 6 à 12, et
en ce que les amides d'acide carboxylique de formule II ou VIII sont de préférence des composés non cycliques, le radical R₁ ainsi que les radicaux R₁' et R₁'', dans les composés correspondants de formule II, désignant un radical alkyle avec une longueur de chaîne d'au moins 4 atomes de carbone, de préférence une chaîne alkyle avec 5 à 15 atomes de carbone, tout particulièrement 6 à 12 atomes de carbone et en ce que, dans les composés non cycliques de formule VIII, tant le radical R₃' que le radical R₁'' désignent un radical alkyle avec une longueur de chaîne d'au moins 4 atomes de carbone, de préférence un radical alkyle avec 5 à 15 atomes de carbone et, tout particulièrement, avec 6 à 12 atomes de carbone.

8. Procédé selon une des revendications 3 ou 5 à 7, caractérisé en ce que les amides d'acide carboxylique utilisés répondent à la formule III suivante 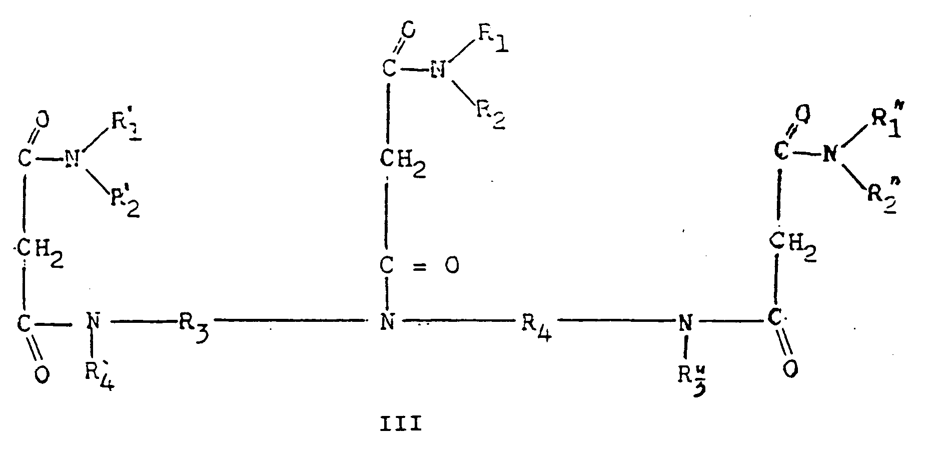 dans laquelle, les radicaux R₃ et R₄ désignent un groupe de formule
-(CH₂)_{z}-
dans laquelle z désigne 5, 6 ou 7, z ayant de préférence la même signification dans les deux radicaux et, tout particulièrement, étant égal à 6,
en ce que R₄' et R₃'' désignent l'hydrogène ou un radical alkyle avec 1 à 4 atomes de carbone,
les radicaux R₁, R₁' et R₁'' désignant des radicaux alkyle avec 5 à 9 atomes de carbone, les radicaux R₁, R₁' et R₁'' étant de préférence identiques l'un à l'autre et désignant tout particulièrement des radicaux alkyle à chaîne droite correspondants et
en ce que les radicaux R₂, R₂' et R₂'' sont de préférence identiques l'un à l'autre et désignent particulièrement un atome d'hydrogène ou un radical alkyle avec 1 à 4 atomes de carbone, et plus spécifiquement un radical méthyle.

9. Procédé selon une des revendications 4 à 7, caractérisé en ce que les amides d'acide carboxylique utilisés répondent à la formule IX suivante 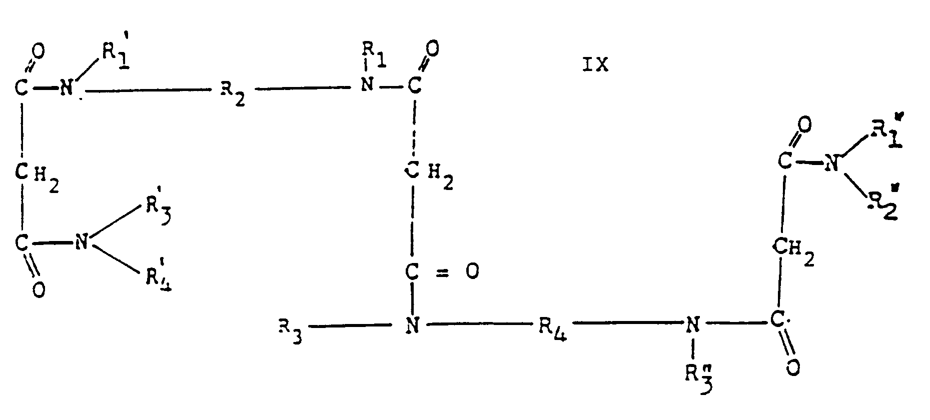 dans laquelle les radicaux R₂ et R₄ désignent indépendamment l'un de l'autre des radicaux de formule
-(CH₂)_{z}-
dans laquelle z désigne un nombre entier de l'ordre de 5 à 9 et les radicaux R₄', R₁', R₃, R₃', R₁, R₁'', R₃'', R₂'' désignent, indépendamment l'un de l'autre, des atomes d'hydrogène ou des radicaux alkyle avec 1 à 12 atomes de carbone.

10. Procédé selon une des revendications 1 à 9, caractérisé en ce que, lors de la réaction d'ions magnésium avec le composé présentant par molécule 6 groupes amide d'acide carboxylique, il se forme des complexes de magnésium lipophiles correspondants qui sont des complexes 1 : 1 à partir d'une mole d'ions magnésium et d'une mole d'amide d'acide carboxylique.

11. Procédé selon une des revendications 1 à 10, caractérisé en ce que les complexes de magnésium lipophiles sont préparés en mettant en contact un matériau liquide qui contient les ions magnésium avec un amide d'acide carboxylique présentant 6 groupes amide d'acide carboxylique par molécule et se trouvant dans ou sur un matériau de support.

12. Dispositif de test de détermination de l'activité ou de la concentration d'ions magnésium dans des échantillons liquides, caractérisé en ce qu'il contient comme composant sélecteur de magnésium un amide d'acide carboxylique présentant 6 groupes amide d'acide carboxylique par molécule et possédant la structure qui est définie pour les amides d'acide carboxylique correspondants dans une des revendications de procédé 1 à 9.

13. Dispositif de test selon la revendication 12, caractérisé en ce qu'il contient le composé sélecteur de magnésium dans un ou sur un support inerte, de préférence dans un matériau polymère.

14. Dispositif de test selon la revendication 12 ou 13, caractérisé en ce qu'il est une partie sélectrice d'ions qui contient l'amide d'acide carboxylique incorporé dans un matériau polymère de préférence un homopolymère d'halogénure de polyvinyle ou un copolymère d'halogénure de polyvinyle.

15. Dispositif de test selon la revendication 14, caractérisé en ce qu'il contient comme autre composant un plastifiant, de préférence un plastifiant d'éther, par exemple le o-nitrophényl-octyléther ou un plastifiant d'ester de préférence, à base d'un diester d'acide dicarboxylique ou d'un tétraester d'acide tétracarboxylique, par exemple un tétraester d'acide benzophénonetétracarboxylique ou un tétraester d'acide benzhydroltétracarboxylique et en ce que la partie sélectrice d'ions contient éventuellement un composant faisant office d'échangeur d'ions, par exemple le tétraphénylborate dans lequel les noyaux benzène portent éventuellement des substituants ou des sels de ces borates.

16. Procédé de préparation d'amides d'acide carboxylique qui peuvent être utilisés comme substance de départ dans le procédé selon la revendication 3, caractérisé en ce que sont préparés des amides d'acide carboxylique correspondants qui répondent à la formule II suivante 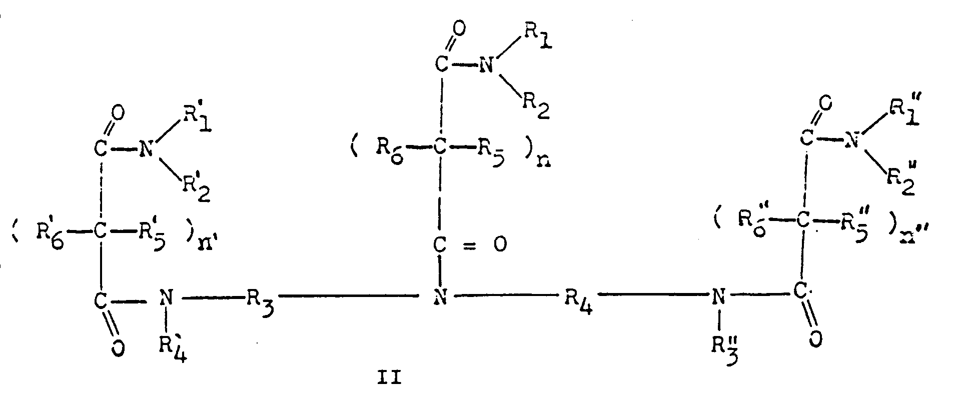 dans laquelle les radicaux R₃ et R₄ ainsi que les radicaux R₁', R₂', R₄', R₅', R₆', R₁, R₂, R₅, R₆, R₁'', R₂'', R₃'', R₅'', R₆'' et les symboles n et n' et n'' présentent la même signification que dans la revendication 3 en faisant réagir une triamine de formule IV 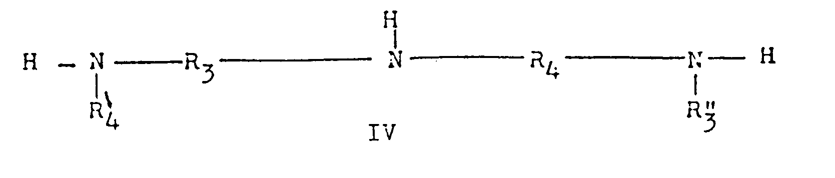 avec les acides carboxyliques des formules Va, Vb, Vc suivantes 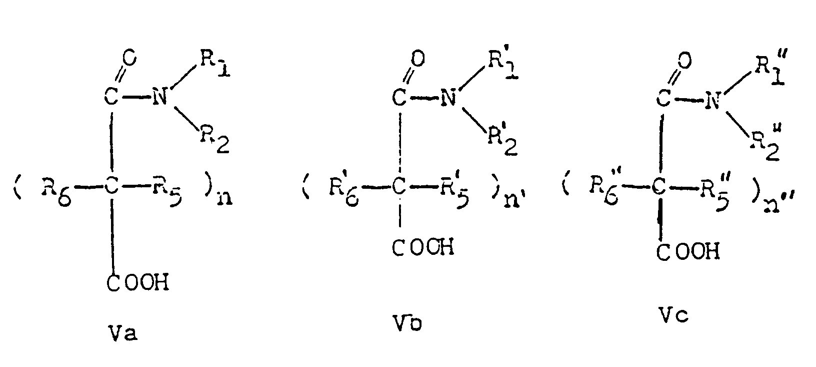 ou des dérivés réactifs de ces acides carboxyliques pour former l'amide d'acide carboxylique correspondant de formule II ou
en faisant d'abord réagir l'amine de formule IV avec des acides dicarboxyliques des formules VIa, VIb, et VIc 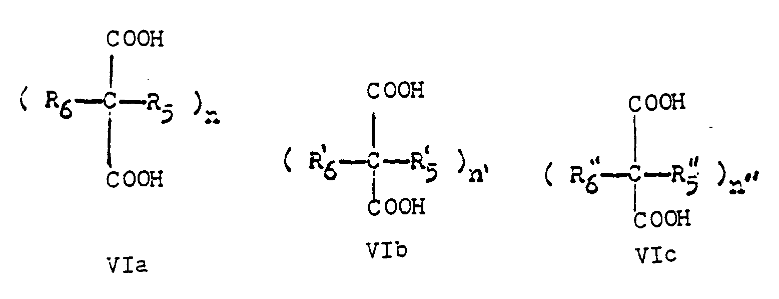 ou des dérivés réactifs de ces acides dicarboxyliques, l'un des deux radicaux carboxyle de l'acide dicarboxylique ou du dérivé d'acide réactif de l'acide dicarboxylique se présentant éventuellement sous forme protégée et en faisant ensuite réagir le produit intermédiaire obtenu de formule VII 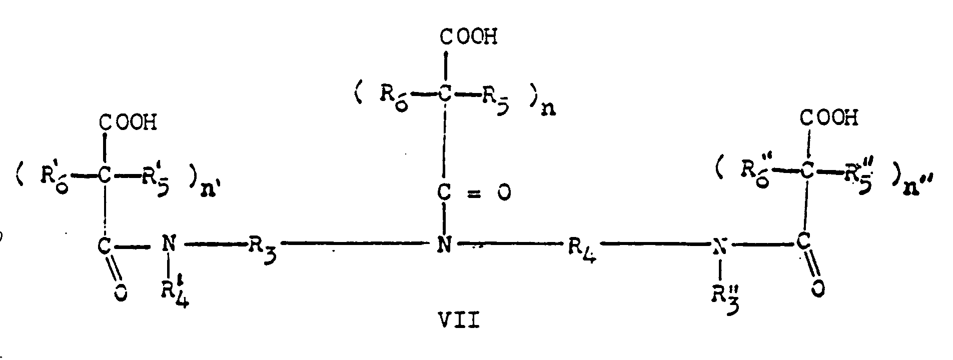 ou un dérivé réactif de ce produit intermédiaire de formule VII avec les amines correspondantes pour former le produit final souhaité de formule II.

17. Procédé selon la revendication 16, caractérisé en ce que sont préparés des amides d'acide carboxylique de formule II qui possèdent la structure définie dans une des revendications du procédé 5 à 8.

18. Procédé de préparation d'amides d'acide carboxylique qui peuvent être utilisés comme substance de départ dans le procédé selon la revendication 4, caractérisé en ce que sont préparés des amides d'acide carboxylique correspondants qui répondent à la formule VIII suivante 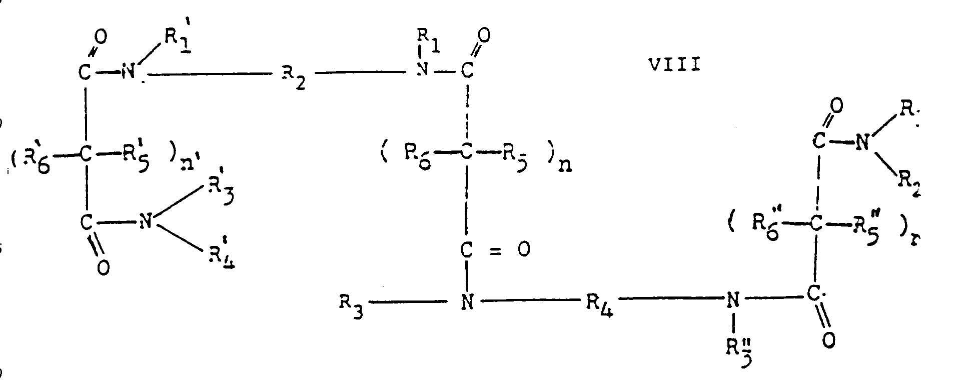 dans laquelle R₁', R₃', R₄', R₅', R₆', R₁, R₃, R₅, R₆, R₁'', R₂'', R₃'', R₅'' et R₆'' ainsi que les symboles n, n' et n'' ont la même signification que dans la revendication 4 en faisant réagir deux composés aminés 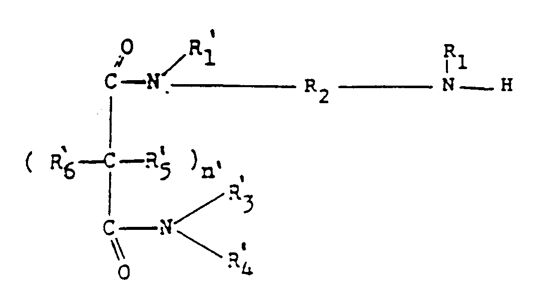 et 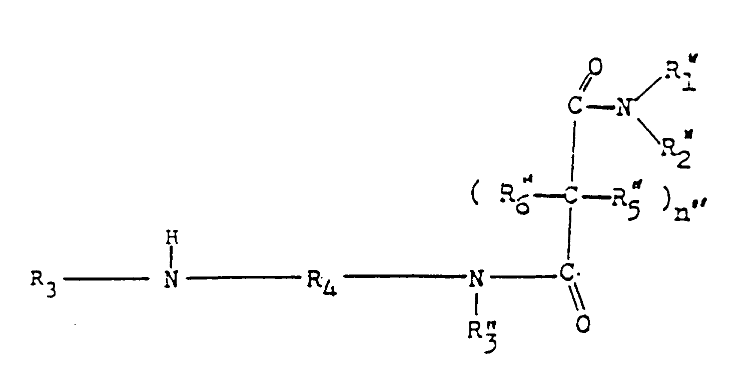 avec un acide dicarboxylique de structure 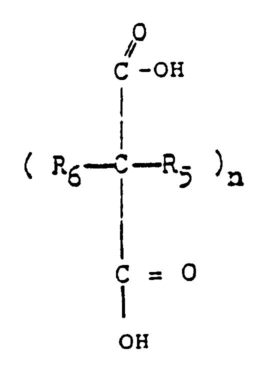 ou avec un dérivé réactif de cet acide dicarboxylique, l'amide d'acide carboxylique de formule VIII étant ainsi obtenu.

19. Procédé selon la revendication 18, caractérisé en ce que sont préparés des amides d'acide carboxylique de formule VIII qui possèdent la structure définie dans les revendications de procédé 5 à 7 ou 9.
